# EUROPEAN PATENT APPLICATION

(11) **EP 1 911 738 A1**
(43) Date of publication of application: **16.04.2008**
(21) Application number: 06782312.0
(22) Date of filing: 28.07.2006
(51) Int. Cl.: C07C 59/68, A61K 31/192, A61K 31/222, A61K 31/277, A61K 31/381, A61K 31/4015, A61K 31/4035, A61K 31/404, A61K 31/4164, A61K 31/4192, A61K 31/42, A61K 31/421, A61K 31/422, A61K 31/4245, A61K 31/426, A61K 31/44, A61K 31/4406, A61K 31/4409, A61K 31/443, A61K 31/4433, A61K 31/4436

(54) **PHENOXYALKANOIC ACID COMPOUND**

(30) Priority: 29.07.2005 JP 2005221627
(71) Applicant: Takeda Pharmaceutical Company Limited, Osaka-shi, Osaka 541-0045 (JP)
(72) Inventor: IMOTO, Hiroshi, Jusohonmachi, 2-chome, Yodogawa-ku, Osaka-shi, 5328686 (JP)
(74) Representative: Jones, Nicholas Andrew
(86) International application number: PCT/JP2006/315452
(87) International publication number: WO 2007/013694

(57) **Abstract**

The present invention provides a compound represented by the formula: 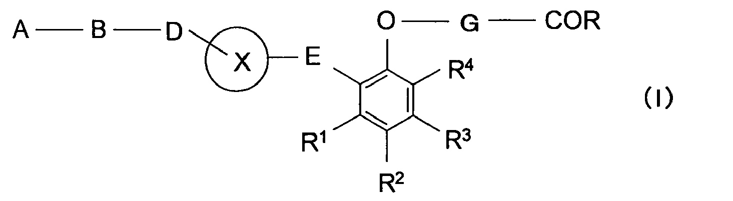 wherein each symbol is as defined in the specification.

Since the compound of the present invention has superior hypoglycemic action and superior hypolipidemic action, it is useful as an agent for the prophylaxis or treatment of diabetes, hyperlipidemia, impaired glucose tolerance and the like.

## Description

### Technical Field

The present invention relates to a novel phenoxyalkanoic acid compound having a superior hypoglycemic action and a superior hypolipidemic action, which is useful as an agent for the prophylaxis or treatment of diabetes, hyperlipidemia, impaired glucose tolerance and the like.

### Background Art

As the alkanoic acid derivative, compounds described in the following documents are known.
(1) WO 00/64876 describes a compound represented by the formula: 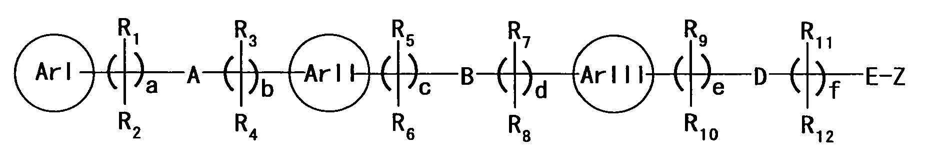 wherein
   ArI, ArII and ArIII are each aryl, heteroaryl and the like (these are optionally substituted by alkyl, aryl, heteroaryl, etc.);
   A is O, S, NR₁₃ and the like;
   B is O, S, CO and the like;
   D is O, S and the like;
   E is a bond or ethylene;
   Z is R₂₁O₂C- and the like;
   a, b, c and e are each 0 to 4;
   d is 0 to 5;
   f is 0 to 6; and
   R₁ to R₁₂ are each H and the like,
   which is a PPAR ligand-receptor binder and useful for the treatment of diabetes and the like.
(2) US Patent No. 4432973 describes a compound represented by formula: 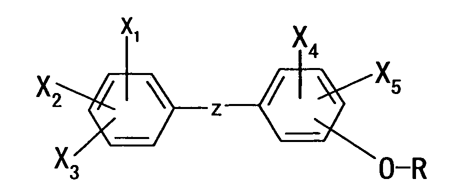 wherein
   Z is CO or CHOH;
   X₂, X₃, X₄ and X₅ are each H, halogen and the like;
   X₁ is H, halogen, -O-C(CH₃)₂CO₂-R"' (wherein R"' is C₁₋₄ alkyl) and the like; and
   R is an optionally substituted unhydrolyzable monosaccharide residue (β-D-glucosyl, β-D-xylosyl etc.),
   which is as an antiulcerogenic drug, an antiplatelet aggregation drug and the like.
(3) US Patent No. 6869975 describes a compound represented by formula:

   R¹-O₂C-CR^{a}R^{b}-Y-Ar¹-X-Ar²-Z¹-Z²-Ar³

   wherein
   X is O, S(O)ₘ, CR'R" or SO₂NR";
   Y is O or CR'R";
   Z¹ and Z² are each O, S(O)ₘ, (CR'R")ₙ and the like;
   Ar¹ and Ar² are each an aromatic group;
   Ar³ is aryl;
   R¹ is H, C₁₋₈ alkyl and the like;
   R', R", R^{a} and R^{b} are each H, C₁₋₄ alkyl and the like; and
   m is 0 to 2,
   which is a PPAR regulator and useful for the treatment of metabolic disorder, diabetes and the like,.
(4) WO 02/053547 describes a compound represented by formula: 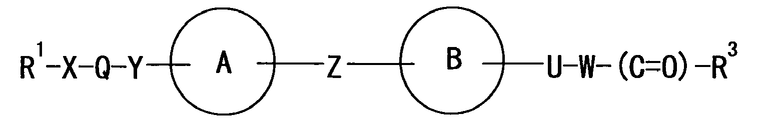 wherein
   R¹ is an optionally substituted 5-membered aromatic heterocyclic group;
   X is a bond and the like;
   Q is a divalent C₁₋₂₀ hydrocarbon group;
   Y is a bond, O and the like;
   ring A is an optionally substituted aromatic ring;
   Z is -(CH₂)ₙ-Z¹- or -Z¹-(CH₂)ₙ- (wherein n is 1 to 8; and Z₁ is O, S, SO, SO₂ or NR¹⁶);
   ring B is pyridine, benzene or naphthalene, each of which is optionally substituted;
   U is a bond, O and the like;
   W is a divalent C₁₋₂₀ hydrocarbon group; and,
   R³ is -OH and the like,
   which is a PPAR ligand.
(5) WO 2004/091604 describes a compound represented by formula: 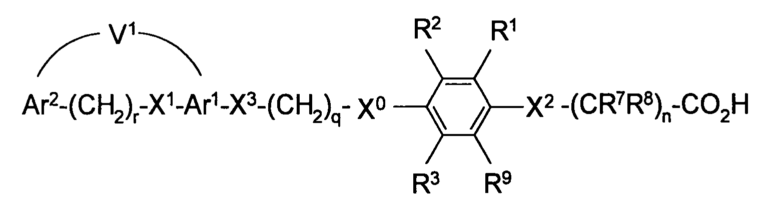 wherein
   X⁰ is absent, O, S, NR⁴, CH₂CH₂, CH=CH or C≡C;
   X¹ and X³ are each absent, O, C=O, S, CHOR¹¹ or NR⁴;
   X² is absent, O, S or NR⁴;
   Ar¹ and Ar² are each absent, substituted or unsubstituted aryl or substituted or unsubstituted heteroaryl;
   V¹ is absent, saturated or unsaturated C₁₋₄ hydrocarbon chain; R¹, R², R³, R⁷, R⁸ and R⁹ are each hydrogen, lower alkyl and the like;
   n is 0 to 5; and
   q and r are each 0 to 10,
   which is a PPAR activity regulating agent.
(6) JP-A-59-177556 describes a compound represented by formula: 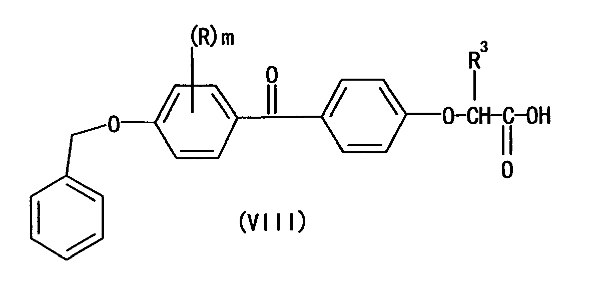
wherein
R is a substituent on the benzene ring;
m is 0 to 4; and
R³ is H, C₁₋₁₈ alkyl or C₆₋₂₄ aryl,
which is a starting material of Coupler.

Peroxisome proliferator-activated receptor gamma (PPAR γ), a member of the nuclear hormone receptor superfamily, which is typically exemplified by steroid hormone receptors and thyroid hormone receptors, plays an important role as a master regulator in the differentiation of adipocytes with its expression induced in the very early stage of adipocyte differentiation. PPAR γ forms a dimer with the retinoid X receptor (RXR) by binding to a ligand, and binds to a responsive site of the target gene in the nucleus to directly control (activate) transcription efficiency. In recent years, the possibility that 15-deoxy-Δ^{12.14} prostaglandin J₂, which is a metabolite of prostaglandin D₂, serves as an endogenous ligand for PPAR γ, has been suggested, and it has been shown that a class of insulin sensitizer, typically exemplified by thiazolidinedione derivatives, possess ligand activity for PPAR γ, and that its potency is proportional to its hypoglycemic action or adipocyte differentiation-promoting action (see Cell, vol. 83, p. 803 (1995): The Journal of Biological Chemistry, vol. 270, p. 12953 (1995); Journal of Medicinal Chemistry, vol. 39, p. 655 (1996)). Furthermore, it has been shown that 1) PPAR γ is expressed in cultured cells of human liposarcoma origin, whose proliferation is ceased by the addition of a PPAR γ ligand (see Proceedings of the National Academy of Sciences of The United States of America, vol. 94, 23), 2) nonsteroidal anti-inflammatory drugs, typically exemplified by indomethacin and fenoprofen, have PPAR γ ligand activity (see The Journal of Biological Chemistry, vol. 272, p. 3406 (1997)), 3) PPAR γ is expressed at high levels in activated macrophages, with the transcription of a gene involved in inflammation inhibited by the addition of a ligand therefor (see Nature, vol. 391, p. 79 (1998)), 4) PPAR γ ligands suppress the production of inflammatory cytokines (TNFα, IL-1β, IL-6) by monocytes (see Nature, vol. 391, p. 82 (1998)) and the like.

However, no reports are regarding to the compound of the present invention.

### Disclosure of the Invention

The development of a novel compound useful as an agent for the prophylaxis or treatment of diabetes, hyperlipidemia, impaired glucose tolerance and the like and having superior properties as a pharmaceutical agent, such as fewer side effects and the like, has been demanded.

The present inventor first found that a compound represented by the formula (I): 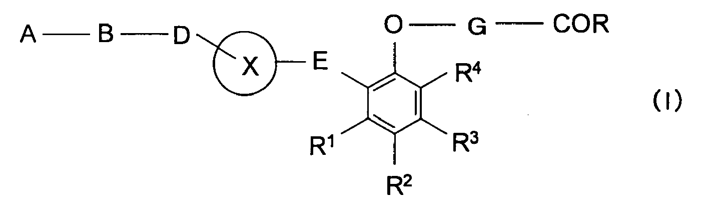 wherein
A is an optionally substituted cyclic group;
B is a bond or a spacer having 1 to 10 carbon atoms in the main chain;
D is O or NR⁵
wherein
R⁵ is a hydrogen atom or a substituent;
X is an optionally further substituted aromatic ring;
E is CO, C(OR⁶)R⁷ or C=NR⁸
wherein
R⁶, R⁷ and R⁸ are the same or different and each is a hydrogen atom or a substituent;
G is an optionally substituted divalent C₁₋₆ hydrocarbon group; R is -OR⁹
wherein R⁹ is a hydrogen atom or an optionally substituted hydrocarbon group, or

-NR¹⁰R¹¹

wherein R¹⁰ and R¹¹ are the same or different and each is a hydrogen atom, an optionally substituted hydrocarbon group or an optionally substituted heterocyclic group, or R¹⁰ and R¹¹ form, together with the adjacent nitrogen atom, an optionally substituted heterocycle; and
R¹, R², R³ and R⁴ are the same or different and each is a hydrogen atom or a substituent,
or a salt thereof (hereinafter, sometimes to be referred to as compound (I)),
which is characterized by a chemical structure wherein a group -E- and a group -O-G-COR are bonded on benzene ring at the ortho positions, has a superior hypoglycemic action and a hypolipidemic action, and is useful for the prophylaxis or treatment of diabetes, hyperlipidemia, impaired glucose tolerance and the like. Based on this finding, the present inventors have conducted studies, which resulted in the completion of the present invention. Accordingly, the present invention relates to
(1) compound (I);
(2) compound (I) wherein A is a C₆₋₁₄ aryl group, a C₃₋₁₀ cycloalkyl group, a 5- or 6-membered aromatic heterocyclic group optionally condensed with a benzene ring, or a 5- or 6-membered non-aromatic heterocyclic group optionally condensed with a benzene ring, each of which is optionally substituted;
(3) compound (I) wherein the spacer having 1 to 10 carbon atoms in the main chain for B is -(CH₂)ₖ-
   wherein k is an integer of 1 to 10;
(4) compound (I) wherein D is O;
(5) compound (I) wherein X is a C₆₋₁₄ arene or a 6-membered aromatic heterocycle, each of which is optionally substituted;
(6) compound (I) wherein E is CO;
(7) compound (I) wherein G is a C₁₋₆ alkylene group optionally substituted by 1 to 3 substituents selected from a halogen atom and a C₆₋₁₄ aryl group;
(8) compound (I) wherein R is a hydroxy group;
(9) compound (I) wherein R¹, R², R³ and R⁴ are the same or different and each is a hydrogen atom, a C₁₋₆ alkoxy group optionally substituted by C₁₋₆ alkoxy group(s), a C₁₋₆ alkyl group, a carboxyl group, a carbamoyl group, a thiocarbamoyl group, a C₁₋₆ alkoxy-carbonyl group, a halogen atom, a cyano group or a nitro group;
(10) compound (I) which is selected from
   (5-methoxy-2-{4-[(5-methyl-2-phenyl-1,3-oxazol-4-yl)methoxy]benzoyl}phenoxy)acetic acid,
   2-(5-methoxy-2-{4-[(5-methyl-2-phenyl-1,3-oxazol-4-yl)methoxy]benzoyl}phenoxy)-2-methylpropanoic acid,
   2-[5-methoxy-2-({6-[(5-methyl-2-phenyl-1,3-oxazol-4-yl)methoxy]pyridin-3-yl}carbonyl)phenoxy]propanoic acid,
   2-(2-{4-[(5-methyl-2-phenyl-1,3-oxazol-4-yl)methoxy]benzoyl}phenoxy)propanoic acid, and
   (2R)-2-(5-methoxy-2-{4-[(5-methyl-2-phenyl-1,3-oxazol-4-yl)methoxy]benzoyl}phenoxy)propanoic acid;
(11) a prodrug of compound (I);
(12) a pharmaceutical agent comprising compound (I) or a prodrug thereof;
(13) the pharmaceutical agent of the abovementioned (12), which is an agent for the prophylaxis or treatment of diabetes;
(14) an insulin sensitizer comprising compound (I) or a prodrug thereof;
(15) a method for the prophylaxis or treatment of diabetes in a mammal, which comprises administering compound (I) or a prodrug thereof to the mammal;
(16) a method of improving insulin resistance in a mammal, which comprises administering compound (I) or a prodrug thereof to the mammal;
(17) use of compound (I) or a prodrug thereof for the production of an agent for the prophylaxis or treatment of diabetes;
(18) use of compound (I) or a prodrug thereof for the production of an insulin sensitizer;
   and the like.

The compound of the present invention has a superior hypoglycemic action and a hypolipidemic action, and is useful as an agent for the prophylaxis or treatment of diabetes, hyperlipidemia, impaired glucose tolerance and the like.

### Best Mode for Embodying the Invention

The definition of each symbol in the formula (I) is described in detail in the following.

In the present specification, the "halogen atom" means a fluorine atom, a chlorine atom, a bromine atom or an iodine atom, unless otherwise specified.

In the present specification, the "C₁₋₃ alkylenedioxy group" means methylenedioxy, ethylenedioxy or the like, unless otherwise specified.

In the present specification, the "C₁₋₆ alkyl group" means methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like, unless otherwise specified.

In the present specification, the "C₁₋₆ alkoxy group" means methoxy, ethoxy, propoxy, isopropoxy, butoxy, isobutoxy, sec-butoxy, tert-butoxy or the like, unless otherwise specified.

In the present specification, the "C₁₋₆ alkoxy-carbonyl group" means methoxycarbonyl, ethoxycarbonyl, propoxycarbonyl, tert-butoxycarbonyl or the like, unless otherwise specified.

In the present specification, the "C₁₋₆ alkyl-carbonyl group" means acetyl, propanoyl, butanoyl, isobutanoyl, pentanoyl, isopentanoyl, hexanoyl or the like, unless otherwise specified.

R¹, R², R³ and R⁴ are the same or different and each is a hydrogen atom or a substituent.

Examples of the "substituent" for R¹, R², R³ or R⁴ include an "optionally substituted hydrocarbon group", an "optionally substituted heterocyclic group", an "optionally substituted hydroxy group", an "optionally substituted mercapto group", an "optionally substituted amino group", a "cyano group", a "nitro group", an "acyl group", a "halogen atom" and the like.

Examples of the "hydrocarbon group" of the above-mentioned "optionally substituted hydrocarbon group" include a C₁₋₁₀ alkyl group, a C₂₋₁₀ alkenyl group, a C₂₋₁₀ alkynyl group, a C₃₋₁₀ cycloalkyl group, a C₃₋₁₀ cycloalkenyl group, a C₄₋₁₀ cycloalkadienyl group, a C₆₋₁₄ aryl group, a C₇₋₁₃ aralkyl group, a C₈₋₁₃ arylalkenyl group, a C₃₋₁₀ cycloalkyl-C₁₋₆ alkyl group and the like.

Here, examples of the C₁₋₁₀ alkyl group include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.

Examples of the C₂₋₁₀ alkenyl group include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.

Examples of the C₂₋₁₀ alkynyl group include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

Examples of the C₃₋₁₀ cycloalkyl group include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like.

Examples of the C₃₋₁₀ cycloalkenyl group include 2-cyclopenten-1-yl, 3-cyclopenten-1-yl, 2-cyclohexen-1-yl, 3-cyclohexen-1-yl and the like.

Examples of the C₄₋₁₀ cycloalkadienyl group include 2,4-cyclopentadien-1-yl, 2,4-cyclohexadien-1-yl, 2,5-cyclohexadien-1-yl and the like.

The above-mentioned C₃₋₁₀ cycloalkyl group, C₃₋₁₀ cycloalkenyl group and C₄₋₁₀ cycloalkadienyl group each may be condensed with a benzene ring, and examples of such fused ring group include indanyl, dihydronaphthyl, tetrahydronaphthyl, fluorenyl and the like.

Examples of the C₆₋₁₄ aryl group include phenyl, naphthyl, anthryl, phenanthryl, acenaphthylenyl, biphenylyl and the like. Of these, phenyl, 1-naphthyl, 2-naphthyl and the like are preferable.

Examples of the C₇₋₁₃ aralkyl group include benzyl, phenethyl, naphthylmethyl, biphenylylmethyl and the like.

Examples of the C₈₋₁₃ arylalkenyl group include styryl and the like.

Examples of the C₃₋₁₀ cycloalkyl-C₁₋₆ alkyl group include cyclohexylmethyl and the like.

The C₁₋₁₀ alkyl group, C₂₋₁₀ alkenyl group and C₂₋₁₀ alkynyl group exemplified as the aforementioned "hydrocarbon group" optionally have 1 to 3 substituents at substitutable positions.

Examples of such substituent include
(1) a C₃₋₁₀ cycloalkyl group (e.g., cyclopropyl, cyclohexyl);
(2) a C₆₋₁₄ aryl group (e.g., phenyl, naphthyl) optionally substituted by 1 to 3 substituents selected from a C₁₋₆ alkyl group optionally substituted by 1 to 3 halogen atoms, a hydroxy group, a C₁₋₆ alkoxy group, a halogen atom and a C₁₋₆ alkylsulfonyloxy group (e.g., methylsulfonyloxy);
(3) an aromatic heterocyclic group (e.g., thienyl, furyl, pyridyl, oxazolyl, thiazolyl, tetrazolyl, oxadiazolyl, pyrazinyl, quinolyl, indolyl) optionally substituted by 1 to 3 substituents selected from a C₁₋₆ alkyl group optionally substituted by 1 to 3 halogen atoms, a hydroxy group, a C₁₋₆ alkoxy group and a halogen atom;
(4) a non-aromatic heterocyclic group (e.g., tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl) optionally substituted by 1 to 3 substituents selected from a C₁₋₆ alkyl group optionally substituted by 1 to 3 halogen atoms, a hydroxy group, a C₁₋₆ alkoxy group, an oxo group and a halogen atom;
(5) an amino group optionally mono- or di-substituted by substituent(s) selected from a C₁₋₆ alkyl group, a C₁₋₆ alkyl-carbonyl group, a C₁₋₆ alkoxy-carbonyl group, a C₆₋₁₄ aryl-carbonyl group (e.g., benzoyl), a C₇₋₁₃ aralkyl-carbonyl group (e.g., benzylcarbonyl, phenethylcarbonyl), a C₁₋₆ alkylcarbamoyl group (e.g., methylcarbamoyl, ethylcarbamoyl), a C₆₋₁₄ aryl-carbamoyl group (e.g., phenylcarbamoyl, 1-naphthylcarbamoyl, 2-naphthylcarbamoyl), a C₇₋₁₃ aralkylcarbamoyl group (e.g., benzylcarbamoyl), a C₁₋₆ alkylsulfonyl group (e.g., methylsulfonyl, ethylsulfonyl, isopropylsulfonyl), a C₆₋₁₄ arylsulfonyl group (e.g., benzenesulfonyl, toluenesulfonyl, 1-naphthalenesulfonyl, 2-naphthalenesulfonyl) and a C₇₋₁₃ aralkylsulfonyl group (e.g., benzylsulfonyl);
(6) an amidino group;
(7) a C₁₋₆ alkyl-carbonyl group optionally substituted by 1 to 3 halogen atoms;
(8) a C₁₋₆ alkoxy-carbonyl group optionally substituted by 1 to 3 halogen atoms;
(9) a C₁₋₆ alkylsulfonyl group (e.g., methylsulfonyl) optionally substituted by 1 to 3 halogen atoms;
(10) a carbamoyl group optionally mono- or di-substituted by substituent(s) selected from a C₁₋₆ alkyl group optionally substituted by 1 to 3 halogen atoms, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(11) a thiocarbamoyl group optionally mono- or di-substituted by C₁₋₆ alkyl group(s) optionally substituted by 1 to 3 halogen atoms;
(12) a sulfamoyl group optionally mono- or di-substituted by C₁₋₆ alkyl group(s) optionally substituted by 1 to 3 halogen atoms;
(13) a carboxyl group;
(14) a hydroxy group;
(15) a C₁₋₆ alkoxy group optionally substituted by 1 to 3 substituents selected from a halogen atom, a carboxyl group, a C₁₋₆ alkoxy group and a C₁₋₆ alkoxy-carbonyl group;
(16) a C₂₋₆ alkenyloxy group (e.g., ethenyloxy) optionally substituted by 1 to 3 halogen atoms;
(17) a C₃₋₁₀ cycloalkyloxy group (e.g., cyclohexyloxy);
(18) a C₇₋₁₃ aralkyloxy group (e.g., benzyloxy);
(19) a C₆₋₁₄ aryloxy group (e.g., phenyloxy, naphthyloxy);
(20) a C₁₋₆ alkyl-carbonyloxy group (e.g., acetyloxy, tert-butylcarbonyloxy);
(21) a mercapto group;
(22) a C₁₋₆ alkylthio group (e.g., methylthio, ethylthio) optionally substituted by 1 to 3 halogen atoms;
(23) a C₇₋₁₃ aralkylthio group (e.g., benzylthio);
(24) a C₆₋₁₄ arylthio group (e.g., phenylthio, naphthylthio);
(25) a sulfo group;
(26) a cyano group;
(27) an azido group;
(28) a nitro group;
(29) a nitroso group;
(30) a halogen atom;
(31) a C₁₋₆ alkylsulfinyl group (e.g., methylsulfinyl);
(32) an oxo group;
(33) a C₃₋₁₀ cycloalkyl-C₁₋₆ alkyloxy group (e.g., cyclopropylmethyloxy);
(34) a C₁₋₃ alkylenedioxy group;
(35) a hydroxyimino group optionally substituted by a C₁₋₆ alkyl group;
and the like. When two or more substituents are used, the substituents may be the same or different.

The C₃₋₁₀ cycloalkyl group, C₃₋₁₀ cycloalkenyl group, C₄₋₁₀ cycloalkadienyl group, a C₆₋₁₄ aryl group, a C₇₋₁₃ aralkyl group, C₈₋₁₃ arylalkenyl group and C₃₋₁₀ cycloalkyl-C₁₋₆ alkyl group exemplified as the aforementioned "hydrocarbon group" optionally have 1 to 3 substituents at substitutable positions.

Examples of such substituent include
(1) the groups exemplified as the substituents that the aforementioned C₁₋₁₀ alkyl group and the like optionally have;
(2) a C₁₋₆ alkyl group optionally substituted by 1 to 3 substituents selected from a halogen atom, a carboxyl group, a hydroxy group, a C₁₋₆ alkoxy-carbonyl group, a C₁₋₆ alkyl-carbonyloxy group (e.g., acetyloxy, tert-butylcarbonyloxy), a carbamoyl group and a non-aromatic heterocyclic group (e.g., piperidino);
(3) a C₂₋₆ alkenyl group (e.g., ethenyl, 1-propenyl) optionally substituted by 1 to 3 substituents selected from a halogen atom, a carboxyl group, a C₁₋₆ alkoxy-carbonyl group and a carbamoyl group;
(4) a C₇₋₁₃ aralkyl group (e.g., benzyl) optionally substituted by 1 to 3 substituents selected from a C₁₋₆ alkyl group optionally substituted by 1 to 3 halogen atoms, a hydroxy group, a C₁₋₆ alkoxy group and a halogen atom;
and the like. When two or more substituents are used, the substituents may be the same or different.

Examples of the "heterocyclic group" of the above-mentioned "optionally substituted heterocyclic group" include an aromatic heterocyclic group and a non-aromatic heterocyclic group.

Here, examples of the aromatic heterocyclic group include a 4- to 7-membered (preferably 5- or 6-membered) monocyclic aromatic heterocyclic group containing, as a ring constituting atom besides carbon atom, 1 to 4 hetero atoms selected from an oxygen atom, a sulfur atom and a nitrogen atom, and a fused aromatic heterocyclic group. Examples of the fused aromatic heterocyclic group include a group derived from a fused ring wherein a ring corresponding to the 4- to 7-membered monocyclic aromatic heterocyclic group and one or two rings selected from a 5- or 6-membered ring containing 1 or 2 nitrogen atoms, a 5-membered ring containing one sulfur atom, a benzene ring and the like are condensed, and the like.

Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl) and the like;
fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like;
and the like.

Examples of the non-aromatic heterocyclic group include a 4- to 7-membered (preferably 5- or 6-membered) monocyclic non-aromatic heterocyclic group containing, as a ring constituting atom besides carbon atom, 1 to 4 hetero atoms selected from an oxygen atom, a sulfur atom and a nitrogen atom, and a fused non-aromatic heterocyclic group. Examples of the fused non-aromatic heterocyclic group include a group derived from a fused ring wherein a ring corresponding to the 4- to 7-membered monocyclic non-aromatic heterocyclic group and one or two rings selected from a 5- or 6-membered ring containing 1 or 2 nitrogen atoms, a 5-membered ring containing one sulfur atom, a benzene ring and the like are condensed, and the like.

Preferable examples of the non-aromatic heterocyclic group include monocyclic non-aromatic heterocyclic groups such as pyrrolidinyl (e.g., 1-pyrrolidinyl, 2-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl) and the like;
fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-l-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl) and the like;
and the like.

The "heterocyclic group" of the aforementioned "optionally substituted heterocyclic group" optionally has 1 to 3 substituents at substitutable positions. Examples of such substituent include those exemplified as the substituents that the C₃₋₁₀ cycloalkyl group and the like exemplified as the "hydrocarbon group" of the aforementioned "optionally substituted hydrocarbon group" may have. When two or more substituents are used, the substituents may be the same or different.

Examples of the aforementioned "optionally substituted hydroxy group" include a hydroxyl group optionally substituted by a substituent selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group, a C₁₋₆ alkyl-carbonyl group, a 5- or 6-membered aromatic heterocyclic group, a fused aromatic heterocyclic group and the like, each of which is optionally substituted.

Here, examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group respectively include those exemplified as the "hydrocarbon group" of the aforementioned "optionally substituted hydrocarbon group".

Examples of the 5- or 6-membered aromatic heterocyclic group include a 5- or 6-membered ring group from among the "aromatic heterocyclic groups" exemplified as the "heterocyclic group" of the aforementioned "optionally substituted heterocyclic group".

Examples of the fused aromatic heterocyclic group include a fused ring group from among the "aromatic heterocyclic group" exemplified as the "heterocyclic group" of the aforementioned "optionally substituted heterocyclic group".

The aforementioned C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, C₁₋₆ alkyl-carbonyl group, 5- or 6-membered aromatic heterocyclic group and fused aromatic heterocyclic group optionally have 1 to 3 substituents at the substitutable positions, respectively. When two or more substituents are used, the substituents may be the same or different.

Here, examples of the substituent of the C₁₋₁₀ alkyl group, C₂₋₁₀ alkenyl group and C₁₋₆ alkyl-carbonyl group include those exemplified as the substituents that the C₁₋₁₀ alkyl group and the like exemplified as the "hydrocarbon group" of the aforementioned "optionally substituted hydrocarbon group" may have.

In addition, examples of the substituent of the C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, 5- or 6-membered aromatic heterocyclic group and fused aromatic heterocyclic group include those exemplified as the substituents that the C₃₋₁₀ cycloalkyl group and the like exemplified as the "hydrocarbon group" of the aforementioned "optionally substituted hydrocarbon group" may have.

Examples of the aforementioned "optionally substituted mercapto group" include a mercapto group optionally substituted by a substituent selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group, a C₁₋₆ alkyl-carbonyl group, a 5- or 6-membered aromatic heterocyclic group, a fused aromatic heterocyclic group and the like, each of which is optionally substituted.

Examples of the substituent include those exemplified as the substituents of the aforementioned "optionally substituted hydroxy group".

Examples of the aforementioned "optionally substituted amino group" include an amino group optionally substituted by 1 or 2 substituents selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group and a C₈₋₁₃ arylalkenyl group each of which is optionally substituted; an acyl group and the like.

Here, examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group include those exemplified as the "hydrocarbon group" of the aforementioned "optionally substituted hydrocarbon group".

The C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group optionally have 1 to 3 substituents at the substitutable positions. When two or more substituents are used, the substituents may be the same or different.

Here, examples of the substituent of the C₁₋₁₀ alkyl group and C₂₋₁₀ alkenyl group include those exemplified as the substituents that the C₁₋₁₀ alkyl group and the like exemplified as the "hydrocarbon group" of the aforementioned "optionally substituted hydrocarbon group" may have.

In addition, examples of the substituent of the C₃₋₁₀ cycloalkyl group, C₃₋₁₀ cycloalkenyl group, C₆₋₁₄ aryl group, C₇₋₁₃ aralkyl group and C₈₋₁₃ arylalkenyl group include those exemplified as the substituents that the C₃₋₁₀ cycloalkyl group and the like exemplified as the "hydrocarbon group" of the aforementioned "optionally substituted hydrocarbon group" may have.

Examples of the "acyl group" exemplified as the substituent of the "optionally substituted amino group" and the "substituent" for R¹ and the like include a group represented by the formula: -COR^{a}, -CO-OR^{a}, -SO₂R^{a}, -SOR^{a}, -CO-NR^{a}'R^{b}', -CS-NR^{a}'R^{b}' wherein R^{a} is a hydrogen atom, an optionally substituted hydrocarbon group or an optionally substituted heterocyclic group, and R^{a}' and R^{b}' are the same or different and each is a hydrogen atom, an optionally substituted hydrocarbon group or an optionally substituted heterocyclic group, or R^{a}' and R^{b}' form, together with the adjacent nitrogen atom, an optionally substituted nitrogen-containing heterocycle, and the like.

Examples of the "optionally substituted hydrocarbon group" and "optionally substituted heterocyclic group" for R^{a}, R^{a}' or R^{b}' include those similar to the "optionally substituted hydrocarbon group" and "optionally substituted heterocyclic group", each of which is exemplified as the "substituent" for R¹, R², R³ or R⁴.

Examples of the "nitrogen-containing heterocycle" of the "optionally substituted nitrogen-containing heterocycle" formed by R^{a}' and R^{b}' together with the adjacent nitrogen atom include a 5- to 7-membered nitrogen-containing heterocycle containing, as a ring constituting atom besides carbon atom, at least one nitrogen atom, and optionally further containing 1 or 2 hetero atoms selected from an oxygen atom, a sulfur atom and a nitrogen atom. Preferable examples of the nitrogen-containing heterocycle include pyrrolidine, imidazolidine, pyrazolidine, piperidine, piperazine, morpholine, thiomorpholine, oxopiperazine and the like.

The nitrogen-containing heterocycle may have 1 to 3 (preferably 1 or 2) substituents at the substitutable positions. Examples of such substituent include those exemplified as the substituents that the C₃₋₁₀ cycloalkyl group and the like exemplified as the "hydrocarbon group" of the aforementioned "optionally substituted hydrocarbon group" may have. When two or more substituents are used, the substituents may be the same or different.

Preferable examples of the "acyl group" include
(1) a formyl group;
(2) a carboxyl group;
(3) a C₁₋₆ alkyl-carbonyl group;
(4) a C₁₋₆ alkoxy-carbonyl group optionally substituted by 1 to 3 substituents selected from a carboxyl group, a carbamoyl group, a thiocarbamoyl group, a C₁₋₆ alkoxy-carbonyl group and a C₁₋₆ alkyl-carbonyloxy group (e.g., methoxycarbonyl, ethoxycarbonyl, propoxycarbonyl, tert-butoxycarbonyl; carboxymethoxycarbonyl, carboxyethoxycarbonyl, carboxybutoxycarbonyl; carbamoylmethoxycarbonyl; thiocarbamoylmethoxycarbonyl; ethoxycarbonylmethoxycarbonyl, ethoxycarbonylethoxycarbonyl, methoxycarbonylbutoxycarbonyl, ethoxycarbonylbutoxycarbonyl; tert-butylcarbonyloxymethoxycarbonyl);
(5) a C₃₋₁₀ cycloalkyl-carbonyl group (e.g., cyclopentylcarbonyl, cyclohexylcarbonyl);
(6) a C₆₋₁₄ aryl-carbonyl group (e.g., benzoyl, 1-naphthoyl, 2-naphthoyl) optionally substituted by 1 to 3 substituents selected from a halogen atom, a cyano group, an optionally halogenated C₁₋₆ alkyl group (that is, a C₁₋₆ alkyl group optionally substituted by 1 to 3 halogen atoms), a C₁₋₆ alkoxy group, a carboxyl group, a C₁₋₆ alkoxy-carbonyl group, an aromatic heterocyclic group (e.g., tetrazolyl, oxadiazolyl), a non-aromatic heterocyclic group (e.g., oxooxadiazolyl) and a carbamoyl group;
(7) a C₆₋₁₄ aryloxy-carbonyl group (e.g., phenyloxycarbonyl, naphthyloxycarbonyl) optionally substituted by 1 to 3 substituents selected from a carboxyl group, a C₁₋₆ alkoxy-carbonyl group and a carbamoyl group;
(8) a C₇₋₁₃ aralkyloxy-carbonyl group optionally substituted by 1 to 3 substituents selected from a carboxyl group, a carbamoyl group, a thiocarbamoyl group, a C₁₋₆ alkoxy-carbonyl group, a halogen atom, a cyano group, a nitro group, a C₁₋₆ alkoxy group, a C₁₋₆ alkylsulfonyl group and a C₁₋₆ alkyl group (e.g., benzyloxycarbonyl, phenethyloxycarbonyl; carboxybenzyloxycarbonyl; methoxycarbonylbenzyloxycarbonyl; biphenylylmethoxycarbonyl);
(9) a carbamoyl group optionally mono- or di-substituted by C₁₋₆ alkyl group(s) optionally substituted by 1 to 3 substituents selected from a halogen atom and a C₁₋₆ alkoxy group (e.g., methylcarbamoyl, ethylcarbamoyl, dimethylcarbamoyl, diethylcarbamoyl, ethylmethylcarbamoyl, propylcarbamoyl, isopropylcarbamoyl, butylcarbamoyl, isobutylcarbamoyl, trifluoroethylcarbamoyl, N-methoxyethyl-N-methylcarbamoyl);
(10) a C₁₋₆ alkylsulfonyl group (e.g., methylsulfonyl, carboxymethylsulfonyl) optionally substituted by 1 to 3 substituents selected from a carboxyl group, a carbamoyl group and a C₁₋₆ alkoxy-carbonyl group;
(11) a C₁₋₆ alkylsulfinyl group (e.g., methylsulfinyl);
(12) a thiocarbamoyl group;
(13) a C₇₋₁₃ aralkyl-carbonyl group (e.g., benzylcarbonyl, phenethylcarbonyl);
(14) an aromatic heterocyclyl (e.g., furyl, thienyl, oxazolyl, thiazolyl, isoxazolyl, isothiazolyl, pyrazolyl, pyridyl, pyrazinyl, benzofuranyl, benzothienyl, quinoxalinyl)-carbonyl group (e.g., furylcarbonyl, thienylcarbonyl, thiazolylcarbonyl, pyrazolylcarbonyl, pyridylcarbonyl, pyrazinylcarbonyl, benzofuranylcarbonyl, benzothienylcarbonyl, quinoxalinylcarbonyl) optionally substituted by 1 to 3 substituents selected from a C₁₋₆ alkyl group, a C₆₋₁₄ aryl group, a C₇₋₁₃ aralkyl group, a C₁₋₆ alkoxy group, a carboxyl group, a C₁₋₆ alkoxy-carbonyl group and a carbamoyl group;
and the like.

R¹, R², R³ and R⁴ are preferably the same or different and each is a hydrogen atom, a C₁₋₆ alkoxy group optionally substituted by C₁₋₆ alkoxy group(s) (preferably, methoxy, ethoxy, methoxymethoxy), a C₁₋₆ alkyl group (preferably, methyl), a carboxyl group, a carbamoyl group, a thiocarbamoyl group, a C₁₋₆ alkoxy-carbonyl group, a halogen atom, a cyano group, a nitro group and the like, more preferably, they are the same or different and each is a hydrogen atom, a C₁₋₆ alkoxy group optionally substituted by C₁₋₆ alkoxy group(s) (preferably, methoxy, ethoxy, methoxymethoxy), a C₁₋₆ alkyl group (preferably, methyl) and the like, particularly preferably, a hydrogen atom, a C₁₋₆ alkoxy group optionally substituted by C₁₋₆ alkoxy group(s) (preferably, methoxy, ethoxy, methoxymethoxy) and the like.

Particularly preferable combinations of R¹, R², R³ and R⁴ include a combination of R¹, R² and R⁴ being hydrogen atoms, and R³ being a hydrogen atom or a C₁₋₆ alkoxy group (preferably, methoxy).

A is an optionally substituted cyclic group.

Examples of the "cyclic group" of the "optionally substituted cyclic group" for A include an aromatic group, a non-aromatic cyclic group and the like.

Examples of the aromatic group include an aromatic hydrocarbon group, an aromatic heterocyclic group and the like.

Examples of the aromatic hydrocarbon group include a C₆₋₁₄ aryl group and the like. Examples of the C₆₋₁₄ aryl group include those exemplified as the aforementioned "substituent" for R¹, R², R³ or R⁴.

Examples of the aromatic heterocyclic group include those exemplified as the aforementioned "substituent" for R¹, R², R³ or R⁴.

Examples of the non-aromatic cyclic group include a non-aromatic cyclic hydrocarbon group, a non-aromatic heterocyclic group and the like.

Examples of the non-aromatic cyclic hydrocarbon group include, a C₃₋₁₀ cycloalkyl group, a C₃₋₁₀ cycloalkenyl group and a C₄₋₁₀ cycloalkadienyl group, each of which is optionally condensed with a benzene ring, and the like. Examples of the C₃₋₁₀ cycloalkyl group, C₃₋₁₀ cycloalkenyl group and C₄₋₁₀ cycloalkadienyl group include those exemplified as the aforementioned "substituent" for R¹, R², R³ or R⁴.

Examples of the non-aromatic heterocyclic group include those exemplified as the aforementioned "substituent" for R¹, R², R³ or R⁴.

The "cyclic group" of the "optionally substituted cyclic group" for A is preferably a C₆₋₁₄ aryl group, a C₃₋₁₀ cycloalkyl group, an aromatic heterocyclic group, a non-aromatic heterocyclic group and the like, more preferably, a C₆₋₁₄ aryl group (preferably, phenyl, naphthyl), a C₃₋₁₀ cycloalkyl group (preferably, cyclopentyl, cyclohexyl), a 5- or 6-membered aromatic heterocyclic group optionally condensed with a benzene ring (preferably, furyl, thienyl, oxazolyl, thiazolyl, isoxazolyl, oxadiazolyl, imidazolyl, pyridyl, indolyl, benzimidazolyl), a 5- or 6-membered non-aromatic heterocyclic group optionally condensed with a benzene ring (preferably, pyrrolidinyl, piperidinyl, morpholinyl, tetrahydrofuryl, tetrahydropyranyl, pyrazolidinyl, dihydrotriazolyl, dihydroisoindolyl) and the like.

The "cyclic group" of the "optionally substituted cyclic group" for A may have 1 to 3 substituents at substitutable positions. Examples of such substituent include those exemplified as the substituents that the C₃₋₁₀ cycloalkyl group and the like exemplified as the aforementioned "substituent" for R¹, R², R³ or R⁴ may have. When two or more substituents are used, the substituents may be the same or different.

Preferable examples of the substituent include
(1) a halogen atom;
(2) a cyano group;
(3) an oxo group;
(4) a C₁₋₆ alkyl group optionally substituted by 1 to 3 halogen atoms;
(5) a C₆₋₁₄ aryl group (e.g., phenyl, naphthyl) optionally substituted by 1 to 3 substituents selected from a C₁₋₆ alkyl group optionally substituted by 1 to 3 halogen atoms, a halogen atom and a C₁₋₆ alkylsulfonyloxy group (e.g., methylsulfonyloxy);
(6) a C₁₋₆ alkoxy group optionally substituted by 1 to 3 halogen atoms;
(7) an amino group optionally mono- or di-substituted by C₁₋₆ alkyl-carbonyl group(s);
(8) a C₇₋₁₃ aralkyl group (e.g., benzyl);
(9) a C₇₋₁₃ aralkyloxy group (e.g., benzyloxy);
(10) an aromatic heterocyclic group (e.g., furyl, thienyl, triazolyl);
(11) a non-aromatic heterocyclic group (e.g., morpholinyl);
and the like.

A is preferably a C₆₋₁₄ aryl group, a C₃₋₁₀ cycloalkyl group, an aromatic heterocyclic group, a non-aromatic heterocyclic group and the like, each of which is optionally substituted by 1 to 3 substituents selected from
(1) a halogen atom;
(2) a cyano group;
(3) an oxo group;
(4) a C₁₋₆ alkyl group optionally substituted by 1 to 3 halogen atoms;
(5) a C₆₋₁₄ aryl group (e.g., phenyl, naphthyl) optionally substituted by 1 to 3 substituents selected from a C₁₋₆ alkyl group optionally substituted by 1 to 3 halogen atoms, a halogen atom and a C₁₋₆ alkylsulfonyloxy group (e.g., methylsulfonyloxy);
(6) a C₁₋₆ alkoxy group optionally substituted by 1 to 3 halogen atoms;
(7) an amino group optionally mono- or di-substituted by C₁₋₆ alkyl-carbonyl group(s);
(8) a C₇₋₁₃ aralkyl group (e.g., benzyl);
(9) a C₇₋₁₃ aralkyloxy group (e.g., benzyloxy);
(10) an aromatic heterocyclic group (e.g., furyl, thienyl, triazolyl);
(11) a non-aromatic heterocyclic group (e.g., morpholinyl);
and the like,
more preferably, a C₆₋₁₄ aryl group (preferably, phenyl, naphthyl), a C₃₋₁₀ cycloalkyl group (preferably, cyclopentyl, cyclohexyl), a 5- or 6-membered aromatic heterocyclic group optionally condensed with a benzene ring (preferably, furyl, thienyl, oxazolyl, thiazolyl, isoxazolyl, oxadiazolyl, imidazolyl, pyridyl, indolyl, benzimidazolyl), a 5- or 6-membered non-aromatic heterocyclic group optionally condensed with a benzene ring (preferably, pyrrolidinyl, piperidinyl, morpholinyl, tetrahydrofuryl, tetrahydropyranyl, pyrazolidinyl, dihydrotriazolyl, dihydroisoindolyl) and the like, each of which is optionally substituted by the above-mentioned 1 to 3 substituents.

A is particularly preferably a 5- or 6-membered aromatic heterocyclic group (preferably oxazolyl, more preferably 4-oxazolyl) optionally substituted by 1 to 3 substituents selected from
(1) a C₁₋₆ alkyl group optionally substituted by 1 to 3 halogen atoms; and
(2) a C₁₋₆ alkyl group optionally substituted by 1 to 3 halogen atoms, a halogen atom and a C₁₋₆ alkylsulfonyloxy group (e.g., methylsulfonyloxy).

B is a bond or a spacer having 1 to 10 carbon atoms in the main chain.

The "main chain" here means a divalent straight chain connecting group A and group D, and the "atom number of the main chain" is counted such that the number of the atoms of the main chain becomes the minimum. The "main chain" consists of 1 to 10 atoms selected from carbon atom and hetero atom (e.g., oxygen atom, sulfur atom, nitrogen atom and the like), and may be saturated or unsaturated. In addition, the sulfur atom may be oxidized.

Specific examples of the "spacer having 1 to 10 carbon atoms in the main chain" include, saturated chains such as
(1) -(CH₂)ₖ- (wherein k is an integer of 1 to 10) (e.g., -CH₂-, -CH₂CH₂-, -CH₂CH₂CH₂- etc.);
(2) -(CH₂)ₖ₁₁-O-(CH₂)ₖ₁₂- (wherein k11 and k12 are independently an integer of 0 to 9, and k11+k12 is an integer of 1 to 9) (e.g., -OCH₂-, -CH₂O-, -OCH₂CH₂-, -CH₂CH₂O- etc.);
(3) -(CH₂)ₖ₂₁-S(O)ₖ₂₃-(CH₂)ₖ₂₂- (wherein k21 and k22 are independently an integer of 0 to 9, k21+k22 is an integer of 1 - 9, and k23 is an integer of 0 to 2) (e.g., -SCH₂-, -CH₂S-, -SCH₂CH₂-, -CH₂CH₂S-, -SOCH₂-, -CH₂SO-, -SO₂CH₂-, -CH₂SO₂- etc.);
(4) -(CH₂)ₖ₃₁-NH-(CH₂)ₖ₃₂- (wherein k31 and k32 are independently an integer of 0 to 9, and k31+k32 is an integer of 1 to 9) (e.g., -NHCH₂-, -CH₂NH-, -NHCH₂CH₂-, -CH₂CH₂NH- etc.);
(5) -(CH₂)ₖ₄₁-O-(CH₂)ₖ₄₂-NH-(CH₂)ₖ₄₃- (wherein k41, k42 and k43 are independently an integer of 0 to 8, and k41+k42+k43 is an integer of 1 to 8) (e.g., -CH₂OCH₂NHCH₂CH₂-, -CH₂OCH₂NHCH₂CH₂CH₂- etc.);
(6) -(CH₂)ₖ₅₁-NH-(CH₂)ₖ₅₂-O-(CH₂)ₖ₅₃- (wherein k51, k52 and k53 are independently an integer of 0 to 8, and k51+k52+k53 is an integer of 1 to 8) (e.g., -CH₂NHCH₂OCH₂- etc.);
(7) -(CH₂)ₖ₆₁-S-(CH₂)ₖ₆₂-NH-(CH₂)ₖ₆₃- (wherein k61, k62 and k63 are independently an integer of 0 to 8, and k61+k62+k63 is an integer of 1 to 8) (e.g., -CH₂SCH₂NHCH₂- etc.);
(8) -(CH₂)ₖ₇₁-NH-(CH₂)ₖ₇₂-S-(CH₂)ₖ₇₃- (wherein k71, k72 and k73 are independently an integer of 0 to 8, and k71+k72+k73 is an integer of 1 to 8) (e.g., -CH₂NHCH₂SCH₂- etc.);
and the like, unsaturated chains in a part or all thereof (e.g., -CH=CH-, -N=CH-, -CH=N- etc.) and the like.

The "spacer having 1 to 10 carbon atoms in the main chain" for B is preferably
(1) -(CH₂)ₖ- (preferably -CH₂-, -CH₂CH₂-, -CH₂CH₂CH₂-);
(2)-(CH₂)ₖ₁₁-O-(CH₂)ₖ₁₂- (preferably -OCH₂CH₂-);
(3)-(CH₂)ₖ₂₁-S(O)ₖ₂₃-(CH₂)ₖ₂₂- (preferably -SCH₂CH₂-);
(4) -(CH₂)ₖ₃₁-NH-(CH₂)ₖ₃₂- (preferably -NHCH₂CH₂-) ;
(5) -(CH₂)ₖ₄₁-O-(CH₂)k₄₂-NH-(CH₂)ₖ₄₃- (preferably -CH₂OCH₂NHCH₂CH₂-, -CH₂OCH₂NHCH₂CH₂CH₂-) ;
and the like, more preferably, -(CH₂)ₖ- (preferably -CH₂-, - CH₂CH₂-, -CH₂CH₂CH₂-) and the like, particularly preferably -CH₂-, -CH₂CH₂- and the like.

A carbon atom and a nitrogen atom constituting the main chain may have one or more substituents at substitutable positions. When two or more substituents are used, the substituents may be the same or different.

Examples of the "substituent" include those exemplified as the substituents that the C₃₋₁₀ cycloalkyl group and the like exemplified as the aforementioned "substituent" for R¹, R², R³ or R⁴ may have.

Preferable examples of the substituent include an oxo group, a C₁₋₆ alkyl group (preferably methyl), a C₆₋₁₄ aryl group (preferably phenyl) and the like.

B is preferably
(a) a bond;
(b)
   (1) -(CH₂)ₖ- (preferably, -CH₂-, -CH₂CH₂-, -CH₂CH₂CH₂-),
   (2) -(CH₂)ₖ₁₁-O-(CH₂)ₖ₁₂- (preferably, -OCH₂CH₂-),
   (3) -(CH₂)ₖ₂₁-S(O)ₖ₂₃-(CH₂)ₖ₂₂- (preferably -SCH₂CH₂),
   (4) -(CH₂)ₖ₃₁-NH-(CH₂)ₖ₃₂- (preferably, -NHCH₂CH₂-),
   (5) -(CH₂)ₖ₄₁-O-(CH₂)ₖ₄₂-NH-(CH₂)ₖ₄₃- (preferably, -CH₂OCH₂NHCH₂CH₂-, -CH₂OCH₂NHCH₂CH₂CH₂-),
   each of which is optionally substituted by 1 to 3 substituents selected from an oxo group, a C₁₋₆ alkyl group, a C₆₋₁₄ aryl group and the like;
   and the like, more preferably
   (a) a bond;
   (b) -(CH₂)ₖ- (preferably, -CH₂-, -CH₂CH₂-, -CH₂CH₂CH₂-) optionally substituted by 1 to 3 substituents selected from an oxo group, a C₁₋₆ alkyl group, a C₆₋₁₄ aryl group, etc.;
and the like.

B is particularly preferably -CH₂-.

D is O or NR⁵ wherein R⁵ is a hydrogen atom or a substituent.

Examples of the "substituent" for R⁵ include those similar to the "substituent" for R¹, R², R³ or R⁴.

R⁵ is preferably a C₁₋₆ alkyl group and the like.

D is preferably O.

X is an optionally further substituted aromatic ring.

The "aromatic ring" of the "optionally further substituted aromatic ring" is substituted by a group -D- and a group -E-, and is optionally further substituted. Examples of the "aromatic ring" of the "optionally further substituted aromatic ring" include an aromatic hydrocarbon, an aromatic heterocycle and the like.

Examples of the aromatic hydrocarbon include a C₆₋₁₄ arene and the like. Examples of the C₆₋₁₄ arene include a ring constituting the C₆₋₁₄ aryl group exemplified as the aforementioned "substituent" for R¹, R², R³ or R⁴.

Examples of the aromatic heterocycle include a ring constituting the aromatic heterocyclic group exemplified as the aforementioned "substituent" for R¹, R², R³ or R⁴.

The "aromatic ring" of the "optionally further substituted aromatic ring" for X is preferably a C₆₋₁₄ arene (preferably, benzene), a 6-membered aromatic heterocycle (preferably, pyridine) and the like, more preferably, benzene, pyridine and the like.

The "aromatic ring" of the "optionally further substituted aromatic ring" for X may have 1 to 3 substituents at substitutable positions. Examples of such substituent include those exemplified as the substituents that the C₃₋₁₀ cycloalkyl group and the like exemplified as the aforementioned "substituent" for R¹, R², R³ or R⁴ may have. When two or more substituents are used, the substituents may be the same or different.

X is preferably a C₆₋₁₄ arene (preferably, benzene), a 6-membered aromatic heterocycle (preferably, pyridine) and the like, each of which is optionally substituted by 1 to 3 C₁₋₆ alkyl groups, more preferably, benzene, pyridine and the like.

E is CO, C(OR⁶)R⁷ or C=NR⁸ wherein R⁶, R⁷ and R⁸ are the same or different and each is a hydrogen atom or a substituent.

Examples of the "substituent" for R⁶, R⁷ or R⁸ include those similar to the "substituent" for R¹, R², R³ or R⁴.

R⁶ is preferably a hydrogen atom, a C₁₋₆ alkyl group and the like.

R⁷ is preferably a hydrogen atom, a C₁₋₆ alkyl group and the like.

R⁸ is preferably a hydroxy group, a C₁₋₆ alkoxy group and the like.

E is preferably CO or C(OR⁶)R⁷ wherein R⁶ and R⁷ are the same or different and each is a hydrogen atom or a substituent, more preferably CO or C(OR⁶)R⁷ wherein R⁶ and R⁷ are the same or different and each is a hydrogen atom or a C₁₋₆ alkyl group, particularly preferably CO.

G is an optionally substituted divalent C₁₋₆ hydrocarbon group.

Examples of the "divalent C₁₋₆ hydrocarbon group" of the "optionally substituted divalent C₁₋₆ hydrocarbon group" for G include a "divalent acyclic C₁₋₆ hydrocarbon group", a "divalent cyclic C₃₋₆ hydrocarbon group", and a divalent C₄₋₆ hydrocarbon group obtained by combining one or more kinds of "divalent acyclic C₁₋₆ hydrocarbon groups" and one or more kinds of "divalent cyclic C₃₋₆ hydrocarbon groups".

Here, examples of the "divalent acyclic C₁₋₆ hydrocarbon group" include a C₁₋₆ alkylene group, a C₂₋₆ alkenylene group, a C₂₋₆ alkynylene group and the like. Specific examples include the following.
(1) a C₁₋₆ alkylene group (e.g., -CH₂-, -(CH₂)₂-, -(CH₂)₃-, -(CH₂)₄-, -(CH₂)₅-, -(CH₂)₆-, -CH(CH₃)-, -C(CH₃)₂-, -CH(C₂H₅)-, -CH(C₃H₇)-, -CH(CH(CH₃)₂)-, -(CH(CH₃))₂-, -(CH₂)₂C(CH₃)₂-, -(CH₂)₃C(CH₃)₂- and the like);
(2) a C₂₋₆ alkenylene group (e.g., -CH=CH-, -CH₂-CH=CH-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂- and the like); and
(3) a C₂₋₆ alkynylene group (e.g., -C≡C-, -CH₂-C≡C-, -CH₂-C≡C-CH₂-CH₂- and the like).

Examples of the "divalent cyclic C₃₋₆ hydrocarbon group" include a C₃₋₆ cycloalkylene group, a C₃₋₆ cycloalkenylene group, a phenylene group and the like. Examples of the C₃₋₆ cycloalkylene group and C₃₋₆ cycloalkenylene group include, from among the C₃₋₁₀ cycloalkyl group and C₃₋₁₀ cycloalkenyl group exemplified as the aforementioned "substituent" for R¹, R², R³ or R⁴, a divalent group having 3 to 6 carbon atoms obtained by removing any one hydrogen atom, and the like.

Specific examples of the "divalent cyclic C₃₋₆ hydrocarbon group" include 1,1-cyclopropylene, 1,2-cyclopropylene, 1,1-cyclobutylene, 1,2-cyclobutylene, 1,1-cyclopentylene, 1,2-cyclopentylene, 1,3-cyclopentylene, 1,1-cyclohexylene, 1,2-cyclohexylene, 1,3-cyclohexylene, 1,4-cyclohexylene, 3-cyclohexen-1,4-ylene, 3-cyclohexen-1,2-ylene, 2,5-cyclohexadien-1,4-ylene, 1,2-phenylene, 1,3-phenylene, 1,4-phenylene and the like.

The "divalent C₁₋₆ hydrocarbon group" of the "optionally substituted divalent C₁₋₆ hydrocarbon group" is preferably a C₁₋₆ alkylene group (preferably, -CH₂-, -(CH₂)₂-, -CH(CH₃)-, -C(CH₃)₂-, -CH(C₂H₅)-, -CH(C₃H₇)-, -CH(CH(CH₃)₂)-), a C₃₋₆ cycloalkylene group (preferably, 1,1-cyclobutylene) and the like, more preferably, a C₁₋₆ alkylene group (preferably, -CH₂-, -(CH₂)₂-, -CH(CH₃)-, -C(CH₃)₂-, -CH(C₂H₅)-, -CH(C₃H₇)-, -CH(CH(CH₃)₂)-) and the like.

The "divalent C₁₋₆ hydrocarbon group" of the "optionally substituted divalent C₁₋₆ hydrocarbon group" for G may have 1 to 3 substituents at substitutable positions. Examples of such substituent include those exemplified as the substituents that C₁₋₁₀ alkyl group and the like exemplified as the aforementioned "substituent" for R¹, R², R³ or R⁴ may have. When two or more substituents are used, the substituents may be the same or different.

Preferable examples of the substituent include a halogen atom (preferably, fluorine atom), a C₆₋₁₄ aryl group (preferably, phenyl) and the like.

G is preferably a C₁₋₆ alkylene group (preferably, -CH₂-, -(CH₂)₂-, -CH(CH₃)-, -C(CH₃)₂-, -CH(C₂H₅)-, -CH(C₃H₇)-, - CH(CH(CH₃)₂)-), a C₃₋₆ cycloalkylene group (preferably, 1,1-cyclobutylene) and the like, each of which is optionally substituted by 1 to 3 substituents selected from a halogen atom (preferably, fluorine atom), a C₆₋₁₄ aryl group (preferably, phenyl) and the like, more preferably, a C₁₋₆ alkylene group (preferably, -CH₂-, -(CH₂)₂-, -CH(CH₃)-, -C(CH₃)₂-, -CH(C₂H₅)-, -CH(C₃H₇)-, -CH(CH(CH₃)₂)-) and the like, which are optionally substituted by the above-mentioned substituent.

G is particularly preferably -CH₂-, -CH(CH₃)- or -C(CH₃)₂-.

R is -OR⁹ wherein R⁹ is a hydrogen atom or an optionally substituted hydrocarbon group, or -NR¹⁰R¹¹ wherein R¹⁰ and R¹¹ are the same or different and each is a hydrogen atom, an optionally substituted hydrocarbon group or an optionally substituted heterocyclic group, or R¹⁰ and R¹¹ form, together with the adjacent nitrogen atom, an optionally substituted heterocycle.

Examples of the "optionally substituted hydrocarbon group" for R⁹ include those similar to the "optionally substituted hydrocarbon group" exemplified as the "substituent" for R¹, R², R³ or R⁴.

R⁹ is preferably a hydrogen atom, a C₁₋₆ alkyl group (preferably, methyl, ethyl, propyl, tert-butyl), a C₂₋₆ alkenyl group (preferably, allyl) and the like, more preferably, a hydrogen atom.

Examples of the "optionally substituted hydrocarbon group" and "optionally substituted heterocyclic group" for R¹⁰ or R¹¹ include those similar to the "optionally substituted hydrocarbon group" and "optionally substituted heterocyclic group", each of which is exemplified as the "substituent" for R¹, R², R³ or R⁴.

Examples of the "optionally substituted heterocycle" formed by R¹⁰ and R¹¹ together with the adjacent nitrogen atom include those exemplified as the aforementioned "optionally substituted nitrogen-containing heterocycle" formed by R^{a}' or R^{b}' together with the adjacent nitrogen atom.

R¹⁰ and R¹¹ are each preferably a hydrogen atom, a C₁₋₆ alkyl group (preferably methyl) and the like.

R is preferably -OR⁹ wherein R⁹ is a hydrogen atom or an optionally substituted hydrocarbon group, and the like, more preferably, a hydroxy group, a C₁₋₆ alkoxy group (preferably, methoxy, ethoxy, propoxy, tert-butoxy), a C₂₋₆ alkenyloxy group (preferably, allyloxy) and the like, more preferably a hydroxy group and the like.

Preferable examples of compound (I) include the following compounds.

### [Compound A]

Compound (I) wherein
A is a C₆₋₁₄ aryl group (preferably, phenyl, naphthyl), a C₃₋₁₀ cycloalkyl group (preferably, cyclopentyl, cyclohexyl), a 5- or 6-membered aromatic heterocyclic group optionally condensed with a benzene ring (preferably, furyl, thienyl, oxazolyl, thiazolyl, isoxazolyl, oxadiazolyl, imidazolyl, pyridyl, indolyl, benzimidazolyl), or a 5- or 6-membered non-aromatic heterocyclic group optionally condensed with a benzene ring (preferably, pyrrolidinyl, piperidinyl, morpholinyl, tetrahydrofuryl, tetrahydropyranyl, pyrazolidinyl, dihydrotriazolyl, dihydroisoindolyl), each of which is optionally substituted by 1 to 3 substituents selected from
(1) a halogen atom,
(2) a cyano group,
(3) an oxo group,
(4) a C₁₋₆ alkyl group optionally substituted by 1 to 3 halogen atoms,
(5) a C₆₋₁₄ aryl group (e.g., phenyl, naphthyl) optionally substituted by 1 to 3 substituents selected from a C₁₋₆ alkyl group optionally substituted by 1 to 3 halogen atoms, a halogen atom and a C₁₋₆ alkylsulfonyloxy group (e.g., methylsulfonyloxy),
(6) a C₁₋₆ alkoxy group optionally substituted by 1 to 3 halogen atoms,
(7) an amino group optionally mono- or di-substituted by C₁₋₆ alkyl-carbonyl group(s),
(8) a C₇₋₁₃ aralkyl group (e.g., benzyl),
(9) a C₇₋₁₃ aralkyloxy group (e.g., benzyloxy),
(10) an aromatic heterocyclic group (e.g., furyl, thienyl, triazolyl), and
(11) a non-aromatic heterocyclic group (e.g., morpholinyl);
   B is
   (a) a bond, or
   (b)
      (1) -(CH₂)ₖ- (preferably, -CH₂-, -CH₂CH₂-, -CH₂CH₂CH₂-),
      (2) -(CH₂)ₖ₁₁-O-(CH₂)ₖ₁₂- (preferably, -OCH₂CH₂-),
      (3) -(CHz)ₖ₂₁-S(O)ₖ₂₃-(CH₂)ₖ₂₂- (preferably -SCH₂CH₂-),
      (4) -(CH₂)ₖ₃₁-NH-(CH₂)ₖ₃₂- (preferably, -NHCH₂CH₂-), or
      (5) -(CH₂)ₖ₄₁-O-(CH₂)ₖ₄₂-NH-(CH₂)ₖ₄₃- (preferably, -CH₂OCH₂NHCH₂CH₂-, -CH₂OCH₂NHCH₂CH₂CH₂-),
each of which is optionally substituted by 1 to 3 substituents selected from an oxo group, a C₁₋₆ alkyl group and a C₆₋₁₄ aryl group;
D is O;
X is a C₆₋₁₄ arene (preferably, benzene) or a 6-membered aromatic heterocycle (preferably, pyridine), each of which is optionally substituted by 1 to 3 C₁₋₆ alkyl groups;
E is CO or C(OR⁶)R⁷ wherein R⁶ and R⁷ are the same or different and each is a hydrogen atom or a C₁₋₆ alkyl group;
G is a C₁₋₆ alkylene group (preferably, -CH₂-, -(CH₂)₂-, -CH(CH₃)-, -C(CH₃)₂-, -CH(C₂H₅)-, -CH(C₃H₇)-, -CH(CH(CH₃)₂)-) optionally substituted by 1 to 3 substituents selected from a halogen atom (preferably, fluorine atom) and a C₆₋₁₄ aryl group (preferably, phenyl);
R is a hydroxy group, a C₁₋₆ alkoxy group (preferably, methoxy, ethoxy, propoxy, tert-butoxy) or a C₂₋₆ alkenyloxy group (preferably, allyloxy); and
R¹, R², R³ and R⁴ are the same or different and each is a hydrogen atom, a C₁₋₆ alkoxy group optionally substituted by C₁₋₆ alkoxy group(s) (preferably, methoxy, ethoxy, methoxymethoxy), or a C₁₋₆ alkyl group (preferably, methyl).

### [Compound B]

(5-methoxy-2-{4-[(5-methyl-2-phenyl-1,3-oxazol-4-yl)methoxy]benzoyl}phenoxy)acetic acid (compound of Example 1),
2-(5-methoxy-2-{4-[(5-methyl-2-phenyl-1,3-oxazol-4-yl)methoxy]benzoyl}phenoxy)-2-methylpropanoic acid (compound of Example 14),
2-[5-methoxy-2-({6-[(5-methyl-2-phenyl-1,3-oxazol-4-yl)methoxy]pyridin-3-yl}carbonyl)phenoxy]propanoic acid (compound of Example 21),
2-(2-{4-[(5-methyl-2-phenyl-1,3-oxazol-4-yl)methoxy]benzoyl}phenoxy)propanoic acid (compound of Example 22), and
(2R)-2-(5-methoxy-2-{4-[(5-methyl-2-phenyl-1,3-oxazol-4-yl)methoxy]benzoyl}phenoxy)propanoic acid (compound of Example 53).

When compound (I) forms a salt, the salt is preferably a pharmacologically acceptable salt. Examples thereof include salts with inorganic base, salts with organic base, salts with inorganic acid, salts with organic acid, salts with basic or acidic amino acid and the like.

Preferable examples of the salts with inorganic base include alkali metal salts such as sodium salt, potassium salt and the like; alkaline earth metal salts such as calcium salt, magnesium salt and the like; aluminum salt, ammonium salt and the like.

Preferable examples of the salt with organic base include salts with trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine, N,N'-dibenzylethylenediamine and the like.

Preferable examples of the salt with inorganic acid include salts with hydrochloric acid, hydrobromic acid, nitric acid, sulfuric acid, phosphoric acid and the like.

Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include salts with arginine, lysine, ornithine and the like.

Preferable examples of the salt with acidic amino acid include salts with aspartic acid, glutamic acid and the like.

The prodrug of compound (I) is a compound which is converted to compound (I) with a reaction due to an enzyme, gastric acid, etc. under the physiological condition in the living body, that is, a compound which is converted to compound (I) by enzymatic oxidation, reduction, hydrolysis, etc.; a compound which is converted to compound (I) by hydrolysis etc. due to gastric acid, and the like. A prodrug of compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, tetrahydropyranylation, pyrrolidylmethylation, pivaloyloxymethylation or tert-butylation); a compound obtained by subjecting a hydroxy group in compound (I) to an acylation, alkylation, phosphorylation or boration (e.g., a compound obtained by subjecting an hydroxy group in compound (I) to an acetylation, palmitoylation, propanoylation, pivaloylation, succinylation, fumarylation, alanylation, dimethylaminomethylcarbonylation, or tetrahydropyranylation); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation) and the like. Any of these compounds can be produced from compound (I) by a method known per se.

A prodrug of compound (I) may be a compound that converts to compound (I) under physiological conditions as described in Development of Pharmaceutical Products, vol. 7, Molecule Design, 163-198, Hirokawa Shoten (1990).

The compound (I) may be in the form of a crystal, and the crystal may have single or plural crystal forms. The crystal can be produced by a crystallization method known *per se*. In the present specification, the melting point is measured using, for example, a micro melting point measurement apparatus (Yanaco, MP-500D or Buchi, B-545) or a DSC (differential scanning calorimetry) device (SEIKO, EXSTAR6000) and the like.

In general, the melting points vary depending on the measurement apparatuses, the measurement conditions and the like. The crystal in the present specification may show different values from the melting point described in the present specification, as long as they are within a general error range.

The crystal of compound (I) is superior in the physicochemical properties (melting point, solubility, stability etc.) and biological properties (pharmacokinetics (absorption, distribution, metabolism, excretion), efficacy expression, etc.), and therefore, it is extremely useful as a medicament.

The compound (I) may be a solvate (e.g., hydrate, etc.) or a non-solvate, both of which are encompassed in compound (I).

A compound labeled with an isotope (e.g., ³H, ¹⁴C, ³⁵S, ¹²⁵I) and the like are also encompassed in compound (I).

The compound (I) or a prodrug thereof (hereinafter sometimes to be simply abbreviated as the compound of the present invention) shows low toxicity (e.g., acute toxicity, chronic toxicity, genetic toxicity, reproductive toxicity, cardiotoxicity, drug interaction, carcinogenicity), and can be used as it is or as a pharmaceutical composition in admixture with a commonly known pharmaceutically acceptable carrier etc., as an agent for the prophylaxis or treatment of the below-mentioned various disease in mammals (e.g., humans, mice, rats, rabbits, dogs, cats, bovines, horses, pigs, monkeys).

Here, as the pharmacologically acceptable carrier, various organic or inorganic carrier substances conventionally used as a preparation material can be used. They are incorporated as excipient, lubricant, binder and disintegrant for solid preparations; solvent, solubilizing agents, suspending agent, isotonicity agent, buffer and soothing agent for liquid preparations and the like. Where necessary, preparation additives such as preservatives, antioxidants, colorants, sweetening agents and the like can be used.

Preferable examples of the excipient include lactose, sucrose, D-mannitol, D-sorbitol, starch, α-starch, dextrin, crystalline cellulose, low-substituted hydroxypropylcellulose, sodium carboxymethylcellulose, gum arabic, pullulan, light anhydrous silicic acid, synthetic aluminum silicate, magnesium aluminometasilicate and the like.

Preferable examples of the lubricant include magnesium stearate, calcium stearate, talc, colloidal silica and the like.

Preferable examples of the binder include α-starch, saccharose, gelatin, gum arabic, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone and the like.

Preferable examples of the disintegrant include lactose, sucrose, starch, carboxymethylcellulose, carboxymethylcellulose calcium, croscarmellose sodium, carboxymethylstarch sodium, light anhydrous silicic acid, low-substituted hydroxypropylcellulose and the like.

Preferable examples of the solvent include water for injection, physiological brine, Ringer solution, alcohol, propylene glycol, polyethylene glycol, sesame oil, corn oil, olive oil, cottonseed oil and the like.

Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, laurylaminopropionic acid, lecithin, benzalkonium chloride, benzethonium chloride, glycerol monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like; polysorbates, polyoxyethylene hydrogenated castor oil and the like.

Preferable examples of the isotonicity agent include sodium chloride, glycerin, D-mannitol, D-sorbitol, glucose and the like.

Preferable examples of the buffer include buffers such as phosphate, acetate, carbonate, citrate, etc. and the like.

Preferable examples of the soothing agent include benzyl alcohol and the like.

Preferable examples of the preservative include p-oxybenzoates, chlorobutanol, benzyl alcohol, phenethyl alcohol, dehydroacetic acid, sorbic acid and the like.

Preferable examples of the antioxidant include sulfite, ascorbate and the like.

Preferable examples of the colorant include aqueous food tar colors (e.g., food colors such as Food Red Nos. 2 and 3, Food Yellow Nos. 4 and 5, Food Blue Nos. 1 and 2 and the like), water insoluble lake dye (e.g., aluminum salts of the aforementioned aqueous food tar colors), natural dyes (e.g., β-carotene, chlorophyll, red iron oxide) and the like.

Preferable examples of the sweetening agent include saccharin sodium, dipotassium glycyrrhizinate, aspartame, stevia and the like.

Examples of the dosage form of the aforementioned pharmaceutical composition include oral preparation such as tablets (including sublingual tablet, orally disintegrating tablet), capsules (including soft capsule, microcapsule), granule, powder, troche, syrup, emulsion, suspension and the like; and parenteral preparation such as injections (e.g., subcutaneous injection, intravenous injection, intramuscular injection, intraperitoneal injection, drip infusion), external preparations (e.g., dermal preparation, ointment), suppositories (e.g., rectal suppository, vaginal suppository), pellets, transnasal preparations, pulmonary preparations (inhalant), eye drops and the like. They can be safely administered orally or parenterally.

These preparations may be controlled-release preparations (e.g., sustained-release microcapsule) such as immediate-release preparation, sustained-release preparation and the like.

The pharmaceutical composition can be produced by a method conventionally used in the technical field of formulation of preparations, such as a method described in the Japanese Pharmacopoeia and the like.

While the content of the compound of the present invention in the pharmaceutical composition varies depending on the dosage form, the dose of the compound of the present invention and the like, it is, for example, about 0.1 to 100 wt%.

The compound of the present invention can be used as an insulin sensitizer, an agent for enhancing insulin sensitivity, a retinoid-related receptor function regulator, a peroxisome proliferator-activated receptor ligand, a retinoid X receptor ligand and the like. The function regulator here means both agonists and antagonists.

The compound of the present invention has hypoglycemic action, hypolipidemic action, insulin sensitizing activity, insulin sensitivity enhancing action and peroxiosome proliferator-activated receptor (hereinafter, sometimes to be abbreviated as PPAR)γ (GenBank Accession No. L40904) partial agonist action. Here, PPAR γ may form heterodimeric receptor with any of retinoid X receptor (hereinafter, sometimes to be abbreviated as RXR)α (GenBank Accession No. X52773), RXRβ (GenBank Accession No. M84820) or RXRγ (GenBank Accession No. U38480).

The compound of the present invention specifically has a selective partial agonist action on PPAR γ.

It is reported that, compared with full agonist to PPAR γ (e.g., thiazolidinedione compound), selective partial agonist to PPAR γ is not accompanied by side effects such as body weight gain, adipocyte accumulation, cardiac hypertrophy and the like (Molecular Endocrinology, vol. 17, column 4, page 662, 2003). Accordingly, the compound of the present invention is useful, in comparison with full agonist to PPAR γ, as a hypoglycemic agent unaccompanied by side effects such as body weight gain, adipocyte accumulation, cardiac hypertrophy and the like.

The compound of the present invention can be used, for example, as an agent for the prophylaxis or treatment of diabetes (e.g., type-1 diabetes, type-2 diabetes, gestational diabetes, obese diabetes); an agent for the prophylaxis or treatment of hyperlipidemia (e.g., hypertriglyceridemia, hypercholesterolemia, hypo-HDL-emia, postprandial hyperlipidemia); insulin sensitizer; an agent for enhancing insulin sensitivity; an agent for the prophylaxis or treatment of impaired glucose tolerance (IGT); and an agent for preventing progress of impaired glucose tolerance into diabetes.

For diagnostic criteria of diabetes, Japan Diabetes Society reported new diagnostic criteria.

According to this report, diabetes is a condition showing any of a fasting blood glucose level (glucose concentration of intravenous plasma) of not less than 126 mg/dl, a 75 g oral glucose tolerance test (75 g OGTT) 2 h level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 h level (glucose concentration of intravenous plasma) of less than 140 mg/dl" (normal type) is called a "borderline type".

In addition, ADA (American Diabetes Association) and WHO reported new diagnostic criteria of diabetes.

According to these reports, diabetes is a condition showing a fasting blood glucose level (glucose concentration of intravenous plasma) of not less than 126 mg/dl and a 75 g oral glucose tolerance test 2 h level (glucose concentration of intravenous plasma) of not less than 200 mg/dl.

According to the above-mentioned reports of ADA and WHO, impaired glucose tolerance is a condition showing a 75 g oral glucose tolerance test 2 h level (glucose concentration of intravenous plasma) of not less than 140 mg/dl and less than 200 mg/dl. According to the report of ADA, a condition showing a fasting blood glucose level (glucose concentration of intravenous plasma) of not less than 100 mg/dl and less than 126 mg/dl is called IFG (Impaired Fasting Glucose). On the other hand, WHO defines the IFG (Impaired Fasting Glucose) to be a condition showing a fasting blood glucose level (glucose concentration of intravenous plasma) of not less than 110 mg/dl and less than 126 mg/dl, and calls it IFG (Impaired Fasting Glycaemia).

The compound of the present invention can also be used as an agent for the prophylaxis or treatment of diabetes, borderline type, impaired glucose tolerance, IFG (Impaired Fasting Glucose) and IFG (Impaired Fasting Glycaemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycaemia) into diabetes.

The compound of the present invention can also be used as an agent for the prophylaxis or treatment of, for example, diabetic complications [e.g., neuropathy, nephropathy, retinopathy, cataract, macroangiopathy, osteopenia, hyperosmolar diabetic coma, infectious disease (e.g., respiratory infection, urinary tract infection, gastrointestinal infection, dermal soft tissue infections, inferior limb infection), diabetic gangrene, xerostomia, hypacusis, cerebrovascular disorder, peripheral blood circulation disorder], obesity, osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, hypertension, polycystic ovary syndrome, kidney disease (e.g., diabetic nephropathy, glomerular nephritis, glomerulosclerosis, nephrotic syndrome, hypertensive nephrosclerosis, end stage kidney disease), muscular dystrophy, myocardial infarction, angina pectoris, cerebrovascular accident (e.g., cerebral infarction, cerebral apoplexy), insulin resistance syndrome, Syndrome X, metabolic syndrome (pathology having three or more selected from hypertriglyceridemia (TG), hypoHDL cholesterolemia (HDL-C), hypertension, abdomen overweight and impaired glucose tolerance), hyperinsulinemia, hyperinsulinemia-induced sensory disorder, tumor (e.g., leukemia, breast cancer, prostate cancer, skin cancer), irritable bowel syndrome, acute or chronic diarrhea, inflammatory diseases (e.g., arteriosclerosis (e.g., atherosclerosis, etc.), chronic rheumatoid arthritis, spondylitis deformans, osteoarthritis, lumbago, gout, postoperative or traumatic inflammation, swelling, neuralgia, pharyngolaryngitis, cystitis, hepatitis (inclusive of nonalcoholic steatohepatitis), pneumonia, pancreatitis, inflammatory bowel disease, ulcerative colitis, visceral obesity syndrome, and the like.

In addition, the compound of the present invention can also be used for ameliorating the conditions such as abdominal pain, nausea, vomiting, discomfort in the upper abdomen and the like, which are associated with peptic ulcer, acute or chronic gastritis, biliary dyskinesia, cholecystitis and the like, and the like.

The compound of the present invention can also be used as an agent for the prophylaxis or treatment of inflammatory disease involving TNF-α. Here, the inflammatory disease involving TNF-α is an inflammatory disease developed by the presence of TNF-α, which can be treated via a TNF-α inhibitory effect. As such inflammatory disease, for example, diabetic complications (e.g., retinopathy, nephropathy, neuropathy, macroangiopathy), chronic rheumatoid arthritis, spondylitis deformans, osteoarthritis, lumbago, gout, postoperative or traumatic inflammation, swelling, neuralgia, pharyngolaryngitis, cystitis, hepatitis, pneumonia, stomach mucous membrane injury (including stomach mucous membrane injury caused by aspirin) and the like can be mentioned.

The compound of the present invention has an apoptosis inhibitory action and can also be used as an agent for the prophylaxis or treatment of diseases involving promotion of apoptosis. As the disease involving promotion of apoptosis, for example, viral diseases (e.g., AIDS, fulminant hepatitis), neurodegenerative diseases (e.g., Alzheimer's disease, Parkinson's disease, amyotrophic lateral sclerosis, pigmentosa, cerebellar degeneration), myelodysplasia (e.g., aplastic anemia), ischemic diseases (e.g., myocardial infarction, cerebral apoplexy), hepatic diseases (e.g., alcoholic hepatitis, hepatitis B, hepatitis C), articular diseases (e.g., osteoarthritis), atherosclerosis and the like can be mentioned.

The compound of the present invention can also be used for reduction of visceral fat, inhibition of visceral fat accumulation, glycometabolism improvement, lipometabolism improvement, insulin resistance improvement, oxidized LDL production inhibition, lipoprotein metabolism improvement, coronary metabolism improvement, prophylaxis or treatment of cardiovascular complications, prophylaxis or treatment of heart failure complications, decrease of blood remnant, prophylaxis or treatment of anovulation, prophylaxis or treatment of hirsutism, prophylaxis or treatment of hyperandrogenemia and the like.

The compound of the present invention can also be used as secondary prevention and suppression of progression of the above-mentioned various diseases (e.g., cardiovascular event such as myocardial infarction and the like).

While the dose of the compound of the present invention varies depending on the administration subject, administration route, target disease, condition and the like, for example, it is generally about 0.005 to 50 mg/kg body weight, preferably 0.01 to 2 mg/kg body weight, more preferably 0.025 to 0.5 mg/kg body weight, for oral administration to adult diabetic patients, which is desirably administered in one to three portions a day.

The compound of the present invention can be used in combination with pharmaceutical agents (hereinafter to be abbreviated as combination drug) such as therapeutic agents for diabetes, therapeutic agents for diabetic complications, therapeutic agents for hyperlipidemia, antihypertensive agents, antiobesity agents, diuretics, chemotherapeutic agents, immunotherapeutic agents, antithrombotic agents, therapeutic agents for osteoporosis, antidementia agents, erectile dysfunction ameliorating agents, therapeutic agents for urinary incontinence or pollakiuria, therapeutic agents for dysuria and the like. These combination drugs may be low-molecular-weight compounds, or high-molecular-weight protein, polypeptide, antibody, vaccine and the like.

The administration time of the compound of the present invention and the combination drug is not restricted, and these can be administered to an administration subject simultaneously, or may be administered at staggered times.

As the administration mode of the compound of the present invention and the combination drug, the following methods can be mentioned: (1) The compound of the present invention and the combination drug are simultaneously formulated to give a single preparation which is administered. (2) The compound of the present invention and the combination drug are separately formulated to give two kinds of preparations which are administered simultaneously by the same administration route. (3) The compound of the present invention and the combination drug are separately formulated to give two kinds of preparations which are administered by the same administration route at staggered times. (4) The compound of the present invention and the combination drug are separately formulated to give two kinds of preparations which are administered simultaneously by the different administration routes. (5) The compound of the present invention and the combination drug are separately formulated to give two kinds of preparations which are administered by the different administration routes at staggered times (e.g., the compound of the present invention and the combination drug are administered in this order, or in the reverse order), and the like.

The dose of the combination drug can be appropriately determined based on the dose employed clinically. The mixing ratio of the compound of the present invention and a combination drug can be appropriately determined depending on the administration subject, administration route, target disease, symptom, combination and the like. When the administration subject is human, for example, a combination drug can be used in 0.01 to 100 parts by weight relative to 1 part by weight of the compound of the present invention.

As the therapeutic agents for diabetes, insulin preparations (e.g., animal insulin preparations extracted from pancreas of bovine and swine; human insulin preparations genetically synthesized using *Escherichia coli*, yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably maleate), Netoglitazone (MC-555), Rivoglitazone (CS-011), FK-614, the compound described in WO01/38325, Tesaglitazar (AZ-242), Ragaglitazar (NN-622), Muraglitazar (BMS-298585), Edaglitazone (BM-13-1258), Metaglidasen (MBX-102), Naveglitazar (LY-519818), MX-6054, LY-510929, AMG131(T-131) or a salt thereof, THR-0921), α-glucosidase inhibitors (e.g., voglibose, acarbose, miglitol, emiglitate), biguanides (e.g., phenformin, metformin, buformin or a salt thereof (e.g., hydrochloride, fumarate, succinate)), insulin secretagogues [sulfonylurea agent (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or calcium salt hydrate thereof], dipeptidyl peptidase IV inhibitors (e.g., Vidagliptin (LAF237), P32/98, Sitagliptin (MK-431), P93/01, PT-100, Saxagliptin (BMS-477118), T-6666, TS-021), β3 agonists (e.g., AJ-9677), GPR40 agonists, GLP-1 receptor agonists [e.g., GLP-1, GLP-1MR agent, NN-2211, AC-2993 (exendin-4), BIM-51077, Aib(8,35)hGLP-1(7,37)NH₂, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095), 11β-hydroxysteroid dehydrogenase inhibitors (e.g., BVT-3498), adiponectin or agonists thereof, IKK inhibitors (e.g., AS-2868), leptin resistance improving drugs, somatostatin receptor agonists (compounds described in WO01/25228, WO03/42204, WO98/44921, WO98/45285, WO99/22735 etc.), glucokinase activators (e.g., Ro-28-1675) and the like can be mentioned.

Examples of the therapeutic agents for diabetic complications include aldose reductase inhibitors (e.g., Tolrestat, Epalrestat, Zenarestat, Zopolrestat, Minalrestat, Fidarestat, CT-112, ranirestat (AS-3201)), neurotrophic factors and increasing drugs thereof (e.g., NGF, NT-3, BDNF, neurotrophin production/secretion promoting agents described in WO01/14372 (e.g., 4-(4-chlorophenyl)-2-(2-methyl-1-imidazolyl)-5-[3-(2-methylphenoxy)propyl]oxazole), PKC inhibitors (e.g., ruboxistaurin mesylate)), AGE inhibitors (e.g., ALT946, pimagedine, N-phenacylthiazolium bromide (ALT-766), EXO-226, Pyridorin, Pyridoxamine), active oxygen scavengers (e.g., thioctic acid), cerebral vasodilators (e.g., tiapuride, mexiletine), somatostatin receptor agonist (e.g., BIM23190), apoptosis signal regulating kinase-1 (ASK-1) inhibitors and the like.

Examples of the therapeutic agents for hyperlipidemia include HMG-CoA reductase inhibitors (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe), Eflucimibe)), anion exchange resins (e.g., colestyramine), probucol, nicotinic acid drugs (e.g., nicomol, niceritrol)), ethyl icosapentate, plant sterol (e.g., soysterol), γ-oryzanol) and the like.

Examples of the antihypertensive agents include angiotensin converting enzyme inhibitors (e.g., captopril, enalapril, delapril), angiotensin II antagonists (e.g., candesartan cilexetil, losartan, eprosartan, valsartan, telmisartan, irbesartan, olmesartan medoxomil, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium channel blockers (e.g., manidipine, nifedipine, nicardipine, amlodipine, efonidipine), potassium channel openers (e.g., levcromakalim, L-27152, AL0671, NIP-121), clonidine and the like.

Examples of the antiobesity agents include antiobesity agents acting on the central nervous system (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonists; 11β-hydroxysteroid dehydrogenase inhibitors (e.g., BVT-3498)), pancreatic lipase inhibitors (e.g., orlistat, ATL-962), β3 agonists (e.g., AJ-9677), peptide anorexiants (e.g., leptin, CNTF (Ciliary Neurotropic Factor)), cholecystokinin agonists (e.g., lintitript, FPL-15849), feeding deterrents (e.g., P-57) and the like.

Examples of the diuretics include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, bentylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.

Examples of the chemotherapeutic agents include alkylating agents (e.g., cyclophosphamide, ifosfamide), metabolic antagonists (e.g., methotrexate, 5-fluorouracil and a derivative thereof), antitumor antibiotics (e.g., mitomycin, adriamycin), plant-derived antitumor agent (e.g., vincristine, vindesine, Taxol), cisplatin, carboplatin, etoposide and the like. Of these, Furtulon or NeoFurtulon, which are 5-fluorouracil derivatives, and the like are preferable.

Examples of the immunotherapeutic agents include microorganism or bacterial components (e.g., muramyl dipeptide derivative, Picibanil), polysaccharides having immunity potentiating activity (e.g., lentinan, schizophyllan, krestin), cytokines obtained by genetic engineering techniques (e.g., interferon, interleukin (IL)), colony stimulating factors (e.g., granulocyte colony stimulating factor, erythropoietin) and the like, with preference given to interleukins such as IL-1, IL-2, IL-12 and the like.

Examples of the antithrombotic agents include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

Examples of the therapeutic agents for osteoporosis include alfacalcidol, calcitriol, elcatonin, calcitonin salmon, estriol, ipriflavone, risedronate disodium, pamidronate disodium, alendronate sodium hydrate, incadronate disodium and the like.

Examples of the antidementia agents include tacrine, donepezil, rivastigmine, galanthamine and the like.

Examples of the erectile dysfunction ameliorating agents include apomorphine, sildenafil citrate and the like.

Examples of the therapeutic agents for urinary incontinence or pollakiuria include flavoxate hydrochloride, oxybutynin hydrochloride, propiverine hydrochloride and the like.

Examples of the therapeutic agents for dysuria include acetylcholine esterase inhibitors (e.g., distigmine) and the like.

Examples of the combination drugs include drugs having a cachexia-ameliorating action established in animal models and clinical situations, such as cyclooxygenase inhibitors (e.g., indomethacin), progesterone derivatives (e.g., megestrol acetate), glucosteroids (e.g., dexamethasone), metoclopramide agents, tetrahydrocannabinol agents, fat metabolism improving agents (e.g., eicosapentanoic acid), growth hormones, IGF-1, or antibodies to a cachexia-inducing factor such as TNF-α, LIF, IL-6, oncostatin M and the like.

As the combination drugs, nerve regeneration promoting drugs (e.g., Y-128, VX853, prosaptide), antidepressants (e.g., desipramine, amitriptyline, imipramine), antiepileptics (e.g., lamotrigine), antiarrhythmic agents (e.g., mexiletine), acetylcholine receptor ligands (e.g., ABT-594), endothelin receptor antagonists (e.g., ABT-627), monoamine uptake inhibitors (e.g., tramadol), narcotic analgesics (e.g., morphine), GABA receptor agonists (e.g., gabapentin), α2 receptor agonists (e.g., clonidine), local analgesics (e.g., capsaicin), antianxiety drugs (e.g., benzothiazepines), dopamine receptor agonists (e.g., apomorphine), midazolam, ketoconazole and the like can also be mentioned.

The combination drug is preferably an insulin preparation, an insulin sensitizer, an α-glucosidase inhibitor, biguanide, insulin secretagogue (preferably sulfonylurea) and the like.

The above-mentioned combination drugs may be used in a mixture of two or more kinds thereof at an appropriate ratio.

When the compound of the present invention is used in combination with a combination drug, the dose of each agent can be reduced within a safe range in consideration of the side effects thereof. Particularly, the doses of insulin sensitizers, insulin secretagogues and biguanides can be reduced from generally dose levels. Therefore, the side effects possibly caused by these agents can be safely prevented. In addition, the doses of the therapeutic agents for diabetic complications, the therapeutic agents for hyperlipidemia and the antihypertensive agents can be reduced, and as a result, the side effects possibly caused by these agents can be effectively prevented.

The production method of the compound of the present invention is explained in the following.

Compound (I) can be produced by a method known per se, for example, Method A to Method M shown below or a method analogous thereto. In the following respective production methods, the starting compound may be used as a salt, and as such salt, those exemplified as the salts of the above-mentioned compound (I) can be used.

Compound (III) to be used as a starting compound in the below-mentioned METHOD B can be produced, for example, according to the following METHOD A.

### [METHOD A]

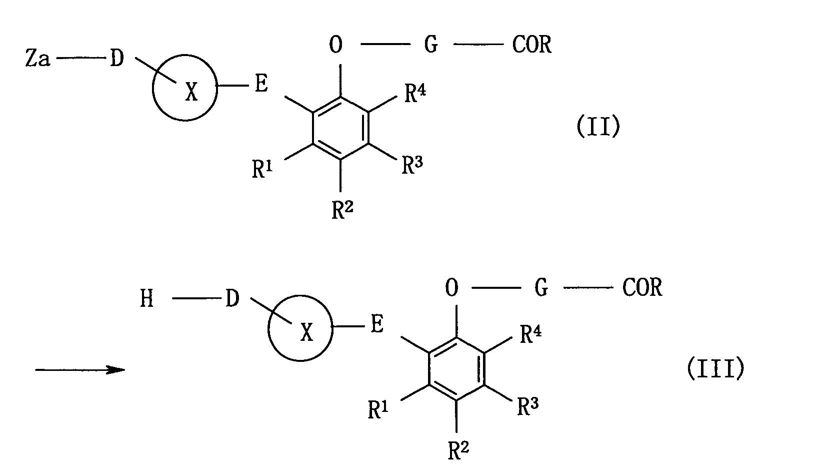

wherein
Za is a hydroxy-protecting group or an amino-protecting group, and other symbols are as defined above.

Here, examples of the "hydroxy-protecting group" for Za include (R¹²)(R¹³) (R¹⁴)Si-, benzyl and the like. Here, R¹², R¹³ and R¹⁴ are each a C₁₋₆ alkyl group or a C₆₋₁₄ aryl group, particularly preferably methyl, ethyl, propyl, isopropyl, tert-butyl, phenyl or the like.

Examples of the "amino-protecting group" for Za include a C₁₋₆ alkoxy-carbonyl group (preferably a tert-butoxycarbonyl group), a C₁₋₆ alkyl-carbonyl group (preferably an acetyl group) and the like.

In this method, compound (III) can be produced by removing protecting group Za of compound (II).

For example, when Za is (R¹²) (R¹³) (R¹⁴)Si-, and D is an oxygen atom, this reaction is performed according to a conventional method and in the presence of a fluoride or an acid in a solvent that does not adversely influence the reaction.

Examples of the fluoride include tetrabutylammonium fluoride, potassium fluoride and the like. The amount of the fluoride to be used is preferably about 1 to about 5 mol per 1 mol of compound (II).

Examples of the acid include inorganic acids such as hydrochloric acid, sulfuric acid and the like; Lewis acids such as boron trichloride, boron tribromide and the like; organic acids such as trifluoroacetic acid, p-toluenesulfonic acid and the like, and the like. The amount of the acid to be used is preferably about 0.01 to about 5 mol per 1 mol of compound (II).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; alcohols such as methanol, ethanol and the like; water and the like. These solvents may be used in a mixture at an appropriate ratio.

The reaction temperature is generally about -80°C to about 150°C, preferably about -10°C to about 100°C.

The reaction time is about 0.5 to about 20 hr.

For example, when Za is benzyl, and D is an oxygen atom, this reaction is performed according to a conventional method and in the presence of hydrogen and a metal catalyst in a solvent that does not adversely influence the reaction.

Examples of the metal catalyst include palladium carbon, palladium black, palladium compounds [e.g., palladium (II) acetate, tetrakis(triphenylphosphine)palladium (0), bis(triphenylphosphine)palladium (II) chloride, dichlorobis(triethylphosphine)palladium (0), tris(dibenzylideneacetone)dipalladium-2,2'-bis(diphenylphosphino)-1,1'-binaphthyl, a complex of palladium (II) acetate and 1,1'-bis(diphenylphosphino)ferrocene], nickel compounds [e.g., tetrakis(triphenylphosphine)nickel (0), bis(triethylphosphine)nickel (II) chloride, bis(triphenylphosphine)nickel (II) chloride], rhodium compounds [e.g., tris(triphenylphosphine)rhodium (III) chloride], platinum compounds and the like. The amount of the metal catalyst to be used is preferably about 0.01 to about 5 mol per 1 mol of compound (II).

Examples of the solvent that does not adversely influence the reaction include ethers such as diethyl ether, tetrahydrofuran, dioxane and the like; aromatic hydrocarbons such as benzene, toluene, xylene and the like; amides such as N,N-dimethylformamide and the like, and the like. These solvents may be used in a mixture at an appropriate ratio.

The reaction temperature is generally about -80°C to about 150°C, preferably about -10°C to about 100°C.

The reaction time is about 0.5 to about 20 hr.

For example, when Za is tert-butoxycarbonyl, and D is NR⁵, this reaction is performed according to a conventional method and in the presence of an acid in a solvent that does not adversely influence the reaction.

As the acid, those similar to the aforementioned acids can be used. The amount of the acid to be used is preferably about 0.01 to about 5 mol per 1 mol of compound (II).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; alcohols such as methanol, ethanol and the like; ketones such as acetone and the like; water and the like. These solvents may be used in a mixture at an appropriate ratio.

The reaction temperature is generally about -80°C to about 150°C, preferably about -10°C to about 100°C.

The reaction time is about 0.5 to about 20 hr.

Compound (III) obtained in this way can be isolated and purified by known separation and purification means, for example, concentration, concentration under reduced pressure, solvent extraction, crystallization, recrystallization, phase transfer, chromatography and the like.

Compound (II) to be used as a starting compound in the above-mentioned METHOD A can be produced according to a method known per se, for example, the method described in Journal of Medicinal Chemistry, page 3354 (2003) or a method analogous thereto.

Compound (I) can be produced, for example, according to the following METHOD B.

### [METHOD B]

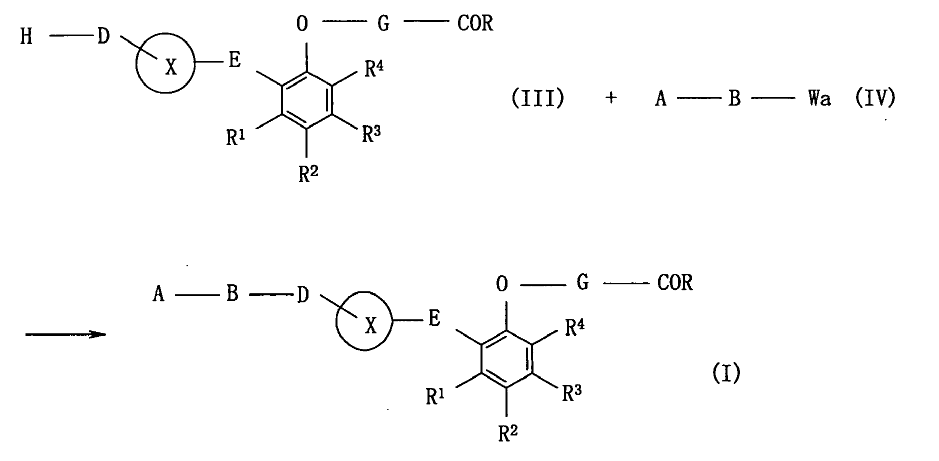

wherein
Wa is a leaving group or a hydroxy group, and other symbols are as defined above.

Here, examples of the "leaving group" for Wa include a halogen atom, -OSO₂R¹⁵ and the like. R¹⁵ is a C₁₋₄ alkyl group, a C₆₋₁₀ aryl group optionally substituted by C₁₋₄ alkyl group(s).

Examples of the "C₁₋₄ alkyl group" and the "C₁₋₄ alkyl group" of the "C₆₋₁₀ aryl group optionally substituted by C₁₋₄ alkyl group(s)" for R¹⁵ include methyl, ethyl, propyl, isopropyl, butyl, isobutyl, sec-butyl and tert-butyl. Of these, methyl is preferable.

In addition, examples of the "C₆₋₁₀ aryl group" of the "C₆₋₁₀ aryl group optionally substituted by C₁₋₄ alkyl group(s)" for R¹⁵ include phenyl, naphthyl and the like. Of these, phenyl is preferable.

R¹⁵ is particularly preferably methyl, tolyl and the like.

In this method, compound (I) can be produced by reacting compound (III) with compound (IV).

When Wa is a leaving group, this reaction is performed according to a conventional method and in the presence of a base in a solvent that does not adversely influence the reaction.

Examples of the base include alkali metal hydrides such as sodium hydride, potassium hydride and the like; alkali metal hydroxides such as sodium hydroxide, potassium hydroxide, lithium hydroxide and the like; alkali earth metal hydroxides such as magnesium hydroxide, calcium hydroxide, barium hydroxide and the like; alkali metal carbonates such as sodium carbonate, potassium carbonate, cesium carbonate and the like; alkali metal hydrogencarbonates such as sodium hydrogencarbonate and the like; alkali metal C₁₋₆ alkoxides such as sodium methoxide, sodium ethoxide, sodium tert-butoxide, potassium tert-butoxide and the like; acetates such as sodium acetate, ammonium acetate and the like; organic lithiums such as methyllithium, n-butyllithium, sec-butyllithium, tert-butyllithium and the like; aromatic amines such as pyridine, lutidine and the like; tertiary amines such as trimethylamine, triethylamine, tripropylamine, tributylamine, N-ethyldiisopropylamine, cyclohexyldimethylamine, 4-dimethylaminopyridine, N,N-dimethylaniline, N-methylpiperidine, N-methylpyrrolidine, N-methylmorpholine and the like; 1,5-diazabicyclo[4.3.0]-5-nonene, 1,4-diazabicyclo[2.2.2]octane, 1,8-diazabicyclo[5.4.0]-7-undecene; metal amides such as sodium amide, lithium diisopropylamide, lithium hexamethyldisilazide and the like; and the like. The amount of the base to be used is preferably about 1 to about 5 mol per 1 mol of compound (III).

The amount of compound (IV) to be used is preferably about 1 to about 5 mol per 1 mol of compound (III).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like, and the like. These solvents may be used in a mixture at an appropriate ratio.

The reaction temperature is generally about -80°C to about 150°C, preferably about -10°C to about 100°C.

The reaction time is about 0.5 to about 20 hr.

When Wa is a hydroxy group, this reaction is performed according to a method known per se, for example, the method described in Synthesis, page 1 (1981), or a method analogous thereto. In other words, this reaction is generally performed in the presence of an organic phosphorus compound and an electrophilic agent in a solvent that does not adversely influence the reaction.

Examples of the organic phosphorus compound include triphenylphosphine, tributylphosphine and the like.

Examples of the electrophilic agent include diethyl azodicarboxylate, diisopropyl azodicarboxylate, azodicarbonyldipiperazine and the like.

The amount of each of the organic phosphorus compound and the electrophilic agent to be used is preferably about 1 to about 5 mol per 1 mol of compound (III).

The amount of compound (IV) to be used is preferably about 1 to about 5 mol per 1 mol of compound (III).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like, and the like. These solvents may be used in a mixture at an appropriate ratio.

The reaction temperature is generally about -80°C to about 150°C, preferably about -10°C to about 100°C.

The reaction time is generally about 0.5 to about 20 hr.

Compound (IV) to be used as a starting compound in the above-mentioned METHOD B can be produced according to a known method.

Compound (VI) to be used as a starting compound in the below-mentioned METHOD D can be produced, for example, according to the following METHOD C.

### [METHOD C]

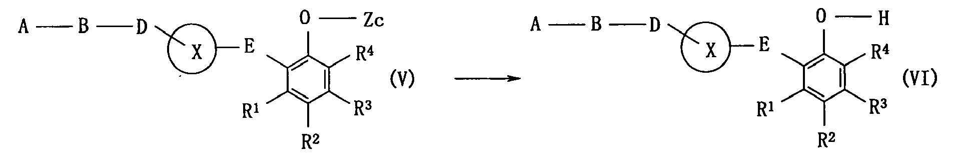

wherein
Zc is a hydroxy-protecting group, and other symbols are as defined above.

Here, examples of the "hydroxyl-protecting group" for Zc include methoxymethyl, benzyl and the like.

In this method, compound (VI) can be produced by removing the protecting group Zc of compound (V).

For example, when Zc is methoxymethyl, this reaction is performed according to a conventional method and in the presence of an acid in a solvent that does not adversely influence the reaction.

Examples of the acid include hydrochloric acid, sulfuric acid, toluenesulfonic acid, trifluoroacetic acid and the like. The amount of the acid to be used is preferably about 0.01 to about 5 mol per 1 mol of compound (V).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; alcohols such as methanol, ethanol and the like; ketones such as acetone and the like; water and the like. These solvents may be used in a mixture at an appropriate ratio.

The reaction temperature is generally about -80°C to about 150°C, preferably about -10°C to about 100°C.

The reaction time is about 0.5 to about 20 hr.

For example, when Zc is benzyl, this reaction is performed according to a conventional method and in the presence of hydrogen and a metal catalyst in a solvent that does not adversely influence the reaction.

As the metal catalyst, those exemplified in the aforementioned METHOD A can be used. The amount of the metal catalyst to be used is preferably about 0.01 to about 5 mol per 1 mol of compound (V).

Examples of the solvent that does not adversely influence the reaction include ethers such as diethyl ether, tetrahydrofuran, dioxane and the like; aromatic hydrocarbons such as benzene, toluene, xylene and the like; amides such as N,N-dimethylformamide and the like; sulfoxides such as dimethyl sulfoxide and the like; alcohols such as methanol, ethanol and the like; esters such as ethyl acetate and the like; water and the like. These solvents may be used in a mixture at an appropriate ratio.

The reaction temperature is generally about -80°C to about 150°C, preferably about -10°C to about 100°C.

The reaction time is about 0.5 to about 20 hr.

Compound (VI) obtained in this way can be isolated and purified by known separation and purification means, for example, concentration, concentration under reduced pressure, solvent extraction, crystallization, recrystallization, phase transfer, chromatography and the like.

Compound (V) to be used as a starting compound in the above-mentioned METHOD C can be produced, for example, by a combination of a method analogous to the above-mentioned METHOD A and a method analogous to the above-mentioned METHOD B. Specifically, compound (V) can be produced by a combination of the above-mentioned METHOD A and METHOD B, in each of which -G-COR is replaced with Zc.

Compound (I) can also be produced, for example, according to the following METHOD D.

### [METHOD D]

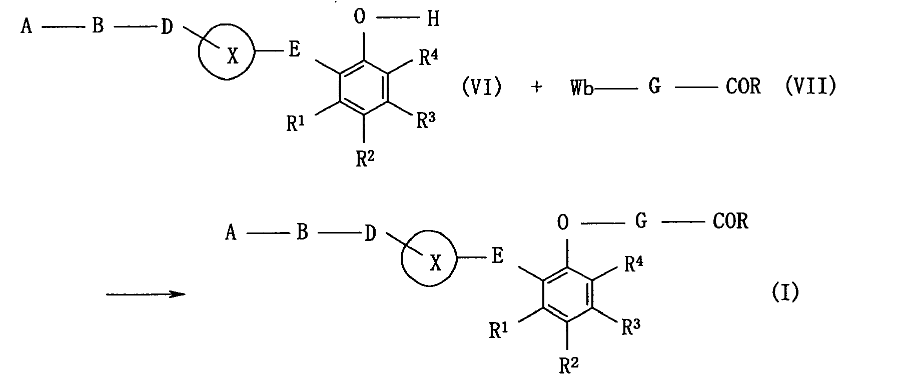

wherein
Wb is a leaving group or a hydroxy group, and other symbols are as defined above.

Here, examples of the "leaving group" for Wb include those exemplified for the aforementioned Wa.

In this method, compound (I) can be produced by reacting compound (VI) with compound (VII).

When Wb is a leaving group, this reaction is performed according to a conventional method and in the presence of a base in a solvent that does not adversely influence the reaction.

As the base, those exemplified in the aforementioned METHOD B can be used. The amount of the base to be used is preferably about 1 to about 5 mol per 1 mol of compound (VI).

The amount of compound (VII) to be used is preferably about 1 to about 10 mol per 1 mol of compound (VI).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; ketones such as acetone and the like. These solvents may be used in a mixture at an appropriate ratio.

The reaction temperature is generally about -80°C to about 150°C, preferably about -10°C to about 100°C.

The reaction time is about 0.5 to about 20 hr.

When Wb is a hydroxy group, this reaction is performed in the same manner as in the reaction when Wa is a hydroxy group in the aforementioned METHOD B.

Compound (VII) to be used as a starting compound in the above-mentioned METHOD D can be produced according to a known method.

Compound (1-3), which is compound (I) wherein R is a hydroxy group, can be produced, for example, according to the following METHOD E.

### [METHOD E]

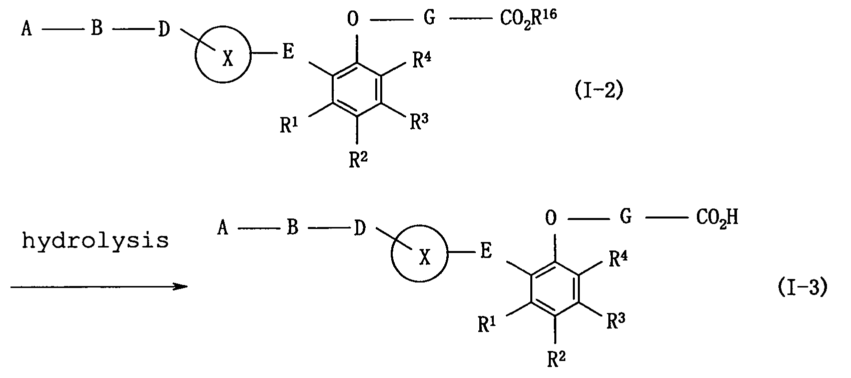

wherein
R¹⁶ is a C₁₋₁₀ alkyl group, a benzyl group optionally substituted by C₁₋₆ alkyl group (s), or a C₆₋₁₄ aryl group optionally substituted by C₁₋₆ alkyl group(s), and other symbols are as defined above.

Examples of the "C₁₋₁₀ alkyl group" for R¹⁶ include those exemplified as the aforementioned "substituent" for R¹, R², R³ or R⁴.

Examples of the "C₆₋₁₄ aryl group" of the "C₆₋₁₄ aryl group optionally substituted by C₁₋₆ alkyl group"(s) for R¹⁶ include those exemplified as the aforementioned "substituent" for R¹, R², R³ or R⁴.

R¹⁶ is particularly preferably methyl, ethyl, tert-butyl, benzyl, phenyl or the like.

In this method, compound (1-3) can be produced by subjecting compound (1-2), which is compound (I) wherein R is OR¹⁶, to hydrolysis.

This reaction is performed according to a conventional method and in the presence of an acid or a base in a solvent that does not adversely influence the reaction.

As the acid, those exemplified in the aforementioned METHOD A can be used. The amount of the acid to be used is preferably about 0.01 to about 5 mol per 1 mol of compound (I-2).

Examples of the base include alkali metal hydroxides such as sodium hydroxide, potassium hydroxide, lithium hydroxide and the like; alkali earth metal hydroxides such as magnesium hydroxide, calcium hydroxide, barium hydroxide and the like; and the like. The amount of the base to be used is preferably about 1 to about 10 mol per 1 mol of compound (I-2).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; alcohols such as methanol, ethanol and the like; and ketones such as acetone and the like. These solvents may be used in a mixture at an appropriate ratio, or may be further diluted with water at an appropriate ratio.

The reaction temperature is generally about -80°C to about 150°C, preferably about -10°C to about 100°C.

The reaction time is about 0.5 to about 20 hr.

Compound (I-2) to be used as a starting compound in the above-mentioned METHOD E can be produced, for example, according to the above-mentioned METHOD D.

Compound (I-5), which is compound (I) wherein E is C(OH)H, can be produced, for example, according to the following METHOD F.

### [METHOD F]

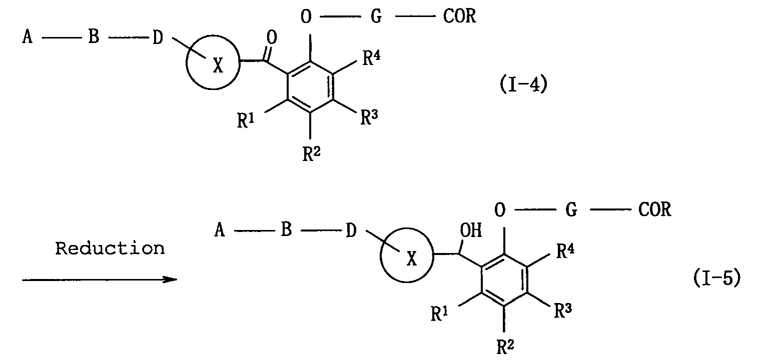

wherein the symbols in the formula are as defined above.

In this method, compound (I-5) can be produced by subjecting compound (I-4), which is compound (I) wherein E is CO, to reduction.

This reaction is generally performed by using a reducing agent in a solvent that does not adversely influence the reaction.

Examples of the reducing agent include metal hydrides such as aluminum hydride, tributyltin hydride and the like; metal hydrogen complex compounds such as lithium aluminum hydride, sodium borohydride and the like; borane complexes such as borane tetrahydrofuran complex, borane dimethylsulfide complex and the like; alkylboranes such as thexylborane, disiamylborane and the like; diborane; metals such as zinc, aluminum, tin, iron and the like; and the like. The amount of the reducing agent to be used is preferably about 1 to about 10 mol per 1 mol of compound (1-4).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; alcohols such as methanol, ethanol and the like; water and the like. These solvents may be used in a mixture at an appropriate ratio.

The reaction temperature is generally about -80 to about 150°C, preferably about -10 to about 100°C.

The reaction time is about 0.5 to about 20 hr.

Compound (I-4) to be used as starting compound in the above-mentioned METHOD F can be produced, for example, according to the above-mentioned METHOD D.

In the above-mentioned METHOD F, compound (VI) wherein E is C(OH)H, which is used as a starting compound in the above-mentioned METHOD D and the below-mentioned METHOD L, can be produced by replacing -G-COR with H.

Compound (I-6), which is compound (I) wherein E is C(OH)R⁷, can be produced, for example, according to the following METHOD G.

### [METHOD G]

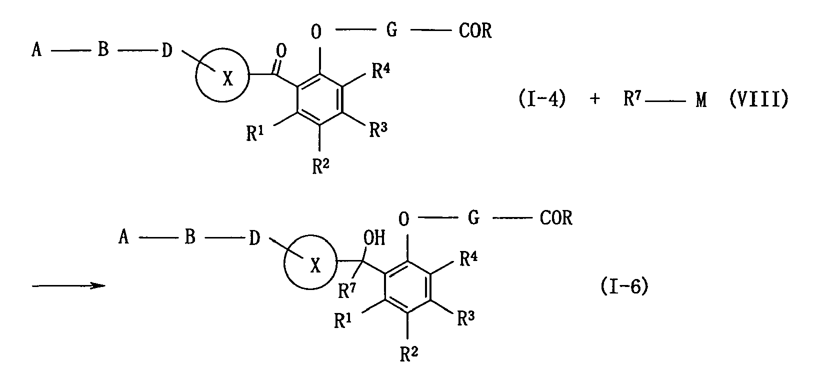

wherein
M is a metal atom optionally having ligand(s), and other symbols are as defined above.

Here, examples of the "metal atom" for M include lithium, magnesium, zinc and the like. In addition, examples of the "ligand" include a halogen atom, triphenylphosphine and the like.

In this method, compound (I-6) can be produced by reacting compound (I-4) with compound (VIII).

This reaction is performed according to a conventional method and without the presence of oxygen and water in a solvent that does not adversely influence the reaction.

The amount of compound (VIII) to be used is preferably about 1 to about 10 mol per 1 mol of compound (I-4).

Examples of the solvent that does not adversely influence the reaction include ethers such as diethyl ether, tetrahydrofuran, dioxane and the like; aromatic hydrocarbons such as benzene, toluene, xylene and the like, and the like. These solvents may be used in a mixture at an appropriate ratio.

The reaction temperature is generally about -80 to about 150°C, preferably about -10 to about 100°C.

The reaction time is about 0.5 to about 20 hr.

Compound (VIII) to be used as a starting material in the above-mentioned METHOD G can be produced according to a known method.

In the above-mentioned METHOD G, a compound (VI) wherein E is C(OH)R⁷, which is used as a starting compound in the above-mentioned METHOD D and the below-mentioned METHOD L, can be produced by replacing -G-COR with H.

Compound (I-7), which is compound (I) wherein E is C(OR⁶)H, can be produced, for example, according to the following METHOD H.

### [METHOD H]

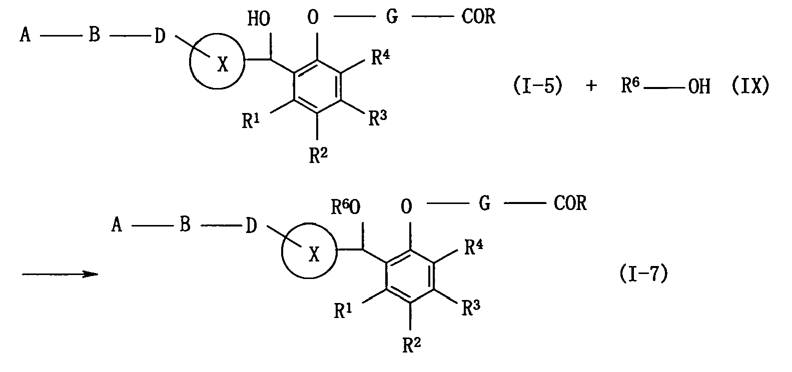

wherein symbols are as defined above.

In this method, compound (I-7) can be produced by reacting with compound (I-5) with compound (IX).

This reaction is performed according to a conventional method and in the presence of a metal catalyst in a solvent that does not adversely influence the reaction.

Examples of the metal catalyst include palladium carbon, palladium black, palladium compounds [e.g., palladium (II) acetate, tetrakis(triphenylphosphine)palladium (0), bis(triphenylphosphine)palladium (II) chloride, dichlorobis(triethylphosphine)palladium (0), tris(dibenzylideneacetone)dipalladium-2,2'-bis(diphenylphosphino)-1,1'-binaphthyl, a complex of palladium acetate (II) and 1,1'-bis(diphenylphosphino)ferrocene, etc.], rhodium compounds [e.g., tris(triphenylphosphine)rhodium (III) chloride, etc.], cobalt compounds and the like. The amount of the metal catalyst to be used is preferably about 0.01 to about 5 mol per 1 mol of compound (I-5).

The amount of compound (IX) to be used is preferably about 1 to about 1000 mol per 1 mol of compound (I-5).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like, and the like. These solvents may be used in a mixture at an appropriate ratio.

The reaction temperature is generally about -80°C to about 150°C, preferably about - 10 to about 100°C.

The reaction time is about 0.5 to about 100 hr.

Compound (IX) to be used as a starting compound in the above-mentioned METHOD H can be produced according to a known method.

In the above-mentioned METHOD H, a compound (VI) wherein E is C(OR⁶)H, which is used as a starting compound in the above-mentioned METHOD D and the below-mentioned METHOD L, can be produced by replacing -G-COR with H.

Compound (I-9), which is compound (I) wherein R is OR²⁰ (wherein R²⁰ is a hydrogen atom or as defined for the aforementioned R¹⁶), and one of R¹ to R⁴ is a hydroxy group, can be produced, for example, according to the following METHOD I.

### [METHOD I]

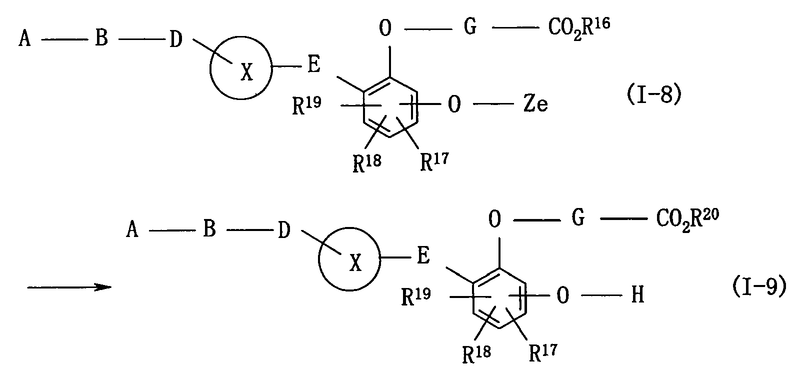

wherein
R¹⁷ to R¹⁹ are each a hydrogen atom or a substituent, Ze is a hydroxy-protecting group, and other symbols are as defined above.

As the substituent for R¹⁷ to R¹⁹, those exemplified for the aforementioned R¹ to R⁴ can be used.

As the hydroxy-protecting group for Ze, those exemplified for the aforementioned Zc can be used.

In this method, compound (I-9) can be produced by removing the protecting group Ze of compound (I-8), which is compound (I) wherein R is OR¹⁶ and one of R¹ to R⁴ is -O-Ze. This method is performed in the same manner as in the aforementioned METHOD C.

Compound (I-8) to be used as a starting compound in the above-mentioned METHOD I can be produced, for example, according to the above-mentioned METHOD D.

In addition, compound (I-11), which is compound (I-9)
wherein R²⁰ is R¹⁶, can be produced, for example, according to the following METHOD J.

### [METHOD J]

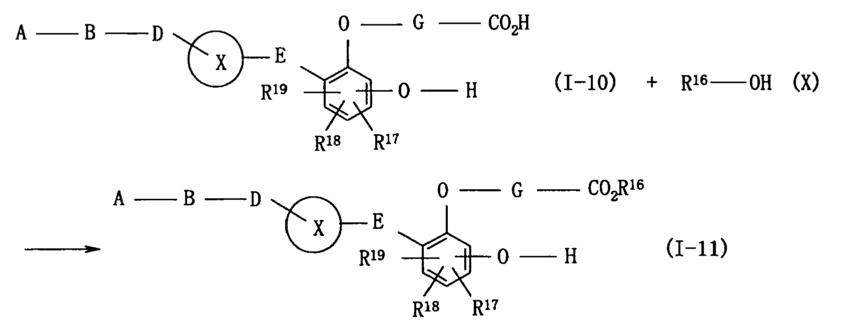

wherein the symbols in the formula are as defined above.

In this method, compound (I-11) can be produced by reacting with compound (I-10), which is compound (I) wherein R is a hydroxy group, and one of R¹ to R⁴ is a hydroxy group, with compound (X).

This reaction is performed according to a conventional method and in the presence of an acid, using compound (X) as a solvent, or in a mixed solvent of compound (X) and a solvent that does not adversely influence the reaction.

As the acid, those exemplified in the aforementioned METHOD A can be used. The amount of the acid to be used is preferably about 0.01 to about 5 mol per 1 mol of compound (I-10).

The amount of compound (X) to be used is preferably about 1 to about 1000 mol per 1 mol of compound (I-10).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like, and the like. These solvents may be used in a mixture at an appropriate ratio.

The reaction temperature is generally about -80°C to about 150°C, preferably about -10°C to about 100°C.

The reaction time is about 0.5 to about 20 hr.

Compound (I-10) to be used as a starting compound in the above-mentioned METHOD J can be produced, for example, according to the above-mentioned METHOD I. In addition, compound (X) can be produced according to a known method.

Compound (I-12), which is compound (I) wherein one of R¹ to R⁴ is -OR²² (wherein R²² is as defined for the aforementioned R¹⁶), can be produced, for example, according to the following METHOD K.

### [METHOD K]

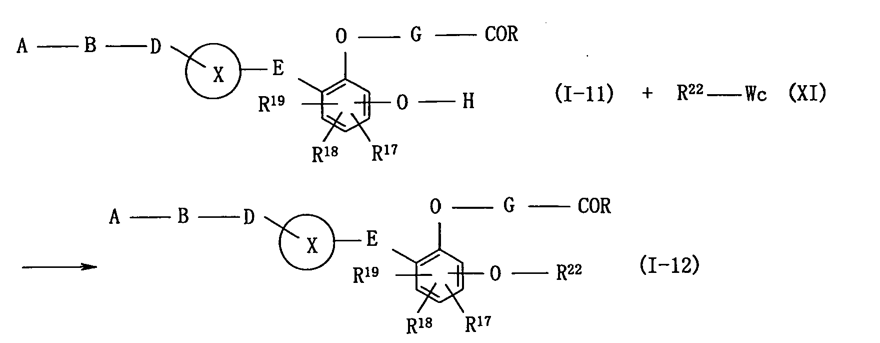

wherein Wc is a leaving group, and other symbols are as defined above.

Here, examples of the "leaving group" for Wc include those exemplified for the aforementioned Wa.

In this method, compound (I-12) can be produced by reacting with compound (I-11), which is compound (I) wherein one of R¹ to R⁴ is a hydroxy group, with compound (XI).

This reaction is performed in the same manner as in a reaction when Wa is a leaving group in the aforementioned METHOD B.

Compound (I-11) to be used as a starting compound in the above-mentioned METHOD K can be produced, for example, according to the above-mentioned METHOD J or a method analogous thereto. In addition, compound (XI) can be produced according to a known method.

Compound (I-13), which is compound (I) wherein G is -CH₂-CH₂-, and R is a hydroxy group, can be produced, for example, according to the following METHOD L.

### [METHOD L]

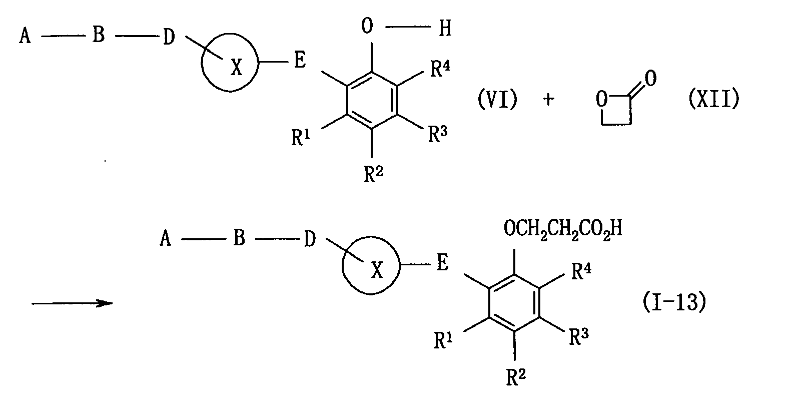

wherein the symbols in the formula are as defined above.

In this method, compound (I-13) can be produced by reacting compound (VI) with compound (XII).

This reaction is performed according to a conventional method and in the presence of a base in a solvent that does not adversely influence the reaction.

Examples of the base include alkali metal hydrides such as sodium hydride, potassium hydride and the like; alkali metal hydroxides such as sodium hydroxide, potassium hydroxide, lithium hydroxide and the like; alkali earth metal hydroxides such as magnesium hydroxide, calcium hydroxide, barium hydroxide and the like; alkali metal carbonates such as sodium carbonate, potassium carbonate, cesium carbonate and the like; alkali metal hydrogencarbonate such as sodium hydrogencarbonate and the like; alkali metal C₁₋₆ alkoxides such as sodium methoxide, sodium ethoxide, sodium tert-butoxide, potassium tert-butoxide and the like; organic lithiums such as methyllithium, n-butyllithium, sec-butyllithium, tert-butyllithium and the like; aromatic amines such as pyridine, lutidine and the like; tertiary amines such as trimethylamine, triethylamine, tripropylamine, tributylamine, N-ethyldiisopropylamine, cyclohexyldimethylamine, 4-dimethylaminopyridine, N,N-dimethylaniline, N-methylpiperidine, N-methylpyrrolidine, N-methylmorpholine and the like; 1,5-diazabicyclo[4.3.0]-5-nonene, 1,4-diazabicyclo[2.2.2]octane, 1,8-diazabicyclo[5.4.0]-7-undecene; metal amides such as sodium amide, lithium diisopropylamide, lithium hexamethyldisilazide and the like; and the like. The amount of base to be used is preferably about 1 to about 5 mol per 1 mol of compound (VI).

The amount of compound (XII) to be used is preferably about 1 to about 10 mol per 1 mol of compound (VI).

Examples of the solvent that does not adversely influence the reaction include ethers such as diethyl ether, tetrahydrofuran, dioxane and the like; aromatic hydrocarbons such as benzene, toluene, xylene and the like; amides such as N,N-dimethylformamide and the like; sulfoxides such as dimethyl sulfoxide and the like, and the like. These solvents may be used in a mixture at an appropriate ratio.

The reaction temperature is generally about -80°C to about 150°C, preferably about -10°C to about 100°C.

The reaction time is about 0.5 to about 20 hr.

Compound (XII) to be used as a starting compound in the above-mentioned METHOD L can be produced according to a known method.

Compound (I) can also be produced, for example, according to the following METHOD M.

### [METHOD M]

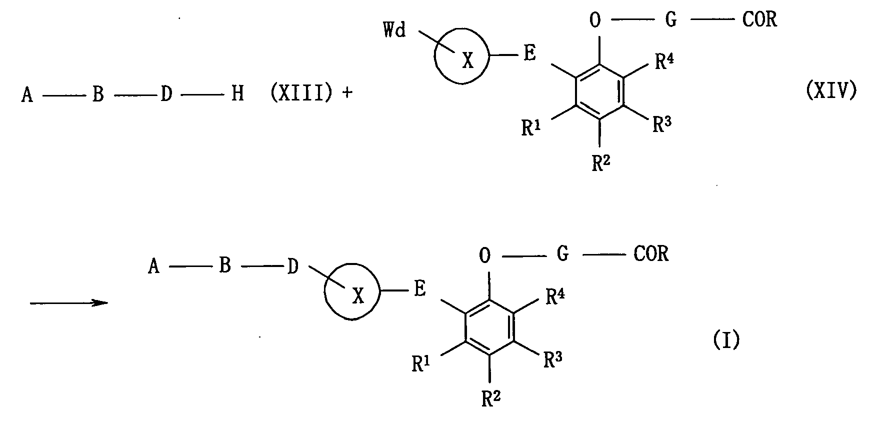

wherein
Wd is a leaving group, and other symbols are as defined above.

Here, examples of the "leaving group" for Wd include those exemplified for the aforementioned Wa.

In this method, compound (I) can be produced by reacting compound (XIII) with compound (XIV).

This reaction is performed in the same manner as in the aforementioned METHOD L.

Compound (XIII) to be used as a starting compound in the above-mentioned METHOD M can be produced according to a known method. In addition, compound (XIV) can be produced according to a method known per se, for example, the method described in Journal of Medicinal Chemistry, page 3354 (2003) or a method analogous thereto.

Compound (VI) to be used as a starting compound in the above-mentioned METHOD D and the below-mentioned METHOD L can be produced by replacing -G-COR to H in the above-mentioned METHOD M.

In each of the aforementioned reactions, when the starting compound has an amino group, a carboxyl group, a hydroxy group or a carbonyl group as a substituent, a protecting group generally used in the peptide chemistry and the like may be introduced into these groups, and the object compound can be obtained by eliminating the protecting group as necessary after the reaction.

In addition, the compound of the present invention obtained by each production method mentioned above can be isolated and purified by a known means such as concentration, concentration under reduced pressure, solvent extraction, crystallization, recrystallization, phase transfer, chromatography and the like. Each starting compound used in each of the above-mentioned production methods can be isolated and purified by a known means similar to those mentioned above. It is also possible to use such starting compound as it is in a reaction mixture without isolation, as a starting material for the next step.

For production of the compound of the present invention, when the starting compound may form a salt, the starting compound can be used as a salt. Examples of such salt include those exemplified as the salts of the compound of the present invention.

When compound (I) contains an optical isomer, a stereoisomer, a positional isomer or a rotational isomer, they are also encompassed in compound (I) and can be obtained as single products by synthesis techniques and separation techniques known per se. For example, when compound (I) contains an optical isomer, an optical isomer separated from the compound is also encompassed in compound (I).

### Examples

The present invention is explained in more detail in the following by referring to Experimental Examples, Reference Examples, Examples and Formulation Examples, which are not to be construed as limitative.

In the following Reference Examples and Examples, % means wt% unless otherwise specified.

In addition, room temperature means a temperature of 1-30°C unless otherwise specified.

### Experimental Example 1

### Blood glucose and blood lipid (triglyceride) lowering action in mice

The test compound was mixed with a powder diet (CE-2, CLEA JAPAN, INC.) at a proportion of 0.01% (0.03% for the compound of Example 1) and freely given to KKAy mice (9 to 12-week-old, 5 mice per group) which are obese ·non-insulin-dependent diabetes (type-2 diabetes) models, for 6 days. During this period, water was freely given to the mice. The blood was drawn from the orbital venous plexus. Glucose and triglyceride in the plasma separated from the blood were quantitated using L Type Wako Glu2 (Wako Pure Chemical Industries, Ltd.) and L Type Wako TG·H (Wako Pure Chemical Industries, Ltd.), respectively, according to an enzyme method. The results are shown in Table 1.

In the Table, the "hypoglycemic action (%)" shows a decrease rate (%) of the blood glucose level of the test compound administration group when the blood glucose level of the test compound non-administration group is 100%. In addition, the "hypolipidemic action (%)" shows a decrease rate (%) of the blood triglyceride of the test compound administration group when the blood triglyceride value of the test compound non-administration group is 100%.

**Table 1**

| Test compound (Example No.) | hypoglycemic action (%) | hypolipidemic action (%) |
|---|---|---|
| 1 | 54 | 44 |
| 14 | 38 | 29 |
| 21 | 32 | 14 |
| 22 | 41 | 48 |
| 53 | 46 | 12 |

As shown above, it is clear that the compound of the present invention has superior hypoglycemic action and hypolipidemic action, and is useful as an agent for the prophylaxis or treatment of diabetes, hyperlipidemia (particularly, hypertriglyceridemia), impaired glucose tolerance and the like.

### Experimental Example 2 (PPARγ-RXRα heterodimer ligand activity)

The PPARγ:RXRα:4ERPP/CHO-K1 cells described in WO03/099793 were cultured in a HamF12 medium [manufactured by Life Technologies, Inc., US] containing 10% calf fetal serum [manufactured by Life Technologies, Inc., US], sown in a 96-well white plate [manufactured by Corning Coster Corporation, US] at 2×10⁴ cells/well, and incubated overnight in a carbon dioxide gas incubator at 37°C.

Then, the medium was removed from the 96 well white plate, 80 µl of HamF12 medium containing 0.1% fatty acid-free bovine serum albumin (BSA) and a test compound (20 µl) were added, and the cells were incubated for 18-24 hr in a carbon dioxide gas incubator at 37°C. The medium was removed, 40 µl of PicaGene7.5 (manufactured by Wako Pure Chemical Industries, Ltd.) 2-fold diluted with HBSS (HANKS' BALANCED SALT SOLUTION) [manufactured by BIO WHITTAKER, US] was added. After stirring, the luciferase activity was determined using the 1420 ARVO Multilabel Counter [manufactured by PerkinElmer, US].

The induction rate was calculated from the luciferase activity of each test compound based on the luciferase activity of the test compound non-administration group as 1. The test compound concentration and the induction rate were analyzed by PRISM [manufactured by GraphPad Software, Inc., US] to calculate EC₅₀ value (compound concentration showing 50% of the maximum value of induction rate) of the test compound.

In addition, the luciferase activity induction magnification maximum value of each test compound relative to that of the 5-{4-[2-(5-methyl-2-phenyl-1,3-oxazol-4-yl)ethoxylbenzyll-1,3-thiazolidin-2,4-dione (PPARγ full agonist) administration group as 100% was defined as Vmax and calculated. The results are shown in Table 2.

**Table 2**

| Test compound (Example No.) | EC₅₀ (nM) | Vmax (%) |
|---|---|---|
| 1 | 100 | 57 |
| 14 | 15 | 61 |
| 21 | 8.1 | 60 |
| 22 | 33 | 70 |
| 42 | 1.0 | 73 |
| 53 | 5.1 | 60 |
| 55 | 15 | 44 |

As shown above, it is clear that the compound of the present invention has superior PPARγ-RXRα heterodimer ligand activity, and shows a PPARγ partial agonist action. Therefore, it is shown that the compound of the present invention is useful as a hypoglycemic agent unaccompanied by side effects such as body weight gain, adipocyte accumulation, cardiac hypertrophy and the like, as compared to a full PPARγ agonist.

### LC-MS measurement conditions

In the following Examples, HPLC-mass spectrum (LC-MS) was measured under the following conditions.

measurement device: Micromass ZMD, and Agilent Technologies HP1100

column: CAPCELL PAK C18UG120, S-3 µm, 1.5X35 mm

solvent: SOLUTION A; 0.05% trifluoroacetic acid containing water, SOLUTION B; 0.04% trifluoroacetic acid containing acetonitrile

gradient cycle: 0.00 min (SOLUTION A/SOLUTION B=90/10), 2.00 min (SOLUTION A/SOLUTION B=5/95), 2.75 min (SOLUTION A/SOLUTION B=5/95), 2.76 min (SOLUTION A/SOLUTION B=90/10), 3.45 min (SOLUTION A/SOLUTION B=90/10)

injection volume: 2 µl, flow rate: 0.5 ml/min, detection method: UV 220 nm

ionization method: Electron Spray Ionization:ESI

### Preparative HPLC conditions

In the following Reference Examples and Examples, the purification by preparative HPLC was performed under the following conditions.

instrument: High Through-put Purification System, Gilson Company, Inc.

column: YMC CombiPrep ODS-A, S-5 µm, 50X20 mm

solvent: SOLUTION A; 0.1% trifluoroacetic acid or formic acid containing water, SOLUTION B; 0.1% trifluoroacetic acid or formic acid containing acetonitrile

gradient cycle: 0.00 min (SOLUTION A/SOLUTION B=90/10), 1.00 min (SOLUTION A/SOLUTION B=90/10), 4.00 min (SOLUTION A/SOLUTION B=10/95), 8.50 min (SOLUTION A/SOLUTION B=10/95), 8.60 min (SOLUTION A/SOLUTION B=90/10), 8.70 min (SOLUTION A/SOLUTION B=90/10)

flow rate: 20 ml/min, detection method: UV 220 nm

### Reference Example 1

To a mixture of 2-hydroxy-4-methoxybenzaldehyde (25.26 g), potassium carbonate (34.42 g) and N,N-dimethylformamide (100 ml) was added dropwise chloromethyl methyl ether (16.39 ml) at 0°C. The reaction mixture was warmed to room temperature and mixed at 40 hr. Diisopropyl ether was added, and the mixture was washed with water and saturated brine, dried (MgSO₄) and concentrated. The residue was subjected to silica gel column chromatography, eluted with ethyl acetate-hexane (1:6→1:4, v/v) to give 2-(methoxymethoxy)-4-methoxybenzaldehyde (20.40 g, yield 63%) as colorless crystals. melting point 55 - 56°C.

### Reference Example 2

To a mixture of 4-bromophenol (25.75 g), triisopropylsilyl trifluoromethanesulfonate (50.17 g) and dichloromethane (150 ml) was added dropwise 2,6-lutidine (20.80 ml) at 0°C. The reaction mixture was stirred at 0°C for 2 hr and concentrated. Ethyl acetate was added to the residue, and the mixture was washed with water and saturated brine, dried (MgSO₄) and concentrated. The residue was subjected to silica gel column chromatography, eluted with hexane to give 1-bromo-4-(triisopropylsilyloxy)benzene (48.73 g, yield 99%) as a colorless oil.
¹H-NMR (300 MHz, CDCl₃) δ:1.04 - 1.12 (18 H, m), 1.17 - 1.30 (3 H, m), 6.76 (2 H, d, J=9.0 Hz), 7.30 (2 H, d, J=9.0 Hz).

### Reference Example 3

To a solution of 1-bromo-4-(triisopropylsilyloxy)benzene (15.00 g) in tetrahydrofuran (100 ml) was added dropwise n-butyllithium (1.6 M hexane solution, 29.9 ml) at -78°C. The reaction mixture was stirred at -78°C for 2 hr, and a solution of 2-(methoxymethoxy)-4-methoxybenzaldehyde (8.94 g) in tetrahydrofuran (30 ml) was added dropwise at -78°C. The reaction mixture was gradually warmed to room temperature, and the mixture was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:6→1:4, v/v) to give [4-methoxy-2-(methoxymethoxy)phenyl][4-(triisopropylsilyloxy)phenyl]methanol (15.61 g, yield 77%) as a colorless oil.
¹H-NMR (300 MHz, CDCl₃) δ:1.00 - 1.14 (18 H, m), 1.16 - 1.29 (3 H, m), 2.72 (1 H, d, J=5.4 Hz), 3.30 (3 H, s), 3.79 (3 H, s), 5.07 - 5.13 (2 H, m), 5.94 (1 H, d, J=5.4 Hz), 6.53 (1 H, dd, J=2.4, 8.4 Hz), 6.67 (1 H, d, J=2.4 Hz), 6.82 (2 H, d, J=8.7 Hz), 7.16 - 7.23 (3 H, m).

### Reference Example 4

A mixture of [4-methoxy-2-(methoxymethoxy)phenyl][4-(triisopropylsilyloxy)phenyl]methanol (5.00 g), manganese dioxide (9.73 g) and tetrahydrofuran (50 ml) was stirred at room temperature for 40 hr, and stirred at 70°C for 4 hr. The insoluble material was removed by a filtration operation, and the filtrate was concentrated. The residue was subjected to silica gel column chromatography, eluted with ethyl acetate-hexane (1:9 → 1:5, v/v) to give [4-methoxy-2-(methoxymethoxy)phenyl][4-(triisopropylsilyloxy)phenyl]methanone (3.94 g, yield 79%) as a colorless oil.
¹H-NMR (300 MHz, CDCl₃) δ:1.00 - 1.18 (18 H, m), 1.19 - 1.33 (3 H, m), 3.33 (3 H, s), 3.85 (3 H, s), 5.04 (2 H, s), 6.62 (1 H, dd, J=2.4, 8.4 Hz), 6.75 (1 H, d, J=2.4 Hz), 6.88 (2 H, d, J=9.0 Hz), 7.36 (1 H, d, J=8.4 Hz), 7.73 (2 H, d, J=9.0 Hz).

### Reference Example 5

A mixture of [4-methoxy-2-(methoxymethoxy)phenyl][4-(triisopropylsilyloxy)phenyl]methanone (3.93 g), tetrabutylammonium fluoride trihydrate (3.35 g) and tetrahydrofuran (50 ml) was stirred at 0°C for 30 min, and the reaction mixture was concentrated. Water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v) to give (4-hydroxyphenyl)[4-methoxy-2-(methoxymethoxy)phenyl]methanone (2.01 g, yield 79%) as a colorless oil.
¹H-NMR (300 MHz, CDCl₃) δ:3.34 (3 H, s), 3.85 (3 H, s), 5.05 (2 H, s), 6.38 - 6.58 (1 H, br), 6.61 (1 H, dd, J=2.4, 8.4 Hz), 6.76 (1 H, d, J=2.4 Hz), 6.86 (2 H, d, J=8.7 Hz), 7.34 (1 H, d, J=8.4 Hz), 7.75 (2 H, d, J=8.7 Hz).

### Reference Example 6

A mixture of 4-(chloromethyl)-5-methyl-2-phenyl-1,3-oxazole (1.58 g), (4-hydroxyphenyl)[4-methoxy-2-(methoxymethoxy)phenyl]methanone (2.00 g), potassium carbonate (1.92 g) and N,N-dimethylformamide (20 ml) was stirred at room temperature for 40 hr. Water was added to the reaction mixture, and the mixture was extracted with ethyl acetate. The ethyl acetate layer was washed with water and saturated brine, dried (MgSO₄) and concentrated. The residue was crystallized from ethyl acetate-hexane to give [4-methoxy-2-(methoxymethoxy)phenyl]{4-[(5-methyl-2-phenyl-1,3-oxazol-4-yl)methoxy]phenyl}methanone (2.69 g, yield 61%) as colorless crystals. The mother liquor was concentrated, and the residue was subjected to silica gel column chromatography, eluted with ethyl acetate-hexane (1:4→1:1, v/v), concentrated, and crystallized from ethyl acetate-hexane to give [4-methoxy-2-(methoxymethoxy)phenyl]{4-[(5-methyl-2-phenyl-1,3-oxazol-4-yl)methoxy]phenyl}methanone (144 mg, yield 3%) as colorless crystals. melting point 119 - 120°C.

### Reference Example 7

A mixture of [4-methoxy-2-(methoxymethoxy)phenyl]{4-[(5-methyl-2-phenyl-1,3-oxazol-4-yl)methoxy]phenyl}methanone (2.69 g), 1 M hydrochloric acid (10 ml) and acetone (50 ml) was stirred at 60°C for 3 hr. The reaction mixture was concentrated, water was added to the residue, and the mixture was extracted with ethyl acetate. The ethyl acetate layer was washed with water and saturated brine, dried (MgSO₄) and concentrated. The residue was crystallized from ethyl acetate-hexane to give (2-hydroxy-4-methoxyphenyl){4-[(5-methyl-2-phenyl-1,3-oxazol-4-yl)methoxy]phenyl}methanone (2.38 g, yield 98%) as pale-yellow crystals. melting point 156 - 157°C.

### Reference Example 8

To a mixture of 3-bromophenol (25.25 g), triisopropylsilyl trifluoromethanesulfonate (49.19 g) and dichloromethane (150 ml) was added dropwise 2,6-lutidine (20.40 ml) at 0°C. The reaction mixture was stirred at 0°C for 2 hr and concentrated. Ethyl acetate was added to the residue, washed with water and saturated brine, dried (MgSO₄) and concentrated. The residue was subjected to silica gel column chromatography, eluted with hexane to give 1-bromo-3-(triisopropylsilyloxy)benzene (33.30 g, yield 69%) as a colorless oil.
¹H-NMR (300 MHz, CDCl₃) δ:1.03 - 1.14 (18 H, m), 1.18 - 1.31 (3 H, m), 6.78 - 6.82 (1 H, m), 7.03 - 7.09 (3 H, m).

### Reference Example 9

To a solution of 1-bromo-3-(triisopropylsilyloxy)benzene (15.00 g) in tetrahydrofuran (100 ml) was added dropwise n-butyllithium (1.6 M hexane solution, 29.9 ml) at -78°C. The reaction mixture was stirred at -78°C for 2 hr, and a solution of 2-(methoxymethoxy)-4-methoxybenzaldehyde (8.94 g) in tetrahydrofuran (30 ml) was added dropwise at -78°C. The reaction mixture was gradually warmed to room temperature, and the mixture was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:6→1:4, v/v) to give [4-methoxy-2-(methoxymethoxy)phenyl][3-(triisopropylsilyloxy)phenyl]methanol (15.12 g, yield 74%) as a colorless oil.
¹H-NMR (300 MHz, CDCl₃)δ:1.00 - 1.11 (18 H, m), 1.12 - 1.29 (3 H, m), 2.77 (1 H, d, J=5.7 Hz), 3.34 (3 H, s), 3.78 (3 H, s), 5.11 (2 H, s), 5.95 (1 H, d, J=5.7 Hz), 6.52 (1 H, dd, J=2.4, 8.4 Hz), 6.67 (1 H, d, J=2.4 Hz), 6.72 - 6.77 (1 H, m), 6.89 - 6.94 (2 H, m), 7.11 - 7.18 (2 H, m).

### Reference Example 10

A mixture of [4-methoxy-2-(methoxymethoxy)phenyl][3-(triisopropylsilyloxy)phenyl]methanol (15.12 g), tetrabutylammonium fluoride trihydrate (12.82 g) and tetrahydrofuran (100 ml) was stirred at 0°C for 30 min, and the reaction mixture was concentrated. The residue was dissolved in N,N-dimethylformamide (50 ml), 4-(chloromethyl)-5-methyl-2-phenyl-1,3-oxazole (7.38 g) and potassium carbonate (9.36 g) were added, and the mixture was stirred at room temperature for 40 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:2, v/v) to give [4-methoxy-2-(methoxymethoxy)phenyl]{3-[(5-methyl-2-phenyl-1,3-oxazol-4-yl)methoxy]phenyl}methanol (13.22 g, yield 85%) as a colorless oil.
¹H-NMR (300 MHz, CDCl₃) δ:2.42 (3 H, s), 2.78 - 2.85 (1 H, br), 3.33 (3 H, s), 3.77 (3 H, s), 4.97 (2 H, s), 5.12 (2 H, s), 5.99 (1 H, d, J=4.8 Hz), 6.50 (1 H, dd, J=2.4, 8.4 Hz), 6.67 (1 H, d, J=2.4 Hz), 6.89 (1 H, dd, J=2.4, 8.4 Hz), 6.97 (1 H, d, J=7.5 Hz), 7.08 - 7.10 (1 H, m), 7.15 (1 H, d, J=8.4 Hz), 7.22 (1 H, d, J=8.4 Hz), 7.40 - 7.47 (3 H, m), 7.97 - 8.03 (2 H, m).

### Reference Example 11

A mixture of [4-methoxy-2-(methoxymethoxy)phenyl]{3-[(5-methyl-2-phenyl-1,3-oxazol-4-yl)methoxy]phenyl}methanol (15.10 g), manganese dioxide (28.4 g) and tetrahydrofuran (150 ml) was stirred at room temperature for 40 hr. The insoluble material was removed by a filtration operation, and the filtrate was concentrated. The residue was subjected to silica gel column chromatography, eluted with ethyl acetate-hexane (1:5→1:2, v/v), concentrated, and crystallized from ethyl acetate-hexane to give [4-methoxy-2-(methoxymethoxy)phenyl]{3-[(5-methyl-2-phenyl-1,3-oxazol-4-yl)methoxy]phenyl}methanone (9.10 g, yield 61%) as colorless crystals. melting point 117 - 118°C.

### Reference Example 12

A mixture of [4-methoxy-2-(methoxymethoxy)phenyl]{3-[(5-methyl-2-phenyl-1,3-oxazol-4-yl)methoxy]phenyl}methanone (8.50 g), 1 M hydrochloric acid (30 ml) and acetone (150 ml) was stirred at 60°C for 3 hr. The reaction mixture was concentrated, water was added to the residue, and the mixture was extracted with ethyl acetate. The ethyl acetate layer was washed with water and saturated brine, dried (MgSO₄) and concentrated. The residue was crystallized from ethyl acetate-hexane to give (2-hydroxy-4-methoxyphenyl){3-[(5-methyl-2-phenyl-1,3-oxazol-4-yl)methoxy]phenyllmethanone (6.24 g, yield 81%) as pale-yellow crystals. Recrystallization from ethyl acetate gave colorless crystals. melting point 117 - 118°C.

### Reference Example 13

To a solution of (2-hydroxy-4-methoxyphenyl){3-[(5-methyl-2-phenyl-1,3-oxazol-4-yl)methoxy]phenyl}methanone (450 mg) in tetrahydrofuran (10 ml) was added dropwise methylmagnesium bromide (1.0M tetrahydrofuran solution, 5.42 ml) at 0°C. The reaction mixture was stirred at 0°C for 3 hr, ethyl acetate was added, and the mixture was washed with water and saturated brine, dried (MgSO₄) and concentrated. The residue was subjected to silica gel column chromatography, eluted with ethyl acetate-hexane (1:3→2:3, v/v) to give 2-(1-hydroxy-1-{3-[(5-methyl-2-phenyl-1,3-oxazol-4-yl)methoxylphenyl)ethyl)-5-methoxyphenol (460 mg, yield 98%) as a colorless oil.
¹H-NMR (300 MHz, CDCl₃) δ:1.91 (3 H, s), 2.41 (3 H, s), 3.08 (1 H, s), 3.74 (3 H, s), 4.94 (2 H, s), 6.35 (1 H, dd, J=2.7, 8.7 Hz), 6.42 (1 H, d, J=2.7 Hz), 6.88 (1 H, d, J=8.7 Hz), 6.89 - 6.94 (1 H, m), 6.98 (1 H, ddd, J=1.2, 1.8, 7.8 Hz), 7.08 - 7.11 (1 H, m), 7.21 - 7.28 (1 H, m), 7.40 - 7.45 (3 H, m), 7.97 - 8.01 (2 H, m), 8.50 (1 H, s).

### Reference Example 14

To a mixture of (2-hydroxy-4-methoxyphenyl){3-[(5-methyl-2-phenyl-1,3-oxazol-4-yl)methoxy]phenyl}methanone (1.00 g), tetrahydrofuran (10 ml) and ethanol (1 ml) was added sodium borohydride (91 mg), and the mixture was stirred at room temperature for 3 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:2→2:1, v/v) to give (2-hydroxy-4-methoxyphenyl){3-[(5-methyl-2-phenyl-1,3-oxazol-4-yl)methoxy]phenyl}methanol (714 mg, yield 71%) as a colorless oil.
¹H-NMR (300 MHz, CDCl₃) δ:2.41 (3 H, s), 3.35 - 3.39 (1 H, br), 3.74 (3 H, s), 4.94 (2 H, s), 5.94 (1 H, d, J=3.3 Hz), 6.33 (1 H, dd, J=2.4, 8.4 Hz), 6.45 (1 H, d, J=2.4 Hz), 6.71 (1 H, d, J=8.4 Hz), 6.92 - 7.00 (2 H, m), 7.04 - 7.06 (1 H, m), 7.25 - 7.31 (1 H, m), 7.40 - 7.45 (3 H, m), 7.97 - 8.01 (2 H, m), 8.13 (1 H, s).

### Reference Example 15

A mixture of 2,4-dihydroxybenzaldehyde (25.20 g), potassium carbonate (42.87 g) and acetone (250 ml) was stirred while adding chloromethyl methyl ether (20.79 ml) dropwise at room temperature. The reaction mixture was stirred at room temperature for 3 hr, an insoluble material was removed by a filtration operation, and the filtrate was concentrated. The residue was subjected to silica gel column chromatography, eluted with ethyl acetate-hexane (1:6 →1:5, v/v), concentrated, and crystallized from ethyl acetate-hexane to give 2-hydroxy-4-(methoxymethoxy)benzaldehyde (24.97 g, yield 75%) as colorless crystals. melting point 51 - 52°C.

### Reference Example 16

To a mixture of (2-hydroxy-4-methoxyphenyl){3-[(5-methyl-2-phenyl-1,3-oxazol-4-yl)methoxy]phenyl}methanol (700 mg), tetrahydrofuran (5 ml) and ethanol (5 ml) was added 10% palladium carbon (1.0 g), and the mixture was stirred at room temperature for 15 hr under a nitrogen atmosphere. An insoluble material was removed by a filtration operation, and the filtrate was concentrated. The residue was subjected to silica gel column chromatography, eluted with ethyl acetate-hexane (1:4→1:1, v/v) to give ethoxy(2-hydroxy-4-methoxyphenyl){3-[(5-methyl-2-phenyl-1,3-oxazol-4-yl)methoxy]phenyl}methane (233 mg, yield 31%) as a colorless oil.
¹H-NMR (300 MHz, CDCl₃) δ:1.22 - 1.31 (3 H, m), 2.41 (3 H, s), 3.54 - 3.66 (2 H, m), 3.74 (3 H, s), 4.95 (2 H, s), 5.46 (1 H, s), 6.33 (1 H, dd, J=2.4, 8.7 Hz), 6.44 (1 H, d, J=2.4 Hz), 6.72 (1 H, d, J=8.7 Hz), 6.91 - 6.96 (2 H, m), 7.00 - 7.03 (1 H, m), 7.23 - 7.29 (1 H, m), 7.39 - 7.45 (3 H, m), 7.96 - 8.02 (2 H, m), 8.32 (1H, s).

### Reference Example 17

To a solution of 1-bromo-4-(triisopropylsilyloxy)benzene (5.00 g) in tetrahydrofuran (25 ml) was added dropwise n-butyllithium (1.6 M hexane solution, 9.96 ml) at -78°C. The reaction mixture was stirred at -78°C for 2 hr, and a solution of 2-(methoxymethoxy)benzaldehyde (2.52 g) in tetrahydrofuran (15 ml) was added dropwise at -78°C. The reaction mixture was gradually warmed to room temperature, and the mixture was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:6→1:4, v/v) to give [2-(methoxymethoxy)phenyl][4-(triisopropylsilyloxy)phenyl]methanol (4.63 g, yield 73%) as a colorless oil.
¹H-NMR (300 MHz, CDCl₃) δ:1.01 - 1.13 (18 H, m), 1.16 - 1.29 (3 H, m), 2.78 (1 H, d, J=5.4 Hz), 3.28 (3 H, s), 5.10 (1 H, d, J=11.7 Hz), 5.12 (1 H, d, J=11.7 Hz), 6.00 (1 H, d, J=5.4 Hz), 6.81 (2 H, d, J=8.4 Hz), 6.98 - 7.08 (2 H, m), 7.18 - 7.28 (3 H, m), 7.32 - 7.36 (1 H, m).

### Reference Example 18

A mixture of 2-hydroxy-4-(methoxymethoxy)benzaldehyde (12.50 g), potassium carbonate (14.22 g) and N,N-dimethylformamide (100 ml) was stirred while adding benzyl bromide (10.61 ml) dropwise at room temperature. The reaction mixture was stirred at room temperature for 5 hr, water was added, and the mixture was extracted with diisopropyl ether. The diisopropyl ether layer was washed with water and saturated brine, dried (MgSO₄) and concentrated. The residue was subjected to silica gel column chromatography, eluted with ethyl acetate-hexane (1:5 →1:3, v/v) to give 2-benzyloxy-4-(methoxymethoxy)benzaldehyde (18.44 g, yield 99%) as a colorless oil.
¹H-NMR (300 MHz, CDCl₃) δ:3.48 (3 H, s), 5.16 (2 H, s), 5.21 (2 H, s), 6.67 - 6.71 (2 H, m), 7.32 - 7.47 (5 H, m), 7.82 (1 H, d, J=9.0 Hz), 10.40 (1 H, s).

### Reference Example 19

A mixture of [2-(methoxymethoxy)phenyl][4-(triisopropylsilyloxy)phenyl]methanol (4.62 g), manganese dioxide (14.5 g) and tetrahydrofuran (40 ml) was stirred at room temperature for 40 hr. The insoluble material was removed by a filtration operation, and the filtrate was concentrated. The residue was dissolved in tetrahydrofuran (20 ml), and tetrabutylammonium fluoride trihydrate (4.20 g) was added. The mixture was stirred at room temperature for 30 min and concentrated. Water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:3 →2:1, v/v) to give (4-hydroxyphenyl)[2-(methoxymethoxy)phenyl]methanone (1.92 g, yield 67%) as a colorless oil.
¹H-NMR (300 MHz, CDCl₃) δ:3.33 (3 H, s), 5.07 (2 H, s), 6.03 (1 H, br s), 6.85 (2 H, d, J=8.4 Hz), 7.09 (1 H, t, J=7.5 Hz), 7.20 (1 H, d, J=8.7 Hz), 7.32 (1 H, dd, J=1.8, 7.5 Hz), 7.39 - 7.46 (1 H, m), 7.77 (2 H, d, J=8.4 Hz).

### Reference Example 20

To a solution of 1-bromo-4-(triisopropylsilyloxy)benzene (22.20 g) in tetrahydrofuran (120 ml) was added dropwise n-butyllithium (1.6 M hexane solution, 44.2 ml) at -78°C. The reaction mixture was stirred at -78°C for 2 hr, and a solution of 2-benzyloxy-4-(methoxymethoxy)benzaldehyde (18.35 g) in tetrahydrofuran (30 ml) was added dropwise at -78°C. The reaction mixture was gradually warmed to room temperature, and the mixture was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:6 →1:3, v/v) to give [2-benzyloxy-4-(methoxymethoxy)phenyl][4-(triisopropylsilyloxy)phenyl]methanol (27.93 g, yield 79%) as a colorless oil.
¹H-NMR (300 MHz, CDCl₃) δ:1.00 - 1.14 (18 H, m), 1.18 - 1.30 (3 H, m), 2.80 (1 H, d, J=5.7 Hz), 3.47 (3 H, s), 5.00 (2 H, s), 5.14 (2 H, s), 5.96 (1 H, d, J=5.7 Hz), 6.62 (1 H, dd, J=2.4, 8.1 Hz), 6.66 (1 H, d, J=2.4 Hz), 6.81 (2 H, d, J=8.7 Hz), 7.13 - 7.35 (8 H, m).

### Reference Example 21

A mixture of [2-benzyloxy-4-(methoxymethoxy)phenyl][4-(triisopropylsilyloxy)phenyl]methanol (27.92 g), manganese dioxide (55.7 g) and tetrahydrofuran (150 ml) was stirred at room temperature for 40 hr. The insoluble material was removed by a filtration operation, and the filtrate was concentrated. The residue was dissolved in tetrahydrofuran (50 ml), tetrabutylammonium fluoride (1.0M tetrahydrofuran solution, 58.8 ml) was added, and the mixture was stirred at room temperature for 30 min and concentrated. Water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:3→1:1, v/v), concentrated, and crystallized from ethyl acetate-diisopropyl ether to give [2-(benzyloxy)-4-(methoxymethoxy)phenyl](4-hydroxyphenyl)methanone (8.09 g, yield 42%) as colorless crystals. melting point 114 - 115°C.

### Reference Example 22

A mixture of 4-(chloromethyl)-5-methyl-2-phenyl-1,3-oxazole (1.77 g), (4-hydroxyphenyl)[2-(methoxymethoxy)phenyl]methanone (1.91 g), potassium carbonate (1.33 g) and N,N-dimethylformamide (20 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:6→1:2, v/v) to give [2-(methoxymethoxy)phenyl]{4-[(5-methyl-2-phenyl-1,3-oxazol-4-yl)methoxy]phenyl}methanone (2.84 g, yield 89%) as a colorless oil.
¹H-NMR (300 MHz, CDCl₃) δ:2.45 (3 H, s), 3.32 (3 H, s), 5.06 (2 H, s), 5.07 (2 H, s), 7.01 - 7.12 (3 H, m), 7.21 (1 H, d, J=8.1 Hz), 7.34 (1 H, dd, J=1.8, 7.5 Hz), 7.38 - 7.47 (4 H, m), 7.83 (2 H, d, J=9.0 Hz), 7.97 - 8.03 (2 H, m).

### Reference Example 23

A mixture of 4-(chloromethyl)-5-methyl-2-phenyl-1,3-oxazole (3.45 g), [2-(benzyloxy)-4-(methoxymethoxy)phenyl](4-hydroxyphenyl)methanone (5.51 g), potassium carbonate (3.13 g) and N,N-dimethylformamide (50 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→2:3, v/v) to give [2-(benzyloxy)-4-(methoxymethoxy)phenyl]{4-[(5-methyl-2-phenyl-1,3-oxazol-4-yl)methoxy]phenyl}methanone (6.47 g, yield 80%) as a pale-brown oily substance.
¹H-NMR (300 MHz, CDCl₃) δ:2.44 (3 H, s), 3.50 (3 H, s), 5.00 (2 H, s), 5.04 (2 H, s), 5.20 (2 H, s), 6.70 - 6.74 (2 H, m), 7.00 - 7.08 (4 H, m), 7.16 - 7.26 (3 H, m), 7.38 (1 H, d, J=8.4 Hz), 7.40 - 7.47 (3 H, m), 7.80 (2 H, d, J=8.7 Hz), 7.98 - 8.04 (2 H, m).

### Reference Example 24

A mixture of [2-(methoxymethoxy)phenyl]{4-[(5-methyl-2-phenyl-1,3-oxazol-4-yl)methoxy]phenyl}methanone (2.69 g), 1 M hydrochloric acid (10 ml) and acetone (50 ml) was stirred at 60°C for 15 hr. The reaction mixture was concentrated, water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:6→1:2, v/v), concentrated, and crystallized from ethyl acetate-hexane to give (2-hydroxyphenyl){4-[(5-methyl-2-phenyl-1,3-oxazol-4-yl)methoxy]phenyl}methanone (1.98 g, yield 82%) as pale-yellow crystals. melting point 121 - 122°C.

### Reference Example 25

A mixture of [2-(benzyloxy)-4-(methoxymethoxy)phenyl]{4-[(5-methyl-2-phenyl-1,3-oxazol-4-yl)methoxy]phenyl}methanone (6.25 g), 5% palladium carbon (2.00 g) and ethyl acetate (50 ml) was stirred at room temperature for 3 hr under a hydrogen atmosphere. The insoluble material was removed by a filtration operation, and the filtrate was concentrated. The residue was subjected to silica gel column chromatography, eluted with ethyl acetate-hexane (1:6→2:3, v/v), concentrated, and crystallized from ethyl acetate-hexane to give [2-hydroxy-4-(methoxymethoxy)phenyl]{4-[(5-methyl-2-phenyl-1,3-oxazol-4-yl)methoxy]phenyl}methanone (3.97 g, yield 76%) as colorless crystals. melting point 105 - 107°C.

### Reference Example 26

To a mixture of 6-chloronicotinoylchloride (57.22 g), 3-methoxyphenol (40.36 g) and nitrobenzene (400 ml) was gradually added aluminum chloride (65.00 g) at 0°C. The reaction mixture was stirred at 0°C for 3 hr, and then at room temperature for 15 hr. The reaction mixture was poured onto ice, 1 M hydrochloric acid and ethyl acetate were added, and the mixture was vigorously stirred at room temperature for 2 hr. Two layers were separated, and the ethyl acetate layer was washed with water and saturated brine, dried (MgSO₄) and concentrated. Diisopropyl ether-hexane was added to the residue, and the resulting crystals were collected by filtration to give (6-chloropyridin-3-yl)(2-hydroxy-4-methoxyphenyl)methanone (48.08 g, yield 56%) as pale-yellow crystals. melting point 138 - 139°C.

### Reference Example 27

To a mixture of (6-chloropyridin-3-yl)(2-hydroxy-4-methoxyphenyl)methanone (20.3 g), benzyl bromide (10.1 ml) and N,N-dimethylformamide (100 ml) was added potassium carbonate (12.8 g) at 0°C. The reaction mixture was stirred at room temperature for 3 hr and concentrated. Ethyl acetate was added to the residue, washed with water and saturated brine, dried (MgSO₄) and concentrated. The residue was crystallized from ethyl acetate-hexane to give [2-(benzyloxy)-4-methoxyphenyl](6-chloropyridin-3-yl)methanone (18.7 g, yield 69%) as pale-yellow crystals. melting point 93 - 94°C.

### Reference Example 28

A mixture of 4-(hydroxymethyl)-5-methyl-2-phenyl-1,3-oxazole (393 mg), [2-(benzyloxy)-4-methoxyphenyl](6-chloropyridin-3-yl)methanone (700 mg) and N,N-dimethylformamide (10 ml) was stirred at 0°C while adding 60% sodium hydride (oily, 83 mg). The reaction mixture was stirred at 0°C for 2 hr, ethyl acetate was added, and the mixture was washed with water and saturated brine, dried (MgSO₄) and concentrated. The residue was subjected to silica gel column chromatography, eluted with ethyl acetate-hexane (1:4→1:2, v/v) to give [2-(benzyloxy)-4-methoxyphenyl]{6-[(5-methyl-2-phenyl-1,3-oxazol-4-yl)methoxy]pyridin-3-yl}methanone (720 mg, yield 72%) as a colorless oil.
¹H-NMR (300 MHz, CDCl₃) δ:2.47 (3 H, s), 3.86 (3 H, s), 4.99 (2 H, s), 5.35 (2 H, s), 6.56 (1 H, d, J=2.4 Hz), 6.61 (1 H, dd, J=2.4, 8.4 Hz), 6.81 (1 H, d, J=8.4 Hz), 7.00 - 7.05 (2 H, m), 7.13 - 7.23 (3 H, m), 7.40 - 7.46 (3 H, m), 7.50 (1 H, d, J=8.4 Hz), 7.99 - 8.05 (3 H, m), 8.56 (1 H, d, J=2.4 Hz).

### Reference Example 29

A mixture of [2-(benzyloxy)-4-methoxyphenyl]{6-[(5-methyl-2-phenyl-1,3-oxazol-4-yl)methoxylpyridin-3-yl}methanone (660 mg), 5% palladium carbon (100 mg) and ethyl acetate (20 ml) was stirred at room temperature for 5 hr under a hydrogen atmosphere. The insoluble material was removed by a filtration operation, and the filtrate was concentrated. The residue was subjected to silica gel column chromatography, eluted with ethyl acetate-hexane (1:5→1:2, v/v), concentrated, and crystallized from ethyl acetate-hexane to give (2-hydroxy-4-methoxyphenyl){6-[(5-methyl-2-phenyl-1,3-oxazol-4-yl)methoxy]pyridin-3-yl}methanone (391 mg, yield 72%) as pale-yellow crystals. melting point 137 - 138°C.

### Reference Example 30

To a mixture of methyl 2-hydroxy-4-methoxybenzoate (50.27 g), potassium carbonate (76.27 g) and N,N-dimethylformamide (200 ml) was added dropwise chloromethyl methyl ether (37.73 ml) at room temperature. The reaction mixture was stirred at 60°C for 6 hr and concentrated. Ethyl acetate was added to the residue, washed with water and saturated brine, dried (MgSO₄) and concentrated. The residue was subjected to silica gel column chromatography, eluted with ethyl acetate-hexane (1:3, v/v) and concentrated. The residue was dissolved in methanol (200 ml), a solution of sodium hydroxide (33.11 g) in water (200 ml) was added, and the mixture was stirred at 60°C for 1 hr. The reaction mixture was concentrated, and neutralized with dil. hydrochloric acid. The precipitated crystals were collected by filtration, washed with water and dried. Recrystallization from ethyl acetate-hexane gave 4-methoxy-2-(methoxymethoxy)benzoic acid (34.67 g, yield 59%) as colorless crystals. melting point 96 - 97°C.

### Reference Example 31

To a mixture of N,O-dimethylhydroxylamine hydrochloride (17.34 g) and N,N-dimethylformamide (200 ml) was added triethylamine (17.99 g), and the mixture was stirred at room temperature for 30 min. 4-Methoxy-2-(methoxymethoxy)benzoic acid (34.30 g), 1-ethyl-3-(3-dimethylaminopropyl)carbodiimide hydrochloride (40.52 g) and 1-hydroxybenzotriazole (27.23 g) were added to the reaction mixture, and the mixture was stirred at room temperature for 15 hr and concentrated. Ethyl acetate was added to the residue, and the mixture was successively washed with water and aqueous potassium carbonate solution, dried (MgSO₄) and concentrated. The residue was subjected to silica gel column chromatography, eluted with ethyl acetate-hexane (1:1→10:1, v/v) to give 4,N-dimethoxy-2-(methoxymethoxy)-N-methylbenzamide (23.60 g, yield 57%) as a colorless oil.
¹H-NMR (300 MHz, CDCl₃) δ:3.29 (3 H, s), 3.48 (3 H, s), 3.50 - 3.70 (3 H, br), 3.81 (3 H, s), 5.18 (2 H, s), 6.57 (1 H, dd, J=2.4, 8.4 Hz), 6.73 (1 H, d, J=2.4 Hz), 7.23 (1 H, d, J=8.4 Hz).

### Reference Example 32

To a solution of 4-(benzyloxy)-1-bromobenzene (21.65 g) in tetrahydrofuran (200 ml) was added dropwise n-butyllithium (1.6 M hexane solution, 53.9 ml) at -78°C. The reaction mixture was stirred at -78°C for 90 min, and a solution of 4,N-dimethoxy-2-(methoxymethoxy)-N-methylbenzamide (20.00 g) in tetrahydrofuran (50 ml) was added dropwise at -78°C. The reaction mixture was gradually warmed to room temperature, and the mixture was stirred at room temperature for 10 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:3→2:3, v/v) to give [4-methoxy-2-(methoxymethoxy)phenyl][4-(benzyloxy)phenyl]methanone (21.61 g, yield 73%) as a colorless oil.
¹H-NMR (300 MHz, CDCl₃) δ:3.32 (3 H, s), 3.85 (3 H, s), 5.04 (2 H, s), 5.13 (2 H, s), 6.62 (1 H, dd, J=2.1, 8.4 Hz), 6.76 (1 H, d, J=2.1 Hz), 6.98 (2 H, d, J=9.0 Hz), 7.30 - 7.46 (6 H, m), 7.80 (2 H, d, J=9.0 Hz).

### Reference Example 33

A mixture of [4-methoxy-2-(methoxymethoxy)phenyl][4-(benzyloxy)phenyl]methanone (21.60 g), 1 M hydrochloric acid (86 ml) and acetone (300 ml) was stirred at 60°C for 5 hr. The reaction mixture was concentrated, water was added to the residue, and the precipitated crystals were collected by a filtration operation, washed with water and dried to give [4-(benzyloxy)phenyl](2-hydroxy-4-methoxyphenyl)methanone (18.04 g, yield 95 %) as pale-yellow crystals. melting point 124 - 125°C.

### Reference Example 34

A mixture of tert-butyl 2-{2-[4-(benzyloxy)benzoyl]-5-methoxyphenoxy}-2-methylpropanoate (11.79 g), 5% palladium carbon (4.00 g) and tetrahydrofuran (100 ml) was stirred at room temperature for 2 hr under a hydrogen atmosphere. The insoluble material was removed by a filtration operation, and the filtrate was concentrated. The residue was crystallized from ethyl acetate-hexane to give tert-butyl 2-[2-(4-hydroxybenzoyl)-5-methoxyphenoxy]-2-methylpropanoate (8.21 g, yield 86%) as colorless crystals. melting point 172 - 173°C.
¹H-NMR (300 MHz, CDCl₃) δ:1.31 (6 H, s), 1.46 (9 H, s), 3.80 (3 H, s), 5.67 (1H, s), 6.33 (1H, d, J=2.1 Hz), 6.58 (1H, dd, J=2.1, 8.4 Hz), 6.83 (2 H, d, J=8.7 Hz), 7.42 (1 H, d, J=8.4 Hz), 7.76 (2 H, d, J=8.7 Hz).

### Reference Example 35

To a mixture of N,O-dimethylhydroxylamine hydrochloride (6.38 g) and N,N-dimethylformamide (100 ml) was added triethylamine (6.62 g), and the mixture was stirred at room temperature for 30 min. 5-Methoxysalicylic acid (10.00 g), 1-ethyl-3-(3-dimethylaminopropyl)carbodiimide hydrochloride (12.54 g) and 1-hydroxybenzotriazole (10.02 g) were added to the reaction mixture, and the mixture was stirred at room temperature for 15 hr. Ethyl acetate was added to the reaction mixture, and the mixture was washed with water and aqueous potassium carbonate solution, dried (MgSO₄) and concentrated. The residue was subjected to silica gel column chromatography, eluted with ethyl acetate-hexane (1:4→1:1, v/v) to give 2-hydroxy-N,5-dimethoxy-N-methylbenzamide (9.73 g, yield 77%) as a colorless oil.
¹H-NMR (300 MHz, CDCl₃) δ:3.41 (3 H, s), 3.66 (3 H, s), 3.76 (3 H, s), 6.92 (1 H, d, J=9.0 Hz), 7.01 (1 H, dd, J=3.0, 9.0 Hz), 7.51 (1 H, d, J=3.0 Hz), 10.59 (1 H, s).

### Reference Example 36

To a mixture of N,O-dimethylhydroxylamine hydrochloride (6.38 g) and N,N-dimethylformamide (100 ml) was added triethylamine (6.62 g), and the mixture was stirred at room temperature for 30 min. 3-Methoxysalicylic acid (10.00 g), 1-ethyl-3-(3-dimethylaminopropyl)carbodiimide hydrochloride (12.54 g) and 1-hydroxybenzotriazole (10.02 g) was added to the reaction mixture, and the mixture was stirred at room temperature for 15 hr. Ethyl acetate was added to the reaction mixture, and the mixture was successively washed with water and aqueous potassium carbonate solution, dried (MgSO₄) and concentrated. The residue was subjected to silica gel column chromatography, eluted with ethyl acetate-hexane (1:2→2:1, v/v) to give 2-hydroxy-N,3-dimethoxy-N-methylbenzamide (8.23 g, yield 65%) as a colorless oil.
¹H-NMR (300 MHz, CDCl₃) δ:3.40 (3 H, s), 3.65 (3 H, s), 3.91 (3 H, s), 6.81 (1 H, dd, J=7.8, 8.4 Hz), 6.97 (1 H, dd, J=1.2, 7.8 Hz), 7.45 (1 H, dd, J=1.2, 8.4 Hz), 10.49 (1 H, s).

### Reference Example 37

To a mixture of 2-hydroxy-N,5-dimethoxy-N-methylbenzamide (9.72 g) and N,N-dimethylformamide (70 ml) was added 60% sodium hydride (oily, 2.02 g) at 0°C, and the mixture was stirred for 1 hr. Chloromethyl methyl ether (4.66 ml) was added dropwise to the reaction mixture, and the mixture was stirred at 0°C for 1 hr. Ethyl acetate was added, and the mixture was washed with water and saturated brine, dried (MgSO₄) and concentrated. The residue was subjected to silica gel column chromatography, eluted with ethyl acetate-hexane (1:2→3:2, v/v) to give N,5-dimethoxy-2-(methoxymethoxy)-N-methylbenzamide (9.64 g, yield 82%) as a colorless oil.
¹H-NMR (300 MHz, CDCl₃) δ:3.10 - 3.90 (6 H, br), 3.48 (3 H, s), 3.78 (3 H, s), 5.12 (2 H, s), 6.83 - 6.87 (1 H, m), 6.89 (1 H, d, J=3.0 Hz), 7.10 (1 H, d, J=9.0 Hz).

### Reference Example 38

To a mixture of 2-hydroxy-N,3-dimethoxy-N-methylbenzamide (8.22 g) and N,N-dimethylformamide (60 ml) was added 60% sodium hydride (oily, 1.71 g) at 0°C, and the mixture was stirred for 1 hr. Chloromethyl methyl ether (3.94 ml) was added dropwise to the reaction mixture, and the mixture was stirred at 0°C for 1 hr. Ethyl acetate was added, and the mixture was washed with water and saturated brine, dried (MgSO₄) and concentrated. The residue was subjected to silica gel column chromatography, eluted with ethyl acetate-hexane (1:1→3:1, v/v) to give N,3-dimethoxy-2-(methoxymethoxy)-N-methylbenzamide (3.58 g, yield 36%) as a colorless oil.
¹H-NMR (300 MHz, CDCl₃) δ:3.10 - 3.60 (6 H, br), 3.54 (3 H, s), 3.86 (3 H, s), 5.14 (2 H, s), 6.91 (1 H, dd, J=1.8, 7.8 Hz), 6.96 (1 H, dd, J=1.8, 8.4 Hz), 7.11 (1 H, dd, J=7.8, 8.4 Hz).

### Reference Example 39

To a solution of 4-(benzyloxy)-1-bromobenzene (10.42 g) in tetrahydrofuran (100 ml) was added dropwise n-butyllithium (1.6 M hexane solution, 25.9 ml) at -78°C. The reaction mixture was stirred at -78°C for 90 min, and a solution of N,5-dimethoxy-2-(methoxymethoxy)-N-methylbenzamide (9.63 g) in tetrahydrofuran (50 ml) was added dropwise at -78°C. The reaction mixture was gradually warmed to room temperature, and the mixture was stirred at room temperature for 15 hr and concentrated. Water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:6→1:3, v/v) to give [4-(benzyloxy)phenyl][5-methoxy-2-(methoxymethoxy)phenyl]methanone (8.14 g, yield 57%) as a colorless oil.
¹H-NMR (300 MHz, CDCl₃) δ:3.30 (3 H, s), 3.78 (3 H, s), 4.98 (2 H, s), 5.13 (2 H, s), 6.86 (1 H, d, J=3.3 Hz), 6.92 - 7.02 (3 H, m), 7.14 (1 H, d, J=9.0 Hz), 7.30 - 7.45 (5 H, m), 7.83 (2 H, d, J=9.0 Hz).

### Reference Example 40

To a solution of 4-(benzyloxy)-1-bromobenzene (4.05 g) in tetrahydrofuran (50 ml) was added dropwise n-butyllithium (1.6 M hexane solution, 10.1 ml) at -78°C. The reaction mixture was stirred at -78°C for 90 min, and a solution of N,3-dimethoxy-2-(methoxymethoxy)-N-methylbenzamide (3.57 g) in tetrahydrofuran (30 ml) was added dropwise at -78°C. The reaction mixture was gradually warmed to room temperature, and the mixture was stirred at room temperature for 15 hr and concentrated. Water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:2, v/v) to give [4-(benzyloxy)phenyl][3-methoxy-2-(methoxymethoxy)phenyl]methanone (2.48 g, yield 47%) as a colorless oil.
¹H-NMR (300 MHz, CDCl₃) δ:3.20 (3 H, s), 3.90 (3 H, s), 4.98 (2 H, s), 5.12 (2 H, s), 6.90 - 7.06 (4 H, m), 7.12 - 7.18 (1 H, m), 7.30 - 7.45 (5 H, m), 7.84 (2 H, d, J=9.0 Hz).

### Reference Example 41

A mixture of [4-(benzyloxy)phenyl][5-methoxy-2-(methoxymethoxy)phenyl]methanone (8.13 g), 5% palladium carbon (1.00 g) and tetrahydrofuran (50 ml) was stirred at room temperature for 5.5 hr under a hydrogen atmosphere. The insoluble material was removed by a filtration operation, and the filtrate was concentrated. The residue was subjected to silica gel column chromatography, eluted with ethyl acetate-hexane (1:2→2:1, v/v) to give (4-hydroxyphenyl)[5-methoxy-2-(methoxymethoxy)phenyl]methanone (1.92 g, yield 31%) as a colorless oil.
¹H-NMR (300 MHz, CDCl₃) δ:3.32 (3 H, s), 3.78 (3 H, s), 4.98 (2 H, s), 5.90 - 6.05 (1 H, br), 6.83 - 6.88 (3 H, m), 6.96 (1 H, dd, J=3.0, 9.0 Hz), 7.14 (1 H, d, J=9.0 Hz), 7.78 (2 H, d, J=8.7 Hz).

### Reference Example 42

A mixture of 4-(chloromethyl)-5-methyl-2-phenyl-1,3-oxazole (1.58 g), (4-hydroxyphenyl)[5-methoxy-2-(methoxymethoxy)phenyl]methanone (1.91 g), potassium carbonate (1.37 g) and N,N-dimethylformamide (15 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:2, v/v) to give [5-methoxy-2-(methoxymethoxy)phenyl]{4-[(5-methyl-2-phenyl-1,3-oxazol-4-yl)methoxy]phenyl}methanone (2.57 g, yield 84%) as a colorless oil.
¹H-NMR (300 MHz, CDCl₃) δ:2.45 (3 H, s), 3.31 (3 H, s), 3.78 (3 H, s), 4.98 (2 H, s), 5.06 (2 H, s), 6.87 (1 H, d, J=3.0 Hz), 6.96 (1 H, dd, J=3.0, 9.0 Hz), 7.04 (2 H, d, J=8.7 Hz), 7.14 (1 H, d, J=9.0 Hz), 7.41 - 7.47 (3 H, m), 7.84 (2 H, d, J=8.7 Hz), 7.97 -8.04 (2 H, m).

### Reference Example 43

A mixture of [5-methoxy-2-(methoxymethoxy)phenyl]{4-[(5-methyl-2-phenyl-1,3-oxazol-4-yl)methoxy]phenyl}methanone (2.56 g), 1 M hydrochloric acid (6 ml) and acetone (20 ml) was stirred at 60°C for 4 hr. The reaction mixture was concentrated, water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→2:3, v/v) to give (2-hydroxy-5-methoxyphenyl){4-[(5-methyl-2-phenyl-1,3-oxazol-4-yl)methoxy]phenyl}methanone (2.11 g, yield 91%) as a pale-yellow oil.
¹H-NMR (300 MHz, CDCl₃) δ:2.47 (3 H, s), 3.72 (3 H, s), 5.09 (2 H, s), 7.01 (1 H, d, J=8.7 Hz), 7.08 - 7.15 (4 H, m), 7.42 - 7.46 (3 H, m), 7.75 (2 H, d, J=8.7 Hz), 8.00 - 8.04 (2 H, m), 11.47 (1 H, s).

### Reference Example 44

A mixture of [4-(benzyloxy)phenyl][3-methoxy-2-(methoxymethoxy)phenyl]methanone (2.47 g), 5% palladium carbon (300 mg) and tetrahydrofuran (20 ml) was stirred at room temperature for 3 hr under a hydrogen atmosphere. The insoluble material was removed by a filtration operation, and the filtrate was concentrated. The residue was subjected to silica gel column chromatography, eluted with ethyl acetate-hexane (1:3→1:1, v/v) to give (4-hydroxyphenyl)[3-methoxy-2-(methoxymethoxy)phenyl]methanone (1.61 g, yield 85%) as a colorless oil.
¹H-NMR (300 MHz, CDCl₃) δ:3.22 (3 H, s), 3.89 (3 H, s), 4.98 (2 H, s), 6.21 - 6.30 (1 H, br), 6.82 (2 H, d, J=8.7 Hz), 6.92 (1 H, dd, J=1.8, 7.2 Hz), 7.04 (1 H, dd, J=1.8, 8.4 Hz), 7.12 - 7.18 (1 H, m), 7.78 (2 H, d, J=8.7 Hz).

### Reference Example 45

A mixture of 4-(chloromethyl)-5-methyl-2-phenyl-1,3-oxazole (1.33 g), (4-hydroxyphenyl)[3-methoxy-2-(methoxymethoxy)phenyl]methanone (1.60 g), potassium carbonate (1.15 g) and N,N-dimethylformamide (10 ml) was stirred at room temperature for 18 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:3→1:1, v/v) to give [3-methoxy-2-(methoxymethoxy)phenyl]{4-[(5-methyl-2-phenyl-1,3-oxazol-4-yl)methoxy]phenyl}methanone (2.18 g, yield 85%) as a colorless oil.
¹H-NMR (300 MHz, CDCl₃) δ:2.45 (3 H, s), 3.21 (3 H, s), 3.90 (3 H, s), 4.98 (2 H, s), 5.05 (2 H, s), 6.93 (1 H, dd, J=1.8, 7.5 Hz), 7.00 -7.06 (3 H, m), 7.12 -7.18 (1 H, m), 7.41 -7.47 (3 H, m), 7.86 (2 H, d, J=9.0 Hz), 7.98 -8.03 (2 H, m).

### Reference Example 46

A mixture of [3-methoxy-2-(methoxymethoxy)phenyl]{4-[(5-methyl-2-phenyl-1,3-oxazol-4-yl)methoxy]phenyl}methanone (2.17 g), 1 M hydrochloric acid (5 ml) and acetone (15 ml) was stirred at 60°C for 4 hr. The reaction mixture was concentrated, water was added to the residue, and the mixture was extracted with ethyl acetate. The ethyl acetate layer was washed with water and saturated brine, dried (MgSO₄) and concentrated. The residue was crystallized from ethyl acetate-hexane to give (2-hydroxy-3-methoxyphenyl){4-[(5-methyl-2-phenyl-1,3-oxazol-4-yl)methoxy]phenyl}methanone (1.63 g, yield 83%) as yellow crystals. melting point 152 - 153°C.

### Reference Example 47

To a solution of 4-(benzyloxy)-l-bromo-2-methylbenzene (4.10 g) in tetrahydrofuran (35 ml) was added dropwise n-butyllithium (1.6 M hexane solution, 9.7 ml) at -78°C. The reaction mixture was stirred at -78°C for 90 min and a solution of N,4-dimethoxy-2-(methoxymethoxy)-N-methylbenzamide (3.60 g) in tetrahydrofuran (15 ml) was added dropwise at -78°C. The reaction mixture was gradually warmed to room temperature, and the mixture was stirred at room temperature for 3 hr and concentrated. Water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:2, v/v) to give [4-(benzyloxy)-2-methylphenyl][4-methoxy-2-(methoxymethoxy)phenyl]methanone (3.61 g, yield 65%) as a colorless oil.
¹H-NMR (300 MHz, CDCl₃) δ:2.49 (3 H, s), 3.27 (3 H, s), 3.84 (3 H, s), 4.99 (2 H, s), 5.10 (2 H, s), 6.58 (1 H, dd, J=2.4, 8.4 Hz), 6.70 (1 H, d, J=2.4 Hz), 6.72 (1 H, dd, J=2.4, 8.7 Hz), 6.85 (1 H, d, J=2.4 Hz), 7.30 - 7.45 (7 H, m).

### Reference Example 48

A mixture of [4-(benzyloxy)-2-methylphenyl][4-methoxy-2-(methoxymethoxy)phenyl]methanone (3.60 g), 5% palladium carbon (500 mg) and tetrahydrofuran (30 ml) was stirred at room temperature for 5 hr under a hydrogen atmosphere. The insoluble material was removed by a filtration operation, and the filtrate was concentrated. The residue was subjected to silica gel column chromatography, eluted with ethyl acetate-hexane (1:2→2:1, v/v) to give (4-hydroxy-2-methylphenyl)[4-methoxy-2-(methoxymethoxy)phenyl]methanone (2.75 g, yield 99%) as a colorless oil.
¹H-NMR (300 MHz, CDCl₃) δ:2.46 (3 H, s), 3.31 (3 H, s), 3.85 (3 H, s), 5.01 (2 H, s), 5.50 - 5.85 (1 H, br), 6.56 - 6.63 (2 H, m), 6.69 - 6.71 (2 H, m), 7.29 (1 H, d, J=8.4 Hz), 7.40 (1 H, d, J=8.4 Hz).

### Reference Example 49

A mixture of 4-(chloromethyl)-5-methyl-2-phenyl-1,3-oxazole (948 mg), (4-hydroxy-2-methylphenyl)[4-methoxy-2-(methoxymethoxy)phenyl]methanone (1.20 g), potassium carbonate (823 mg) and N,N-dimethylformamide (10 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), concentrated, and crystallized from ethyl acetate-hexane to give [4-methoxy-2-(methoxymethoxy)phenyl]{2-methyl-4-[(5-methyl-2-phenyl-1,3-oxazol-4-yl)methoxy]phenyl}methanone (1.35 g, yield 72%) as colorless crystals. melting point 107 - 108°C.

### Reference Example 50

A mixture of [4-methoxy-2-(methoxymethoxy)phenyl]{2-methyl-4-[(5-methyl-2-phenyl-1,3-oxazol-4-yl)methoxy]phenyl}methanone (1.25 g), 1 M hydrochloric acid (3 ml) and acetone (10 ml) was stirred at 60°C for 4 hr. The reaction mixture was concentrated, water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), concentrated, and crystallized from ethyl acetate-hexane to give (2-hydroxy-4-methoxyphenyl){2-methyl-4-[(5-methyl-2-phenyl-1,3-oxazol-4-yl)methoxy]phenyl}methanone (1.11 g, yield 97%) as colorless crystals. melting point 109 - 110°C.

### Reference Example 51

A mixture of ethyl 4-methoxy-2-(methoxymethoxy)-6-methylbenzoate (8.11 g), sodium hydroxide (3.83 g), ethanol (30 ml) and water (30 ml) was stirred at 60°C for 6 days and concentrated. The residue was neutralized with dil. hydrochloric acid, and extracted with ethyl acetate. The ethyl acetate layer was washed with water and saturated brine, dried (MgSO₄) and concentrated. The residue was crystallized from ethyl acetate-hexane to give 4-methoxy-2-(methoxymethoxy)-6-methylbenzoic acid (6.56 g, yield 91%) as colorless crystals. melting point 111 - 112°C.

### Reference Example 52

To a mixture of N,O-dimethylhydroxylamine hydrochloride (3.06 g) and N,N-dimethylformamide (40 ml) was added triethylamine (3.18 g), and the mixture was stirred at room temperature for 30 min. 4-Methoxy-2-(methoxymethoxy)-6-methylbenzoic acid (6.45 g), 1-ethyl-3-(3-dimethylaminopropyl)carbodiimide hydrochloride (6.02 g) and 1-hydroxybenzotriazole (4.81 g) were added to the reaction mixture, and the mixture was stirred at room temperature for 15 hr and concentrated. Ethyl acetate was added to the residue, successively washed with water and aqueous sodium hydrogen carbonate solution, dried (MgSO₄) and concentrated. The residue was subjected to silica gel column chromatography, eluted with ethyl acetate-hexane (1:2→2:1, v/v) to give N,4-dimethoxy-2-(methoxymethoxy)-N,6-dimethylbenzamide (6.71 g, yield 87%) as a colorless oil.
¹H-NMR (300 MHz, CDCl₃) δ:2.25 (2.25 H, s), 2.27 (0.75 H, s), 3.11 (0.75 H, s), 3.37 (2.25 H, s), 3.45 (2.25 H, s), 3.46 (3 H, s), 3.79 (3 H, s), 3.88 (0.75 H, s), 5.15 (2 H, s), 6.38 - 6.41 (1 H, m), 6.51 - 6.58 (1 H, m).

### Reference Example 53

To a solution of lithium aluminum hydride (528 mg) in tetrahydrofuran (40 ml) was added dropwise a solution of N,4-dimethoxy-2-(methoxymethoxy)-N,6-dimethylbenzamide (3.75 g) in tetrahydrofuran (20 ml) at 0°C. The reaction mixture was stirred at 0°C for 4 hr, and at room temperature for 15 hr, and cooled to 0°C. Sodium fluoride (2.33 g) and water (0.77 ml) were added to the reaction mixture, and the mixture was stirred at room temperature for 30 min. The insoluble material was removed by a filtration operation, and the filtrate was concentrated. The residue was subjected to silica gel column chromatography, eluted with ethyl acetate-hexane (1:5→1:2, v/v) to give 4-methoxy-2-(methoxymethoxy)-6-methylbenzaldehyde (2.07 g, yield 71%) as a colorless oil. ¹H-NMR (300 MHz, CDCl₃) δ:2.58 (3 H, s), 3.51 (3 H, s), 3.84 (3 H, s), 5.26 (2 H, s), 6.38 - 6.40 (1 H, m), 6.58 (1 H, d, J=2.1 Hz), 10.52 (1 H, s).

### Reference Example 54

To a solution of 4-(benzyloxy)-1-bromobenzene (2.71 g) in tetrahydrofuran (30 ml) was added dropwise n-butyllithium (1.6 M hexane solution, 6.4 ml) at -78°C. The reaction mixture was stirred at -78°C for 90 min and a solution of 4-methoxy-2-(methoxymethoxy)-6-methylbenzaldehyde (2.06 g) in tetrahydrofuran (10 ml) was added dropwise at -78°C. The reaction mixture was gradually warmed to room temperature, and the mixture was stirred at room temperature for 15 hr. Water was added to the reaction mixture, and the mixture was extracted with ethyl acetate. The ethyl acetate layer was washed with water and saturated brine, dried (MgSO₄) and concentrated. The residue was dissolved in tetrahydrofuran (30 ml), manganese dioxide (8.52 g) was added, and the mixture was stirred at room temperature for 48 hr. The insoluble material was removed by a filtration operation, and the filtrate was concentrated. The residue was subjected to silica gel column chromatography, eluted with ethyl acetate-hexane (1:6→1:2, v/v), concentrated, and crystallized from ethyl acetate-hexane to give [4-(benzyloxy)phenyl][4-methoxy-2-(methoxymethoxy)-6-methylphenyl]methanone (2.03 g, yield 53%) as colorless crystals. melting point 49 - 50°C.

### Reference Example 55

A mixture of [4-(benzyloxy)phenyl][4-methoxy-2-(methoxymethoxy)-6-methylphenyl]methanone (2.48 g), 5% palladium carbon (400 mg) and tetrahydrofuran (30 ml) was stirred at room temperature for 2 hr under a hydrogen atmosphere. The insoluble material was removed by a filtration operation, and the filtrate was concentrated. The residue was subjected to silica gel column chromatography, eluted with ethyl acetate-hexane (1:3→1:1, v/v) to give (4-hydroxyphenyl)[4-methoxy-2-(methoxymethoxy)-6-methylphenyl]methanone (1.89 g, yield 99%) as a colorless oil. ¹H-NMR (300 MHz, CDCl₃) δ:2.13 (3 H, s), 3.26 (3 H, s), 3.82 (3 H, s), 5.00 (2 H, s), 6.10 - 6.60 (1 H, br), 6.43 - 6.45 (1 H, m), 6.58 - 6.59 (1 H, m), 6.84 (2 H, d, J=8.7 Hz), 7.75 (2 H, d, J=8.7 Hz).

### Reference Example 56

A mixture of 4-(chloromethyl)-5-methyl-2-phenyl-1,3-oxazole (1.55 g), (4-hydroxyphenyl)[4-methoxy-2-(methoxymethoxy)-6-methylphenyl]methanone (1.88 g), potassium carbonate (1.29 g) and N,N-dimethylformamide (10 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→2:3, v/v) to give [4-methoxy-2-(methoxymethoxy)-6-methylphenyl]{4-[(5-methyl-2-phenyl-1,3-oxazol-4-yl)methoxy]phenyl}methanone (2.48 g, yield 84%) as a colorless amorphous form.
¹H-NMR (300 MHz, CDCl₃) δ:2.14 (3 H, s), 2.45 (3 H, s), 3.25 (3 H, s), 3.82 (3 H, s), 5.01 (2 H, s), 5.05 (2 H, s), 6.45 (1 H, d, J=2.4 Hz), 6.59 (1 H, d, J=2.4 Hz), 7.04 (2 H, d, J=8.7 Hz), 7.40- 7.47 (3 H, m), 7.82 (2 H, d, J=8.7 Hz), 7.98- 8.03 (2 H, m) .

### Reference Example 57

A mixture of [4-methoxy-2-(methoxymethoxy)-6-methylphenyl]{4-[(5-methyl-2-phenyl-1,3-oxazol-4-yl)methoxy]phenyl}methanone (2.47 g), 1 M hydrochloric acid (6 ml) and acetone (20 ml) was stirred at 50°C for 15 hr. The reaction mixture was concentrated, water was added to the residue, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v) to give (2-hydroxy-4-methoxy-6-methylphenyl){4-[(5-methyl-2-phenyl-1,3-oxazol-4-yl)methoxy]phenyl}methanone(850 mg, yield 38%) as colorless crystals. The crystals were recrystallized from ethyl acetate. melting point 171 - 172°C.

### Reference Example 58

To a solution (50 ml) of (6-chloropyridin-3-yl)(2-hydroxy-4-methoxyphenyl)methanone (4.17 g) in N,N-dimethylformamide were added tert-butyl 2-bromo-2-methylpropanoate (17.6 g), potassium carbonate (10.9 g) and magnesium sulfate (5.70 g), and the mixture was stirred at 95°C for 18 hr. Water was added to the reaction mixture, and the mixture was extracted with ethyl acetate. The ethyl acetate layer was washed with water and saturated brine, dried over anhydrous magnesium sulfate and concentrated. The residue was subjected to silica gel column chromatography, eluted with ethyl acetate-hexane (1:7, v/v) to give tert-butyl 2-{2-[(6-chloropyridin-3-yl)carbonyl]-5-methoxyphenoxy}-2-methylpropanoate as a colorless oil. Recrystallization from ethyl acetate-hexane gave colorless prism crystals (3.49 g, 54%). melting point 128 - 129°C.

### Reference Example 59

To a solution (60 ml) of [2-(benzyloxy)-4-methoxyphenyl](6-chloropyridin-3-yl)methanone (3.0 g) and 2-(5-methyl-2-phenyl-1,3-oxazol-4-yl)ethanol (1.9 g) in N,N-dimethylformamide was added sodium hydride (60%, oily, 408 mg) at 0°C, and the mixture was stirred at room temperature for 3 hr. Saturated aqueous ammonium chloride solution was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5, v/v) to give [2-(benzyloxy)-4-methoxyphenyl]{6-[2-(5-methyl-2-phenyl-1,3-oxazol-4-yl)ethoxy]pyridin-3-yl}methanone as a colorless oil (4.1 g, yield 93%).
¹H-NMR (300 MHz, CDCl₃) δ:2.33 (3H, s), 2.98 (2H, t, J=6.7 Hz), 3.85 (3H, s), 4.81 (2H, t, J=6.7 Hz), 4.97 (2H, s), 6.52-6.62 (2H, m), 6.70 (1H, d, J=8.7 Hz), 7.01-7.07 (2H, m), 7.13-7.24 (3H, m), 7.36-7.53 (4H, m), 7.94-8.06 (3H, m), 8.51 (1H, d, J=2.1 Hz).

### Reference Example 60

To a solution (120 ml) of [2-(benzyloxy)-4-methoxyphenyl]{6-[2-(5-methyl-2-phenyl-1,3-oxazol-4-yl)ethoxy]pyridin-3-yl}methanone (4.1 g) in tetrahydrofuran was added 10% palladium carbon (0.5 g), and the mixture was stirred under a hydrogen stream for 2 hr. The reaction mixture was filtrated, and the filtrate was concentrated. The residue was recrystallized from ethyl acetate-hexane to give (2-hydroxy-4-methoxyphenyl){6-[2-(5-methyl-2-phenyl-1,3-oxazol-4-yl)ethoxy]pyridin-3-yl}methanone as pale-yellow prism crystals (3.1 g, yield 91%). melting point 114 - 115°C.

### Reference Example 61

To a solution (25 ml) of [4-(benzyloxy)phenyl](2-hydroxy-4-methoxyphenyl)methanone (3.34 g), allyl (2S)-2-hydroxypropanoate (1.56 g) and triphenylphosphine (6.58 g) in dichloromethane was added diethyl azodicarboxylate (40% toluene solution, 11.4 ml) at 0°C, and the mixture was stirred at room temperature for 5 hr and concentrated. The residue was subjected to silica gel column chromatography, eluted with ethyl acetate-hexane (1:5 - 1:3, v/v). To a solution (120 ml) of the obtained oil in tetrahydrofuran was added 10% palladium carbon (0.5 g), and the mixture was stirred under a hydrogen stream for 3.5 hr. The reaction mixture was filtrated, and the filtrate was concentrated. The residue was subjected to silica gel column chromatography and eluted with ethyl acetate-hexane (1:4 - 1:2, v/v) to give propyl (2R)-2-[2-(4-hydroxybenzoyl)-5-methoxyphenoxy]propanoate as a brown oil (530 mg, 15%).
¹H-NMR (300 MHz, CDCl₃) δ:0.88 (3H, t, J=7.4 Hz), 1.30 (3H, d, J=6.8 Hz), 1.53-1.71 (2H, m), 3.82 (3H, s), 4.08 (2H, t, J=6.6 Hz), 4.62 (1H, q, J=6.8 Hz), 5.97 (1H, s), 6.34 (1H, d, J=2.3 Hz), 6.59 (1H, dd, J=8.5, 2.3 Hz), 6.77-6.87 (2H, m), 7.41 (1H, d, J=8.5 Hz), 7.73-7.83 (2H, m).

### Example 1

A mixture of (2-hydroxy-4-methoxyphenyl){4-[(5-methyl-2-phenyl-1,3-oxazol-4-yl)methoxy]phenyl}methanone (12.00 g), ethyl bromoacetate (4.16 ml), potassium carbonate (5.99 g) and N,N-dimethylformamide (100 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:2→1:1, v/v), and concentrated. The residue was dissolved in tetrahydrofuran (100 ml), ethanol (50 ml), water (50 ml) and lithium hydroxide monohydrate (3.47 g) were added thereto, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→3:1, v/v), concentrated, and crystallized from ethyl acetate-hexane to give (5-methoxy-2-{4-[(5-methyl-2-phenyl-1,3-oxazol-4-yl)methoxy]benzoyl}phenoxy)acetic acid (12.56 g, yield 92%) as colorless crystals. melting point 146 - 147°C.
¹H-NMR (300 MHz, CDCl₃) δ:2.47 (3 H, s), 3.88 (3 H, s), 4.80 (2 H, s), 5.08 (2 H, s), 6.59 - 6.64 (2 H, m), 7.09 (2 H, d, J=9.0 Hz), 7.39 - 7.46 (4 H, m), 7.83 (2 H, d, J=9.0 Hz), 7.98 - 8.02 (2 H, m).

### Example 2

A mixture of (2-hydroxy-4-methoxyphenyl){3-[(5-methyl-2-phenyl-1,3-oxazol-4-yl)methoxy]phenyl}methanone (3.24 g), ethyl bromoacetate (1.12 ml), potassium carbonate (2.16 g) and N,N-dimethylformamide (30 ml) was stirred at room temperature for 40 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:3→2:3, v/v), concentrated, and crystallized from ethyl acetate-hexane to give (5-methoxy-2-{3-[(5-methyl-2-phenyl-1,3-oxazol-4-yl)methoxy]benzoyl}phenoxy)ethyl acetate (2.68 g, yield 69%) as colorless crystals. melting point 90 - 91°C.
¹H-NMR (300 MHz, CDCl₃) δ:1.23 (3 H, t, J=7.2 Hz), 2.44 (3 H, s), 3.84 (3 H, s), 4.18 (2 H, q, J=7.2 Hz), 4.51 (2 H, s), 5.03 (2 H, s), 6.37 (1 H, d, J=2.4 Hz), 6.60 (1 H, dd, J=2.4, 8.7 Hz), 7.17 - 7.22 (1 H, m), 7.33 (1 H, t, J=7.8 Hz), 7.39 - 7.46 (5 H, m), 7.48 - 7.51 (1 H, m), 7.99 - 8.03 (2 H, m).

### Example 3

To a mixture of (5-methoxy-2-{3-[(5-methyl-2-phenyl-1,3-oxazol-4-yl)methoxy]benzoyl}phenoxy)ethyl acetate (900 mg), tetrahydrofuran (20 ml) and water (2 ml) was added sodium borohydride (68 mg), and the mixture was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:2→2:1, v/v) and concentrated. The residue was dissolved in tetrahydrofuran (6 ml), ethanol (4 ml), water (4 ml) and lithium hydroxide monohydrate (72 mg) was added thereto, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was precipitated from ethyl acetate-hexane to give [2-(hydroxy{3-[(5-methyl-2-phenyl-1,3-oxazol-4-yl)methoxy]phenyl}methyl)-5-methoxyphenoxy]acetic acid (111 mg, yield 13%) as a colorless amorphous form.
¹H-NMR (300 MHz, CDCl₃) δ:2.44 (3 H, s), 3.76 (3 H, s), 4.36 - 4.51 (2 H, m), 5.00 (2 H, s), 5.91 (1 H, s), 6.38 (1 H, d, J=2.4 Hz), 6.49 (1 H, dd, J=2.4, 8.4 Hz), 6.83 - 6.91 (2 H, m), 7.12 (1 H, d, J=8.4 Hz), 7.19 (1 H, d, J=7.8 Hz), 7.23 - 7.26 (1 H, m), 7.42 - 7.47 (3 H, m), 7.96 - 8.02 (2 H, m).

### Example 4

A mixture of (5-methoxy-2-{3-[(5-methyl-2-phenyl-1,3-oxazol-4-yl)methoxy]benzoyl}phenoxy)ethyl acetate (350 mg), tetrahydrofuran (6 ml), ethanol (4 ml), water (4 ml) and lithium hydroxide monohydrate (88 mg) was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was crystallized from ethyl acetate-hexane to give (5-methoxy-2-{3-[(5-methyl-2-phenyl-1,3-oxazol-4-yl)methoxy]benzoyl}phenoxy)acetic acid (320 mg, yield 97%) as colorless crystals. melting point 198 - 199°C.
¹H-NMR (300 MHz, CDCl₃) δ:2.45 (3 H, s), 3.86 (3 H, s), 4.73 (2 H, s), 5.02 (2 H, s), 6.55 - 6.61 (2 H, m), 7.22 - 7.27 (1 H, m), 7.36 - 7.49 (7 H, m), 8.00 - 8.05 (2 H, m).

### Example 5

A mixture of 2-(1-hydroxy-1-{3-[(5-methyl-2-phenyl-1,3-oxazol-4-yl)methoxy]phenyl}ethyl)-5-methoxyphenol (417 mg), ethyl bromoacetate (0.139 ml), potassium carbonate (267 mg) and N,N-dimethylformamide (5 ml) was stirred at room temperature for 18 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:3→1:1, v/v), concentrated, and crystallized from ethyl acetate-hexane to give [2-(1-hydroxy-1-{3-[(5-methyl-2-phenyl-1,3-oxazol-4-yl)methoxy]phenyl}ethyl)-5-methoxyphenoxy]ethyl acetate (428 mg, yield 86%) as colorless crystals. melting point 144 - 145°C.
¹H-NMR (300 MHz, CDCl₃) δ:1.28 (3 H, t, J=7.2 Hz), 1.82 (3 H, s), 2.41 (3 H, s), 3.79 (3 H, s), 4.22 (2 H, q, J=7.2 Hz), 4.27 (1 H, d, J=15.6 Hz), 4.43 (1 H, d, J=15.6 Hz), 4.93 (2 H, s), 5.09 (1 H, s), 6.33 (1 H, d, J=2.4 Hz), 6.54 (1 H, dd, J=2.4, 8.4 Hz), 6.81 - 6.85 (1 H, m), 6.95 (1 H, d, J=7.5 Hz), 7.00 - 7.02 (1 H, m), 7.17 (1 H, t, J=8.0 Hz), 7.36 (1 H, d, J=8.4 Hz), 7.39 - 7.47 (3 H, m), 7.97 - 8.02 (2 H, m).

### Example 6

A mixture of [2-(1-hydroxy-1-{3-[(5-methyl-2-phenyl-1,3-oxazol-4-yl)methoxy]phenyl}ethyl)-5-methoxyphenoxy]ethyl acetate (368 mg), tetrahydrofuran (6 ml), ethanol (4 ml), water (4 ml) and lithium hydroxide monohydrate (85 mg) was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was crystallized from ethyl acetate-hexane to give [2-(1-hydroxy-1-{3-[(5-methyl-2-phenyl-1,3-oxazol-4-yl)methoxylphenyl}ethyl)-5-methoxyphenoxylacetic acid (340 mg, yield 98%) as colorless crystals. melting point 148 - 149°C.
¹H-NMR (300 MHz, CDCl₃) δ:1.86 (3 H, s), 2.48 (3 H, s), 3.72 (1 H, d, J=15.0 Hz), 3.79 (3 H, s), 4.39 (1 H, d, J=15.0 Hz), 4.91 (1 H, d, J=11.7 Hz), 5.14 (1 H, d, J=11.7 Hz), 6.40 (1 H, d, J=2.4 Hz), 6.57 - 6.63 (2 H, m), 6.76 (1 H, ddd, J=1.2, 2.4, 7.8 Hz), 7.10 (1 H, dd, J=7.2, 7.8 Hz), 7.39 - 7.41 (1 H, m), 7.43 - 7.48 (4 H, m), 7.97 - 8.02 (2 H, m).

### Example 7

A mixture of ethoxy(2-hydroxy-4-methoxyphenyl){3-[(5-methyl-2-phenyl-1,3-oxazol-4-yl)methoxy]phenyl}methane (217 mg), ethyl bromoacetate (0.070 ml), potassium carbonate (135 mg) and N,N-dimethylformamide (5 ml) was stirred at room temperature for 18 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:2, v/v), and concentrated. The residue was dissolved in tetrahydrofuran (6 ml), ethanol (4 ml), water (4 ml) and lithium hydroxide monohydrate (95 mg) were added thereto, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was crystallized from ethyl acetate-hexane to give [2-(ethoxy{3-[(5-methyl-2-phenyl-1,3-oxazol-4-yl)methoxy]phenyl}methyl)-5-methoxyphenoxy]acetic acid (162 mg, yield 66%) as colorless crystals. melting point 129 - 130°C.
¹H-NMR (300 MHz, CDCl₃) δ:1.27 (3 H, t, J=7.1 Hz), 2.45 (3 H, s), 3.60 (2 H, q, J=7.1 Hz), 3.77 (3 H, s), 4.39 (1 H, d, J=15.3 Hz), 4.62 (1 H, d, J=15.3 Hz), 4.91 (1 H, d, J=11.4 Hz), 5.02 (1 H, d, J=11.4 Hz), 5.58 (1 H, s), 6.40 (1 H, d, J=2.4 Hz), 6.53 (1 H, dd, J=2.4, 8.4 Hz), 6.85 - 6.90 (1 H, m), 6.98 (1 H, d, J=7.5 Hz), 7.15 - 7.17 (1 H, m), 7.19 - 7.29 (2 H, m), 7.42 - 7.47 (3 H, m), 7.99 - 8.05 (2 H, m).

### Example 8

A mixture of (2-hydroxyphenyl){4-[(5-methyl-2-phenyl-1,3-oxazol-4-yl)methoxy]phenyl}methanone (1.71 g), ethyl bromoacetate (0.64 ml), potassium carbonate (920 mg) and N,N-dimethylformamide (10 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), and concentrated. The residue was dissolved in tetrahydrofuran (20 ml), ethanol (10 ml), water (10 ml) and lithium hydroxide monohydrate (532 mg) were added, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was crystallized from ethyl acetate-hexane to give (2-{4-[(5-methyl-2-phenyl-1,3-oxazol-4-yl)methoxy]benzoyl}phenoxy)acetic acid (1.72 g, yield 87%) as colorless crystals. melting point 174 - 175°C.
¹H-NMR (300 MHz, CDCl₃) δ:2.47 (3 H, s), 4.81 (2 H, s), 5.09 (2 H, s), 7.07 - 7.17 (4 H, m), 7.41 - 7.47 (4 H, m), 7.51 - 7.58 (1 H, m), 7.89 (2 H, d, J=9.0 Hz), 7.98 - 8.04 (2 H, m).

### Example 9

A mixture of [2-hydroxy-4-(methoxymethoxy)phenyl]{4-[(5-methyl-2-phenyl-1,3-oxazol-4-yl)methoxy]phenyl}methanone (3.00 g), ethyl bromoacetate (0.97 ml), potassium carbonate (1.40 g) and N,N-dimethylformamide (10 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v) to give (5-(methoxymethoxy)-2-{4-[(5-methyl-2-phenyl-1,3-oxazol-4-yl)methoxy]benzoyl}phenoxy)ethyl acetate (3.58 g, yield 100%) as a colorless oil.
¹H-NMR (300 MHz, CDCl₃) δ:1.23 (3 H, t, J=7.2 Hz), 2.45 (3 H, s), 3.49 (3 H, s), 4.18 (2 H, q, J=7.2 Hz), 4.53 (2 H, s), 5.05 (2 H, s), 5.20 (2 H, s), 6.52 (1 H, d, J=2.1 Hz), 6.76 (1 H, dd, J=2.1, 8.7 Hz), 7.03 (2 H, d, J=9.0 Hz), 7.37 (1 H, d, J=8.7 Hz), 7.41 - 7.48 (3 H, m), 7.86 (2 H, d, J=9.0 Hz), 7.98 - 8.04 (2 H, m).

### Example 10

(5-(Methoxymethoxy)-2-{4-[(5-methyl-2-phenyl-1,3-oxazol-4-yl)methoxy]benzoyl}phenoxy)ethyl acetate (250 mg) was dissolved in tetrahydrofuran (6 ml), ethanol (4 ml), water (4 ml) and lithium hydroxide monohydrate (56 mg) were added, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→3:1, v/v) to give (5-(methoxymethoxy)-2-{4-[(5-methyl-2-phenyl-1,3-oxazol-4-yl)methoxy]benzoyl}phenoxy)acetic acid (218 mg, yield 92%) as colorless amorphous form.
¹H-NMR (300 MHz, CDCl₃) δ:2.45 (3 H, s), 3.48 (3 H, s), 4.74 (2 H, s), 5.07 (2 H, s), 5.21 (2 H, s), 6.72 - 6.78 (2 H, m), 7.06 (2 H, d, J=9.0 Hz), 7.37 (1 H, d, J=8.1 Hz), 7.39 - 7.46 (3 H, m), 7.84 (2 H, d, J=9.0 Hz), 7.97 - 8.02 (2 H, m), 9.90 - 10.40 (1 H, br).

### Example 11

A mixture of ethyl (5-(methoxymethoxy)-2-{4-[(5-methyl-2-phenyl-1,3-oxazol-4-yl)methoxy]benzoyl}phenoxy)acetate (3.26 g), 1 M hydrochloric acid (6 ml) and acetone (30 ml) was stirred at 60°C for 8 hr. The reaction mixture was concentrated, water was added to the residue, and the mixture was extracted with ethyl acetate. The ethyl acetate layer was washed with water and saturated brine, dried (MgSO₄) and concentrated. The residue was crystallized from ethyl acetate-hexane to give (5-hydroxy-2-{4-[(5-methyl-2-phenyl-1,3-oxazol-4-yl)methoxy]benzoyl}phenoxy)acetic acid (184 mg, yield 6.5%) as colorless crystals. melting point 229 - 230°C.
¹H-NMR (300 MHz, DMSO-d₆) δ:2.47 (3 H, s), 4.55 (2 H, s), 5.10 (2 H, s), 6.35 (1 H, d, J=1.8 Hz), 6.48 (1 H, dd, J=1.8, 8.4 Hz), 7.10 (2 H, d, J=8.7 Hz), 7.17 (1 H, d, J=8.4 Hz), 7.50 - 7.55 (3 H, m), 7.74 (2 H, d, J=8.7 Hz), 7.92 - 7.96 (2 H, m), 10.07 (1 H, s), 13.00 (1 H, br s).

### Example 12

To a residue obtained by concentrating the mother liquor of Example 11 were added ethanol (30 ml) and conc. sulfuric acid (0.5 ml), and the mixture was stirred at 80°C for 1 hr. The reaction mixture was concentrated, water was added to the residue, and the mixture was extracted with ethyl acetate. The ethyl acetate layer was washed with water and saturated brine, dried (MgSO₄) and concentrated. The residue was crystallized from ethyl acetate-hexane to give ethyl (5-hydroxy-2-{4-[(5-methyl-2-phenyl-1,3-oxazol-4-yl)methoxy]benzoyl}phenoxy)acetate (2.37 g, yield 79%) as colorless crystals. melting point 184 - 186°C.
¹H-NMR (300 MHz, CDCl₃) δ:1.17 (3 H, t, J=7.2 Hz), 2.48 (3 H, s), 4.10 (2 H, q, J=7.2 Hz), 4.44 (2 H, s), 5.03 (2 H, s), 6.32 (1 H, d, J=2.4 Hz), 6.54 (1 H, dd, J=2.4, 8.4 Hz), 6.90 (2 H, d, J=9.0 Hz), 7.26 (1 H, d, J=8.4 Hz), 7.42 - 7.48 (3 H, m), 7.81 (2 H, d, J=9.0 Hz), 7.99 - 8.06 (2 H, m).

### Example 13

A mixture of (2-hydroxy-4-methoxyphenyl){4-[(5-methyl-2-phenyl-1,3-oxazol-4-yl)methoxy]phenyl}methanone (500 mg), tert-butyl 2-bromopropanoate (755 mg), potassium carbonate (665 mg), magnesium sulfate (145 mg) and N,N-dimethylformamide (10 ml) was stirred at room temperature for 5 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), and concentrated. The residue was dissolved in trifluoroacetic acid (5 ml), and the mixture was stirred at room temperature for 30 min. The reaction mixture was concentrated, and the residue was crystallized from ethyl acetate-hexane to give 2-(5-methoxy-2-{4-[(5-methyl-2-phenyl-1,3-oxazol-4-yl)methoxy]benzoyl}phenoxy)propanoic acid (530 mg, yield 90%) as colorless crystals. melting point 100 - 102°C.
¹H-NMR (300 MHz, CDCl₃) δ:1.71 (3 H, d, J=6.9 Hz), 2.47 (3 H, s), 3.87 (3 H, s), 5.00 (1 H, q, J=6.9 Hz), 5.09 (2 H, s), 6.60 (1 H, dd, J=2.4, 8.4 Hz), 6.64 (1 H, d, J=2.4 Hz), 7.10 (2 H, d, J=8.7 Hz), 7.39 (1 H, d, J=8.4 Hz), 7.41 - 7.47 (3 H, m), 7.86 (2 H, d, J=8.7 Hz), 7.98 - 8.04 (2 H, m).

### Example 14

A mixture of (2-hydroxy-4-methoxyphenyl){4-[(5-methyl-2-phenyl-1,3-oxazol-4-yl)methoxy]phenyl}methanone (500 mg), tert-butyl 2-bromo-2-methylpropanoate (806 mg), potassium carbonate (665 mg), magnesium sulfate (145 mg) and N,N-dimethylformamide (10 ml) was stirred at 75°C for 40 hr. To the reaction mixture were added tert-butyl 2-bromo-2-methylpropanoate (806 mg), potassium carbonate (665 mg) and magnesium sulfate (145 mg), and the mixture was further stirred at 75°C for 40 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:2→1:1, v/v), and concentrated. The residue was dissolved in trifluoroacetic acid (5 ml), and the mixture was stirred at room temperature for 30 min. The reaction mixture was concentrated, and the residue was crystallized from ethyl acetate-hexane to give 2-(5-methoxy-2-{4-[(5-methyl-2-phenyl-1,3-oxazol-4-yl)methoxy]benzoyl}phenoxy)-2-methylpropanoic acid (432 mg, yield 72%) as colorless crystals. melting point 175 - 177°C.
¹H-NMR (300 MHz, CDCl₃) δ:1.67 (6 H, s), 2.47 (3 H, s), 3.86 (3 H, s), 5.08 (2 H, s), 6.64 (1 H, dd, J=2.4, 8.4 Hz), 6.71 (1 H, d, J=2.4 Hz), 7.07 (2 H, d, J=9.0 Hz), 7.42 - 7.48 (4 H, m), 7.79 (2 H, d, J=9.0 Hz), 7.98 - 8.03 (2 H, m).

### Example 15

To a mixture of (2-hydroxy-4-methoxyphenyl){4-[(5-methyl-2-phenyl-1,3-oxazol-4-yl)methoxy]phenyl}methanone (500 mg), potassium tert-butoxide (135 mg), tetrahydrofuran (5 ml) and N,N-dimethylformamide (5 ml) was added β-propiolactone (0.0832 ml), and the mixture was stirred at room temperature for 15 hr. 1 M Hydrochloric acid was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:2→5:1, v/v), concentrated, and crystallized from ethyl acetate-hexane to give 3-(5-methoxy-2-{4-[(5-methyl-2-phenyl-1,3-oxazol-4-yl)methoxy]benzoyl}phenoxy)propanoic acid (131 mg, yield 22%) as colorless crystals. melting point 142 - 143°C.
¹H-NMR (300 MHz, CDCl₃) δ:2.43 (2 H, t, J=5.3 Hz), 2.49 (3 H, s), 3.86 (3 H, s), 4.08 (2 H, t, J=5.3 Hz), 5.16 (2 H, s), 6.44 (1 H, d, J=2.4 Hz), 6.58 (1 H, dd, J=2.4, 8.7 Hz), 7.00 (2 H, d, J=8.7 Hz), 7.41 - 7.47 (3 H, m), 7.54 (1 H, d, J=8.7 Hz), 7.66 (2 H, d, J=8.7 Hz), 7.94 - 7.99 (2 H, m).

### Example 16

A mixture of ethyl (5-hydroxy-2-{4-[(5-methyl-2-phenyl-1,3-oxazol-4-yl)methoxy]benzoyl}phenoxy)acetate (200 mg), ethyl iodide (0.043 ml), potassium carbonate (85 mg) and N,N-dimethylformamide (5 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in tetrahydrofuran (6 ml), ethanol (4 ml), water (4 ml) and lithium hydroxide monohydrate (49 mg) were added thereto, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→3:1, v/v) to give (5-ethoxy-2-{4-[(5-methyl-2-phenyl-1,3-oxazol-4-yl)methoxy]benzoyl}phenoxy)acetic acid (135 mg, yield 68%) as colorless amorphous form.
¹H-NMR (300 MHz, CDCl₃) δ:1.46 (3 H, t, J=7.1 Hz), 2.47 (3 H, s), 4.10 (2 H, q, J=7.1 Hz), 4.79 (2 H, s), 5.08 (2 H, s), 6.56 - 6.61 (2 H, m), 7.09 (2 H, d, J=8.7 Hz), 7.38 - 7.47 (4 H, m), 7.84 (2 H, d, J=8.7 Hz), 7.99 - 8.03 (2 H, m).

### Example 17

A mixture of (2-hydroxy-4-methoxyphenyl){4-[(5-methyl-2-phenyl-1,3-oxazol-4-yl)methoxy]phenyl}methanone (400 mg), methyl 2-bromobutanoate (261 mg), potassium carbonate (226 mg) and N,N-dimethylformamide (3 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), and concentrated. The residue was dissolved in tetrahydrofuran (6 ml), methanol (4 ml), water (4 ml) and lithium hydroxide monohydrate (116 mg) were added thereto, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was crystallized from ethyl acetate-hexane to give 2-(5-methoxy-2-{4-[(5-methyl-2-phenyl-1,3-oxazol-4-yl)methoxy]benzoyl}phenoxy)butanoic acid (455 mg, yield 94%) as colorless crystals. melting point 161 - 162°C.
¹H-NMR (300 MHz, CDCl₃) δ:1.10 (3 H, t, J=7.4 Hz), 2.01 - 2.15 (2 H, m), 2.47 (3 H, s), 3.87 (3 H, s), 4.93 (1 H, dd, J=4.8, 6.6 Hz), 5.09 (2 H, s), 6.59 (1 H, dd, J=2.4, 8.7 Hz), 6.65 (1 H, d, J=2.4 Hz), 7.11 (2 H, d, J=9.0 Hz), 7.39 (1 H, d, J=8.7 Hz), 7.42 - 7.46 (3 H, m), 7.87 (2 H, d, J=9.0 Hz), 7.99 - 8.04 (2 H, m).

### Example 18

A mixture of (2-hydroxy-4-methoxyphenyl){4-[(5-methyl-2-phenyl-1,3-oxazol-4-yl)methoxy]phenyl}methanone (400 mg), ethyl 2-bromo-3-methylbutanoate (302 mg), potassium carbonate (226 mg) and N,N-dimethylformamide (3 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), and concentrated. The residue was dissolved in tetrahydrofuran (6 ml), ethanol (4 ml), water (4 ml) and lithium hydroxide monohydrate (116 mg) were added, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→3:1, v/v), concentrated, and crystallized from ethyl acetate-hexane to give 2-(5-methoxy-2-{4-[(5-methyl-2-phenyl-1,3-oxazol-4-yl)methoxy]benzoyl}phenoxy)-3-methylbutanoic acid (382 mg, yield 77%) as colorless crystals. melting point 87 - 88°C.
¹H-NMR (300 MHz, CDCl₃) δ:1.06 (3 H, d, J=6.9 Hz), 1.14 (3 H, d, J=6.9 Hz), 2.36 - 2.46 (1 H, m), 2.47 (3 H, s), 3.86 (3 H, s), 4.85 (1 H, d, J=3.6 Hz), 5.10 (2 H, s), 6.58 (1 H, dd, J=2.4, 8.7 Hz), 6.65 (1 H, d, J=2.4 Hz), 7.11 (2 H, d, J=8.7 Hz), 7.38 (1 H, d, J=8.7 Hz), 7.42 - 7.47 (3 H, m), 7.87 (2 H, d, J=8.7 Hz), 7.99 - 8.04 (2 H, m).

### Example 19

A mixture of (2-hydroxy-4-methoxyphenyl){4-[(5-methyl-2-phenyl-1,3-oxazol-4-yl)methoxy]phenyl}methanone (400 mg), ethyl bromodifluoroacetate (293 mg), potassium carbonate (226 mg) and N,N-dimethylformamide (3 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:2, v/v), and concentrated. The residue was dissolved in tetrahydrofuran (6 ml), ethanol (4 ml), water (4 ml) and lithium hydroxide monohydrate (116 mg) were added thereto, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→3:1, v/v), concentrated, and crystallized from ethyl acetate-hexane to give difluoro(5-methoxy-2-{4-[(5-methyl-2-phenyl-1,3-oxazol-4-yl)methoxy]benzoyl}phenoxy)acetic acid (108 mg, yield 22%) as colorless crystals. melting point 166 - 167°C.
¹H-NMR (300 MHz, CDCl₃) δ:2.49 (3 H, s), 3.89 (3 H, s), 5.11 (2 H, s), 6.87 (1 H, dd, J=2.4, 8.7 Hz), 6.93 (1 H, s like), 7.08 (2 H, d, J=9.0 Hz), 7.43 - 7.48 (3 H, m), 7.60 (1 H, d, J=8.7 Hz), 7.78 (2 H, d, J=9.0 Hz), 7.95 - 8.00 (2 H, m).

### Example 20

A mixture of (2-hydroxy-4-methoxyphenyl){4-[(5-methyl-2-phenyl-1,3-oxazol-4-yl)methoxy]phenyl}methanone (400 mg), ethyl 2-bromopentanoate (302 mg), potassium carbonate (226 mg) and N,N-dimethylformamide (3 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), and concentrated. The residue was dissolved in tetrahydrofuran (6 ml), ethanol (4 ml), water (4 ml) and lithium hydroxide monohydrate (116 mg) were added thereto, and the mixture was stirred at room temperature for 4 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→3:1, v/v), concentrated, and crystallized from ethyl acetate-hexane to give 2-(5-methoxy-2-(4-[(5-methyl-2-phenyl-1,3-oxazol-4-yl)methoxy]benzoyl}phenoxy)pentanoic acid (432 mg, yield 87%) as colorless crystals. melting point 139 - 140°C.
¹H-NMR (300 MHz, CDCl₃) δ:0.97 (3 H, t, J=7.4 Hz), 1.49 - 1.62 (2 H, m), 1.97 - 2.06 (2 H, m), 2.47 (3 H, s), 3.87 (3 H, s), 4.97 (1 H, t, J=5.7 Hz), 5.09 (2 H, s), 6.59 (1 H, dd, J=2.4, 8.7 Hz), 6.64 (1 H, d, J=2.4 Hz), 7.11 (2 H, d, J=9.0 Hz), 7.38 (1 H, d, J=8.7 Hz), 7.41 - 7.47 (3 H, m), 7.87 (2 H, d, J=9.0 Hz), 7.99 - 8.04 (2 H, m).

### Example 21

A mixture of (2-hydroxy-4-methoxyphenyl){6-[(5-methyl-2-phenyl-1,3-oxazol-4-yl)methoxy]pyridin-3-yl}methanone (300 mg), tert-butyl 2-bromopropanoate (226 mg), potassium carbonate (169 mg) and N,N-dimethylformamide (3 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), and concentrated. The residue was dissolved in trifluoroacetic acid (3 ml), and the mixture was stirred at room temperature for 30 min. The reaction mixture was concentrated, and the residue was crystallized from ethyl acetate-hexane to give 2-[5-methoxy-2-({6-[(5-methyl-2-phenyl-1,3-oxazol-4-yl)methoxy]pyridin-3-yl}carbonyl)phenoxy]propanoic acid (204 mg, yield 58%) as colorless crystals. melting point 162 - 163°C.
¹H-NMR (300 MHz, CDCl₃) δ:1.71 (3 H, d, J=6.9 Hz), 2.50 (3 H, s), 3.89 (3 H, s), 4.99 (1 H, q, J=6.9 Hz), 5.41 (2 H, s), 6.60 - 6.65 (2 H, m), 6.91 (1 H, d, J=7.8 Hz), 7. 41 - 7.48 (4 H, m), 7.99 - 8.05 (2 H, m), 8.10 (1 H, dd, J=2.4, 8.4 Hz), 8.63 (1 H, d, J=2.4 Hz).

### Example 22

A mixture of (2-hydroxyphenyl){4-[(5-methyl-2-phenyl-1,3-oxazol-4-yl)methoxy]phenyl}methanone (180 mg), tert-butyl 2-bromopropanoate (127 mg), potassium carbonate (97 mg) and N,N-dimethylformamide (3 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), and concentrated. The residue was dissolved in trifluoroacetic acid (3 ml), and the mixture was stirred at room temperature for 30 min. The reaction mixture was concentrated, and the residue was crystallized from ethyl acetate-hexane to give 2-(2-{4-[(5-methyl-2-phenyl-1,3-oxazol-4-yl)methoxy]benzoyl}phenoxy)propanoic acid (181 mg, yield 85%) as colorless crystals. melting point 186 - 187°C.
¹H-NMR (300 MHz, CDCl₃) δ:1.69 (3 H, d, J=6.9 Hz), 2.47 (3 H, s), 5.04 (1 H, q, J=6.9 Hz), 5.10 (2 H, s), 7.08 - 7.16 (4 H, m), 7.41 - 7.48 (4 H, m), 7.50 - 7.57 (1 H, m), 7.90 (2 H, d, J=8.7 Hz), 7.98 - 8.04 (2 H, m).

### Example 23

A mixture of (2-hydroxy-4-methoxyphenyl){4-[(5-methyl-2-phenyl-1,3-oxazol-4-yl)methoxy]phenyl}methanone (400 mg), ethyl 1-bromocyclobutanecarboxylate (1.20 g), potassium carbonate (1.06 g), magnesium sulfate (232 mg) and N,N-dimethylformamide (5 ml) was stirred at 75°C for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:3→2:1, v/v), and concentrated. The residue was dissolved in tetrahydrofuran (6 ml), ethanol (4 ml), water (4 ml) and lithium hydroxide monohydrate (116 mg) was added thereto, and the mixture was stirred at room temperature for 15 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→3:1, v/v), concentrated, and crystallized from ethyl acetate-hexane to give 1-(5-methoxy-2-{4-[(5-methyl-2-phenyl-1,3-oxazol-4-yl)methoxy]benzoyl}phenoxy)cyclobutanecarboxylic acid (270 mg, yield 55%) as colorless crystals. melting point 199 - 201°C.
¹H-NMR (300 MHz, CDCl₃) δ:1.85 - 2.12 (2 H, m), 2.47 (3 H, s), 2.51 - 2.65 (2 H, m), 2.78 - 2.87 (2 H, m), 3.81 (3 H, s), 5.09 (2 H, s), 6.51 (1 H, d, J=2.4 Hz), 6.58 (1 H, dd, J=2.4, 8.7 Hz), 7.10 (2 H, d, J=9.0 Hz), 7.31 (1 H, d, J=8.7 Hz), 7.42 - 7.48 (3 H, m), 7.90 (2 H, d, J=9.0 Hz), 7.99 - 8.04 (2 H, m).

### Example 24

A mixture of (2-hydroxy-4-methoxyphenyl){4-[(5-methyl-2-phenyl-1,3-oxazol-4-yl)methoxy]phenyl}methanone (400 mg), methyl 2-bromo-2-phenylacetate (331 mg), potassium carbonate (226 mg) and N,N-dimethylformamide (3 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:4→1:1, v/v), and concentrated. The residue was dissolved in tetrahydrofuran (6 ml), methanol (4 ml), water (4 ml) and lithium hydroxide monohydrate (116 mg) were added thereto, and the mixture was stirred at room temperature for 1 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:1→10:1, v/v) to give (5-methoxy-2-{4-[(5-methyl-2-phenyl-1,3-oxazol-4-yl)methoxy]benzoyl}phenoxy) (phenyl)acetic acid (210 mg, yield 40%) as colorless amorphous form.
¹H-NMR (300 MHz, CDCl₃) δ:2.47 (3 H, s), 3.83 (3 H, s), 5.10 (2 H, s), 5.81 (1 H, s), 6.61 (1 H, dd, J=2.1, 8.4 Hz), 6.65 (1 H, d, J=2.1 Hz), 7.11 (2 H, d, J=9.0 Hz), 7.35 - 7.47 (7 H, m), 7.53 - 7.59 (2 H, m), 7.88 (2 H, d, J=9.0 Hz), 7.98 - 8.04 (2 H, m).

### Example 25

A mixture of [4-(benzyloxy)phenyl](2-hydroxy-4-methoxyphenyl)methanone (10.00 g), tert-butyl 2-bromo-2-methylpropanoate (40.04 g), potassium carbonate (33.07 g), magnesium sulfate (7.20 g) and N,N-dimethylformamide (100 ml) was stirred at 80°C for 18 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:6→1:5, v/v) to give tert-butyl 2-{2-[4-(benzyloxy)benzoyl]-5-methoxyphenoxy}-2-methylpropanoate (12.07 g, yield 85%) as a pale-yellow oil.
¹H-NMR (300 MHz, CDCl₃) δ:1.28 (6 H, s), 1.45 (9 H, s), 3.80 (3 H, s), 5.14 (2 H, s), 6.32 (1 H, d, J=2.1 Hz), 6.58 (1 H, dd, J=2.1, 8.7 Hz), 6.96 (2 H, d, J=9.0 Hz), 7.30 - 7.46 (6 H, m), 7.80 (2 H, d, J=9.0 Hz).

### Example 26

tert-Butyl 2-{2-[4-(benzyloxy)benzoyl]-5-methoxyphenoxy}-2-methylpropanote (250 mg) was dissolved in trifluoroacetic acid (2 ml), and the solution was stirred at room temperature for 1 hr. The reaction mixture was concentrated, and the residue was subjected to silica gel column chromatography and eluted with ethyl acetate-hexane (1:3→10:1, v/v) to give 2-{2-[4-(benzyloxy)benzoyl]-5-methoxyphenoxy}-2-methylpropanoic acid (202 mg, yield 92%) as a pale-yellow amorphous form.
¹H-NMR (300 MHz, CDCl₃)δ:1.70 (6 H, s), 3.87 (3 H, s), 5.16 (2 H, s), 6.65 (1 H, dd, J=2.4, 8.7 Hz), 6.72 (1 H, d, J=2.4 Hz), 7.02 (2 H, d, J=9.0 Hz), 7.34 - 7.46 (6 H, m), 7.78 (2 H, d, J=9.0 Hz).

### Example 27

A mixture of 5-(chloromethyl)-3-phenyl-1,2,4-oxadiazol (151 mg), tert-butyl 2-[2-(4-hydroxybenzoyl)-5-methoxyphenoxy]-2-methylpropanoate (200 mg), 1,8-diazabicyclo[5.4.0]-7-undecene (0.116 ml) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v) to give 2-(5-methoxy-2-{4-[(3-phenyl-1,2,4-oxadiazol-5-yl)methoxy]benzoyl}phenoxy)-2-methylpropanoic acid (150 mg, yield 59%) as pale-yellow amorphous form.
¹H-NMR (300 MHz, CDCl₃) δ:1.68 (6 H, s), 3.87 (3 H, s), 5.44 (2 H, s), 6.64 (1 H, dd, J=2.4, 8.7 Hz), 6.71 (1 H, d, J=2.4 Hz), 7.09 (2 H, d, J=8.7 Hz), 7.42 - 7.57 (4 H, m), 7.80 (2 H, d, J=8.7 Hz), 8.08 - 8.12 (2 H, m).

### Example 28

A mixture of 3-(chloromethyl)-5-phenyl-1,2,4-oxadiazol (111 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:2, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v) to give 2-(5-methoxy-2-{4-[(5-phenyl-1,2,4-oxadiazol-3-yl)methoxy]benzoyl}phenoxy)-2-methylpropanoic acid (221 mg, yield 87%) as pale-yellow amorphous form.
¹H-NMR (300 MHz, CDCl₃) δ:1.67 (6 H, s), 3.86 (3 H, s), 5.34 (2 H, s), 6.64 (1 H, dd, J=2.4, 8.7 Hz), 6.71 (1 H, d, J=2.4 Hz), 7.12 (2 H, d, J=9.0 Hz), 7.44 (1 H, d, J=8.7 Hz), 7.51 - 7.66 (3 H, m), 7.79 (2 H, d, J=9.0 Hz), 8.14 - 8.18 (2 H, m).

### Example 29

A mixture of 4-(chloromethyl)-5-methyl-2-phenyl-1,3-thiazole (127 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr. The reaction mixture was concentrated, and the residue was crystallized from ethyl acetate-hexane to give 2-(5-methoxy-2-14-[(5-methyl-2-phenyl-1,3-thiazol-4-yl)methoxy]benzoyl}phenoxy)-2-methylpropanoic acid (222 mg, yield 83%) as colorless crystals. melting point 145 - 147°C.
¹H-NMR (300 MHz, CDCl₃) δ:1.69 (6 H, s), 2.55 (3 H, s), 3.87 (3 H, s), 5.26 (2 H, s), 6.65 (1 H, dd, J=2.4, 8.7 Hz), 6.73 (1 H, d, J=2.4 Hz), 7.11 (2 H, d, J=8.7 Hz), 7.40 - 7.47 (4 H, m), 7.79 (2 H, d, J=8.7 Hz), 7.87 - 7.91 (2 H, m).

### Example 30

A mixture of 3-(chloromethyl)-5-phenylisoxazole (110 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v) to give 2-(5-methoxy-2-{4-[(5-phenylisoxazol-3-yl)methoxy]benzoyl}phenoxy)-2-methylpropanoic acid (157 mg, yield 62%) as pale-yellow amorphous form.
¹H-NMR (300 MHz, CDCl₃) δ:1.68 (6 H, s), 3.86 (3 H, s), 5.29 (2 H, s), 6.62 - 6.66 (2 H, m), 6.71 (1 H, d, J=2.4 Hz), 7.07 (2 H, d, J=9.0 Hz), 7.42 - 7.52 (4 H, m), 7.76 - 7.81 (4 H, m).

### Example 31

A mixture of 5-(chloromethyl)-3-phenylisoxazole (110 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v) to give 2-(5-methoxy-2-{4-[(3-phenylisoxazol-5-yl)methoxy]benzoyl}phenoxy)-2-methylpropanoic acid (205 mg, yield 81%) as a pale-yellow amorphous form.
¹H-NMR (300 MHz, CDCl₃) δ:1.67 (6 H, s), 3.86 (3 H, s), 5.30 (2 H, s), 6.62 - 6.72 (3 H, m), 7.05 (2 H, d, J=9.0 Hz), 7.42 - 7.49 (4 H, m), 7.78 - 7.84 (4 H, m).

### Example 32

A mixture of 4-(chloromethyl)-2-phenyl-1,3-oxazole (110 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:2, v/v), and concentrated. The residue was dissolved in trifluoroacetic acid (2 ml), and the mixture was stirred at room temperature for 1 hr. The reaction mixture was concentrated, and the residue was crystallized from ethyl acetate-hexane to give 2-(5-methoxy-2-{4-[(2-phenyl-1,3-oxazol-4-yl)methoxy]benzoyl}phenoxy)-2-methylpropanoic acid (209 mg, yield 83%) as colorless crystals. melting point 165 - 167°C.
¹H-NMR (300 MHz, CDCl₃) δ:1.70 (6 H, s), 3.87 (3 H, s), 5.17 (2 H, s), 6.65 (1 H, dd, J=2.4, 8.7 Hz), 6.73 (1 H, d, J=2.4 Hz), 7.08 (2 H, d, J=9.0 Hz), 7.43 - 7.51 (4 H, m), 7.77 - 7.83 (3 H, m), 8.03 - 8.09 (2 H, m).

### Example 33

A mixture of 4-(chloromethyl)-5-methyl-2-(2-furyl)-1,3-oxazole(113 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v) to give 2-[2-(4-{[2-(2-furyl)-5-methyl-1,3-oxazol-4-yl]methoxy}benzoyl)-5-methoxyphenoxy]-2-methylpropanoic acid (200 mg, yield 79%) as pale-yellow amorphous form.
¹H-NMR (300 MHz, CDCl₃) δ:1.67 (6 H, s), 2.44 (3 H, s), 3.86 (3 H, s), 5.07 (2 H, s), 6.52 - 6.55 (1 H, m), 6.64 (1 H, dd, J=2.4, 9.0 Hz), 6.71 (1 H, d, J=2.4 Hz), 6.99 (1 H, d, J=3.3 Hz), 7.05 (2 H, d, J=9.0 Hz), 7.44 (1 H, d, J=9.0 Hz), 7.53 - 7.55 (1 H, m), 7.78 (2 H, d, J=9.0 Hz).

### Example 34

A mixture of 5-(chloromethyl)-3-(4-chlorophenyl)-1,2,4-oxadiazole (130 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v) to give 2-[5-methoxy-2-(4-{[3-(4-chlorophenyl)-1,2,4-oxadiazol-5-yl]methoxy}benzoyl)phenoxy]-2-methylpropanoic acid (138 mg, yield 51%) as a pale-yellow amorphous form.
¹H-NMR (300 MHz, CDCl₃) δ:1.67 (6 H, s), 3.86 (3 H, s), 5.43 (2 H, s), 6.64 (1 H, dd, J=2.1, 9.0 Hz), 6.70 (1 H, d, J=2.1 Hz), 7.09 (2 H, d, J=9.0 Hz), 7.44 (1 H, d, J=9.0 Hz), 7.48 (2 H, d, J=9.0 Hz), 7.80 (2 H, d, J=9.0 Hz), 8.04 (2 H, d, J=9.0 Hz).

### Example 35

A mixture of 3-(chloromethyl)-5-[4-(trifluoromethyl)phenyl]-1,2,4-oxadiazole (150 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v) to give 2-{5-methoxy-2-[4-({5-[4-(trifluoromethyl)phenyl]-1,2,4-oxadiazol-3-yl}methoxy)benzoyl]phenoxy}-2-methylpropanoic acid (226 mg, yield 79%) as pale-yellow amorphous form.
¹H-NMR (300 MHz, CDCl₃) δ:1.66 (6 H, s), 3.86 (3 H, s), 5.36 (2 H, s), 6.64 (1 H, dd, J=1.8, 9.0 Hz), 6.69 (1 H, d, J=1.8 Hz), 7.11 (2 H, d, J=8.7 Hz), 7.44 (1 H, d, J=9.0 Hz), 7.71 (1 H, t, J=7.8 Hz), 7.80 (2 H, d, J=8.7 Hz), 7.89 (1 H, d, J=7.8 Hz), 8.35 (1 H, d, J=7.8 Hz), 8.45 (1 H, s).

### Example 36

A mixture of 4-(2-bromo-1-oxoethyl)-5-methyl-2-phenyl-1,3-oxazole (159 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v) to give 2-(5-methoxy-2-{4-[2-(5-methyl-2-phenyl-1,3-oxazol-4-yl)-2-oxoethoxy]benzoyl}phenoxy)-2-methylpropanoic acid (208 mg, yield 76%) as a pale-yellow amorphous form.
¹H-NMR (300 MHz, CDCl₃) δ:1.69 (6 H, s), 2.75 (3 H, s), 3.86 (3 H, s), 5.48 (2 H, s), 6.64 (1 H, dd, J=2.4, 8.7 Hz), 6.72 (1 H, d, J=2.4 Hz), 7.03 (2 H, d, J=9.0 Hz), 7.44 - 7.53 (4 H, m), 7.78 (2 H, d, J=9.0 Hz), 8.04 - 8.09 (2 H, m).

### Example 37

A mixture of 4-(chloromethyl)-5-methyl-2-[3-(methanesulfonyloxy)phenyl]-1,3-oxazole (172 mg), tert-butyl 2-[2-(4-hydroxybenzoyl)-5-methoxyphenoxy]-2-methylpropanoate (200 mg), potassium carbonate (107 mg) and N,N-dimethylformamide (2 ml) was stirred at room temperature for 15 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography, eluted with ethyl acetate-hexane (1:5→1:1, v/v), and concentrated. The residue was dissolved in trifluoroacetic acid (2 ml), and the mixture was stirred at room temperature for 1 hr and concentrated. Water was added to the residue, and the mixture was extracted with ethyl acetate. The ethyl acetate layer was successively washed with aqueous sodium hydrogen carbonate solution and saturated brine, dried (MgSO₄) and concentrated. The residue was subjected to silica gel column chromatography, eluted with ethyl acetate-hexane (1:2→10:1, v/v), concentrated, and crystallized from ethyl acetate-hexane to give 2-{5-methoxy-2-[4-({5-methyl-2-[3-(methanesulfonyloxy)phenyl]-1,3-oxazol-4-yl}methoxy)benzoyl]phenoxy}-2-methylpropanoic acid (120 mg, yield 39%) as colorless crystals. melting point 182 - 183°C. ¹H-NMR (300 MHz, CDCl₃) δ:1.69 (6 H, s), 2.48 (3 H, s), 3.20 (3 H, s), 4.09 (3 H, s), 5.08 (2 H, s), 6.65 (1 H, dd, J=2.4, 8.7 Hz), 6.72 (1 H, d, J=2.4 Hz), 7.07 (2 H, d, J=9.0 Hz), 7.35 - 7.40 (1 H, m), 7.45 (1 H, d, J=8.7 Hz), 7.52 (1 H, t, J=8.0 Hz), 7.79 (2 H, d, J=9.0 Hz), 7.91 - 7.93 (1 H, m), 7.97 - 8.01 (1 H, m).

### Example 38

To a mixture of 2-(5-methyl-2-phenyl-1,3-oxazol-4-yl)ethanol (355 mg), tert-butyl 2-[2-(4-hydroxybenzoyl)-5-methoxyphenoxy]-2-methylpropanoate (450 mg), triphenylphosphine (611 mg) and dichloromethane (5 ml) was added dropwise diethyl azodicarboxylate (40% toluene solution, 1.06 ml) at 0°C. The reaction mixture was stirred at room temperature for 1 h and concentrated. The residue was subjected to silica gel column chromatography, eluted with ethyl acetate-hexane (1:4→2:3, v/v), and concentrated. The residue was dissolved in acetone (10 ml), 6 M hydrochloric acid (2 ml) was added, and the mixture was stirred at 60°C for 15 hr. The reaction mixture was concentrated, water was added to the residue, and the mixture was extracted with ethyl acetate. The ethyl acetate layer was washed with saturated brine, dried (MgSO₄) and concentrated. The residue was subjected to silica gel column chromatography, eluted with ethyl acetate-hexane (1:2→10:1, v/v), concentrated, and crystallized from ethyl acetate-hexane to give 2-(5-methoxy-2-{4-[2-(5-methyl-2-phenyl-1,3-oxazol-4-yl)ethoxy]benzoyl}phenoxy)-2-methylpropanoic acid (295 mg, yield 49%) as colorless crystals. melting point 81 - 82°C.
¹H-NMR (300 MHz, CDCl₃) δ:1.68 (6 H, s), 2.39 (3 H, s), 3.02 (2 H, t, J=6.6 Hz), 3.85 (3 H, s), 4.33 (2 H, t, J=6.6 Hz), 6.62 (1H, dd, J=2.4, 8.7 Hz), 6.71 (1H, d, J=2.4 Hz), 6.94 (2 H, d, J=9.0 Hz), 7.39 - 7.45 (4 H, m), 7.75 (2 H, d, J=9.0 Hz), 7.95 - 7.99 (2 H, m).

### Example 39

To a solution (13 ml) of (2-hydroxy-5-methoxyphenyl){4-[(5-methyl-2-phenyl-1,3-oxazol-4-yl)methoxy]phenyl}methanone (2.10 g), methyl (2S)-2-hydroxypropanoate (789 mg) and triphenylphosphine (2.66 g) in dichloromethane was added dropwise diethyl azodicarboxylate (40% toluene solution, 4.60 ml) at 0°C, and the mixture was stirred at room temperature for 3 hr. The reaction mixture was concentrated, and the residue was subjected to silica gel column chromatography, eluted with ethyl acetate-hexane (1:3→1:1, v/v) and concentrated. The residue was dissolved in tetrahydrofuran (15 ml), water (5 ml) was added and the mixture was cooled to 0°C. Lithium hydroxide monohydrate (339 mg) was added, and the mixture was stirred at 0°C for 3 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:2→10:1, v/v), concentrated, and crystallized from ethyl acetate-hexane to give (2R)-2-(4-methoxy-2-{4-[(5-methyl-2-phenyl-1,3-oxazol-4-yl)methoxy]benzoyl}phenoxy)propanoic acid (1.64 g, yield 67%) as colorless crystals. melting point 119 - 120°C.
¹H-NMR (300 MHz, CDCl₃) δ:1.65 (3 H, d, J=6.9 Hz), 2.47 (3 H, s), 3.76 (3 H, s), 4.95 (1 H, q, J=6.9 Hz), 5.09 (2 H, s), 6.92 (1 H, d, J=3.0 Hz), 7.01 - 7.13 (4 H, m), 7.42 - 7.46 (3 H, m), 7.91 (2 H, d, J=9.0 Hz), 7.99 - 8.03 (2 H, m).

### Example 40

To a solution (3 ml) of (2-hydroxy-3-methoxyphenyl){4-[(5-methyl-2-phenyl-1,3-oxazol-4-yl)methoxy]phenyl}methanone (500 mg), methyl (2S)-2-hydroxypropanoate (188 mg) and triphenylphosphine (634 mg) in dichloromethane was added dropwise diethyl azodicarboxylate (40% toluene solution, 1.10 ml) at 0°C, and the mixture was stirred at room temperature for 2 hr. The reaction mixture was concentrated, and the residue was subjected to silica gel column chromatography, eluted with ethyl acetate-hexane (1:4→2:3, v/v) and concentrated. The residue was dissolved in tetrahydrofuran (7.5 ml), water (2.5 ml) was added thereto and the mixture was cooled to 0°C. Lithium hydroxide monohydrate (81 mg) was added thereto, and the mixture was stirred at 0°C for 1.5 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:2→10:1, v/v) to give (2R)-2-(6-methoxy-2-{4-[(5-methyl-2-phenyl-1,3-oxazol-4-yl)methoxy]benzoyl}phenoxy)propanoic acid (356 mg, yield 61%) as colorless amorphous form.
¹H-NMR (300 MHz, CDCl₃) δ:1.53 (3 H, d, J=7.2 Hz), 2.46 (3 H, s), 3.93 (3 H, s), 5.08 (2 H, s), 5.26 (1 H, q, J=7.2 Hz), 6.97 (1 H, dd, J=2.4, 6.9 Hz), 7.06 - 7.18 (4 H, m), 7.42 - 7.47 (3 H, m), 7.84 (2 H, d, J=9.0 Hz), 7.98 - 8.03 (2 H, m).

### Example 41

To a solution (6 ml) of (2-hydroxy-4-methoxyphenyl){2-methyl-4-[(5-methyl-2-phenyl-1,3-oxazol-4-yl)methoxy]phenyl}methanone (1.00 g), methyl (2S)-2-hydroxypropanoate (364 mg) and triphenylphosphine (1.23 g) in dichloromethane was added dropwise diethyl azodicarboxylate (40% toluene solution, 2.12 ml) at 0°C, and the mixture was stirred at room temperature for 4 hr. The reaction mixture was concentrated, and the residue was subjected to silica gel column chromatography, eluted with ethyl acetate-hexane (1:4→1:1, v/v), and concentrated. The residue was dissolved in tetrahydrofuran (7.5 ml), water (2.5 ml) was added thereto and the mixture was cooled to 0°C. Lithium hydroxide monohydrate (298 mg) was added thereto, and the mixture was stirred at 0°C for 3 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:2→10:1, v/v) to give (2R)-2-(5-methoxy-2-{2-methyl-4-[(5-methyl-2-phenyl-1,3-oxazol-4-yl)methoxy]benzoyl}phenoxy)propanoic acid (451 mg, yield 39%) as pale-yellow amorphous form.
¹H-NMR (300 MHz, CDCl₃) δ:1.74 (3 H, d, J=7.2 Hz), 2.46 (3 H, s), 2.48 (3 H, s), 3.87 (3 H, s), 4.99 (1 H, q, J=7.2 Hz), 5.09 (2 H, s), 6.54 (1 H, dd, J=2.4, 8.4 Hz), 6.60 (1 H, d, J=2.4 Hz), 6.83 (1 H, dd, J=2.4, 8.4 Hz), 6.92 (1 H, d, J=2.4 Hz), 7.27 (1 H, d, J=8.4 Hz), 7.38 (1 H, d, J=8.4 Hz), 7.46 - 7.49 (3 H, m), 7.98 - 8.03 (2 H, m).

### Example 42

To a solution (5 ml) of (2-hydroxy-4-methoxy-6-methylphenyl){4-[(5-methyl-2-phenyl-1,3-oxazol-4-yl)methoxy]phenyl}methanone (716 mg), methyl (2S)-2-hydroxypropanoate (348 mg) and triphenylphosphine (1.10 g) in dichloromethane was added dropwise diethyl azodicarboxylate (40% toluene solution, 1.90 ml) at 0°C, and the mixture was stirred at room temperature for 1 hr. The reaction mixture was concentrated, and the residue was subjected to silica gel column chromatography, eluted with ethyl acetate-hexane (1:5→1:2, v/v) and concentrated. The residue was dissolved in tetrahydrofuran (7.5 ml), water (2.5 ml) was added and the mixture was cooled to 0°C. Lithium hydroxide monohydrate (217 mg) was added, and the mixture was stirred at 0°C for 3 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→10:1, v/v) and concentrated, and crystallized from ethyl acetate-hexane to give (2R)-2-(5-methoxy-3-methyl-2-{4-[(5-methyl-2-phenyl-1,3-oxazol-4-yl)methoxy]benzoyl}phenoxy)propanoic acid (201 mg, yield 24%) as colorless crystals. melting point 122 - 123°C.
¹H-NMR (300 MHz, CDCl₃) δ:1.52 (3 H, d, J=6.9 Hz), 2.08 (3 H, s), 2.46 (3 H, s), 3.82 (3 H, s), 4.87 (1 H, q, J=6.9 Hz), 5.07 (2 H, s), 6.40 - 6.42 (1 H, m), 6.44 - 6.46 (1 H, m), 7.07 (2 H, d, J=9.0 Hz), 7.42 - 7.47 (3 H, m), 7.84 (2 H, d, J=9.0 Hz), 7.99 - 8.03 (2 H, m).

### Example 43

To a solution (15 ml) of (5-methyl-2-phenyl-1,3-oxazol-4-yl)methanol (298 mg) in N,N-dimethylformamide was added sodium hydride (60%, oily, 66 mg) at room temperature and the mixture was stirred for 20 min. To this reaction mixture was added tert-butyl 2-{2-[(6-chloropyridin-3-yl)carbonyl]-5-methoxyphenoxy}-2-methylpropanoate (609 mg), and the mixture was stirred at room temperature for 2.5 hr. Saturated aqueous ammonium chloride solution was added to the reaction mixture, and the mixture was extracted with ethyl acetate. The ethyl acetate layer was washed with water and saturated brine, dried over anhydrous magnesium sulfate and concentrated. The residue was subjected to silica gel column chromatography, eluted with ethyl acetate-hexane (1:4, v/v) to give tert-butyl 2-[5-methoxy-2-({6-[(5-methyl-2-phenyl-1,3-oxazol-4-yl)methoxy]pyridin-3-yl}carbonyl)phenoxy]-2-methylpropanoate as a colorless oil (610 mg, yield 73%).
¹H-NMR (300 MHz, CDCl₃) δ:1.30 (6H, s), 1.46 (9H, s), 2.48 (3H, s), 3.81 (3H, s), 5.37 (2H, s), 6.30 (1H, d, J=2.1 Hz), 6.60 (1H, dd, J=8.5, 2.3 Hz), 6.79-6.85 (1H, m), 7.39-7.52 (4H, m), 7.98-8.08 (3H, m), 8.63 (1H, dd, J=2.4, 0.7 Hz).

### Example 44

To a solution (10 ml) of phenol (99 mg) in N,N-dimethylformamide was added sodium hydride (60%, oily, 44 mg) at room temperature, and the mixture was stirred for 20 min. To this reaction mixture was added tert-butyl 2-{2-[(6-chloropyridin-3-yl)carbonyl]-5-methoxyphenoxy}-2-methylpropanoate (406 mg), and the mixture was stirred at 50°C for 15 hr. Saturated aqueous ammonium chloride solution was added to the reaction mixture, and the mixture was extracted with ethyl acetate. The ethyl acetate layer was washed with water and saturated brine, dried over anhydrous magnesium sulfate and concentrated. The residue was subjected to silica gel column chromatography, eluted with ethyl acetate-hexane (1:4, v/v) to give tert-butyl 2-{5-methoxy-2-[(6-phenoxypyridin-3-yl)carbonyl]phenoxy}-2-methylpropanoate as a colorless oil (436 mg, yield 94%).
¹H-NMR (300 MHz, CDCl₃) δ:1.33 (6H, s), 1.43 (9H, s), 3.80 (3H, s), 6.30 (1H, d, J=2.3 Hz), 6.59 (1H, dd, J=8.6, 2.2 Hz), 6.90 (1H, d, J=8.7 Hz), 7.10-7.28 (3H, m), 7.35-7.47 (2H, m), 7.50 (1H, d, J=8.6 Hz), 8.14 (1H, dd, J=8.7, 2.5 Hz), 8.58 (1H, d, J=1.9 Hz).

### Example 45

To a solution (5 ml) of tert-butyl 2-{2-[(6-chloropyridin-3-yl)carbonyl]-5-methoxyphenoxy}-2-methylpropanoate (365 mg) and benzyl alcohol (112 µl) in N,N-dimethylformamide was added sodium hydride (60%, oily, 43 mg) at 0°C, and the mixture was stirred at 80°C for 1 hr. Saturated aqueous ammonium chloride solution was added to the reaction mixture, and the mixture was extracted with ethyl acetate. The ethyl acetate layer was washed with water and saturated brine, dried over anhydrous magnesium sulfate and concentrated. The residue was subjected to silica gel column chromatography, eluted with ethyl acetate-hexane (1:9 - 1:3, v/v) to give tert-butyl 2-(2-{[6-(benzyloxy)pyridin-3-yl]carbonyl}-5-methoxyphenoxy)-2-methylpropanoate as a colorless oil (355 mg, yield 83%). Recrystallization from ethyl acetate-hexane gave colorless prism crystals. melting point 58 - 60°C.

### Example 46

To a solution (5 ml) of tert-butyl 2-{2-[(6-chloropyridin-3-yl)carbonyl]-5-methoxyphenoxy}-2-methylpropanoate (365 mg) and 2-phenylethanol (162 µl) in N,N-dimethylformamide was added sodium hydride (60%, oily, 54 mg) at 0°C and the mixture was stirred at room temperature for 3.5 hr. Saturated aqueous ammonium chloride solution was added to the reaction mixture, and the mixture was extracted with ethyl acetate. The ethyl acetate layer was washed with water and saturated brine, dried over anhydrous magnesium sulfate and concentrated. The residue was subjected to silica gel column chromatography, eluted with ethyl acetate-hexane (1:4, v/v) to give tert-butyl 2-(5-methoxy-2-{[6-(2-phenylethoxy)pyridin-3-yl]carbonyl}phenoxy)-2-methylpropanoate as a colorless oil (203 mg, yield 46%).
¹H-NMR (300 MHz, CDCl₃) δ:1.32 (6H, s), 1.46 (9H, s), 3.10 (2H, t, J=7.1 Hz), 3.81 (3H, s), 4.60 (2H, t, J=7.1 Hz), 6.31 (1H, d, J=2.1 Hz), 6.59 (1H, dd, J=8.6, 2.1 Hz), 6.72 (1H, d, J=8.7 Hz), 7.16-7.37 (5H, m), 7.47 (1H, d, J=8.7 Hz), 8.04 (1H, dd, J=8.6, 2.4 Hz), 8.59 (1H, d, J=2.4 Hz).

### Example 47

To a solution (8 ml) of (2-hydroxy-4-methoxyphenyl){4-[(5-methyl-2-phenyl-1,3-oxazol-4-yl)methoxy]phenyl}methanone (1.0 g), allyl (2S)-2-hydroxypropanoate (0.47 g) and triphenylphosphine (0.76 g) in dichloromethane was added diethyl azodicarboxylate (40% toluene solution, 1.3 ml) at 0°C, and the mixture was stirred at room temperature for 20 hr. The reaction mixture was concentrated, and the residue was subjected to silica gel column chromatography, eluted with ethyl acetate-hexane (1:5 - 1:3, v/v) to give allyl (2R)-2-(5-methoxy-2-{4-[(5-methyl-2-phenyl-1,3-oxazol-4-yl)methoxy]benzoyl}phenoxy)propanoate as a colorless oil (1.2 g, yield 92%).
¹H-NMR (300 MHz, CDCl₃) δ:1.29 (3H, d, J=6.8 Hz), 2.45 (3H, s), 3.82 (3H, s), 4.56-4.69 (3H, m), 5.05 (2H, s), 5.19-5.34 (2H, m), 5.77-5.93 (1H, m), 6.34 (1H, d, J=2.3 Hz), 6.80 (1H, dd, J=8.5, 2.3 Hz), 6.98-7.06 (2H, m), 7.39-7.49 (4H, m), 7.80-7.89 (2H, m), 7.97-8.06 (2H, m).

### Example 48

To a solution (8 ml) of (2-hydroxy-4-methoxyphenyl){6-[(5-methyl-2-phenyl-1,3-oxazol-4-yl)methoxy]pyridin-3-yl}methanone (1.25 g), allyl (2S)-2-hydroxypropanoate (0.59 g) and triphenylphosphine (1.58 g) in dichloromethane was added diethyl azodicarboxylate (40% toluene solution, 2.7 ml) at 0°C, and the mixture was stirred at room temperature for 15 min. The reaction mixture was concentrated, and the residue was subjected to silica gel column chromatography, eluted with ethyl acetate-hexane (1:4, v/v) to give allyl (2R)-2-[5-methoxy-2-({6-[(5-methyl-2-phenyl-1,3-oxazol-4-yl)methoxy]pyridin-3-yl}carbonyl)phenoxy]propanoate as a colorless oil (1.50 g, yield 95%).
¹H-NMR (300 MHz, CDCl₃) δ:1.29 (3H, d, J=6.8 Hz), 2.49 (3H, s), 3.83 (3H, s), 4.57-4.73 (3H, m), 5.20-5.34 (2H, m), 5.37 (2H, s), 5.77-5.97 (1H, m), 6.31 (1H, d, J=2.1 Hz), 6.62 (1H, dd, J=8.5, 2.1 Hz), 6.82 (1H, d, J=8.7 Hz), 7.37-7.56 (4H, m), 7.95-8.13 (3H, m), 8.63 (1H, d, J=2.5 Hz).

### Example 49

To a solution (12 ml) of (2-hydroxy-4-methoxyphenyl){6-[2-(5-methyl-2-phenyl-1,3-oxazol-4-yl)ethoxy]pyridin-3-yl}methanone (1.72 g), allyl (2S)-2-hydroxypropanoate (0.62 g) and triphenylphosphine (2.63 g) in dichloromethane was added diethyl azodicarboxylate (40% toluene solution, 4.6 ml) at 0°C, and the mixture was stirred at room temperature for 30 min. The reaction mixture was concentrated, and the residue was subjected to silica gel column chromatography, eluted with ethyl acetate-hexane (1:4, v/v) to give allyl (2R)-2-[5-methoxy-2-({6-[2-(5-methyl-2-phenyl-1,3-oxazol-4-yl)ethoxy]pyridin-3-yl}carbonyl)phenoxy]propanoate as a colorless oil (1.59 g, yield 73%).
¹H-NMR (300 MHz, CDCl₃) δ:1.30 (3H, d, J=6.8 Hz), 2.34 (3H, s), 3.00 (2H, t, J=6.8 Hz), 3.82 (3H, s), 4.56-4.74 (5H, m), 5.18-5.34 (2H, m), 5.77-5.95 (1H, m), 6.31 (1H, d, J=2.1 Hz), 6.61 (1H, dd, J=8.6, 2.1 Hz), 6.69-6.77 (1H, d, J=8.7 Hz), 7.35-7.52(4H, m), 7.93-8.02 (2H, m), 8.07 (1H, dd, J=8.7, 2.3 Hz), 8.58 (1H, d, J=2.3 Hz).

### Example 50

To a solution (4.7 ml) of propyl (2R)-2-[2-(4-hydroxybenzoyl)-5-methoxyphenoxy]propanoate (516 mg), 2-(5-methyl-2-phenyl-1,3-oxazol-4-yl)ethanol (351 mg) and triphenylphosphine (945 mg) in dichloromethane was added diethyl azodicarboxylate (40% toluene solution, 1.65 ml) at room temperature, and the mixture was stirred for 1 hr. The reaction mixture was concentrated, and the residue was subjected to silica gel column chromatography, eluted with ethyl acetate-hexane (1:4, v/v) to give propyl (2R)-2-(5-methoxy-2-{4-[2-(5-methyl-2-phenyl-1,3-oxazol-4-yl)ethoxy]benzoyl}phenoxy)propanoate as a colorless oil (736 mg, yield 94%).
¹H-NMR (300 MHz, CDCl₃) δ:0.87 (3H, t, J=7.4 Hz), 1.27 (3H, d, J=6.8 Hz), 1.53-1.70 (2H, m), 2.38 (3H, s), 3.00 (2H, t, J=6.7 Hz), 3.82 (3H, s), 4.07 (2H, t, J=6.7 Hz), 4.31 (2H, t, J=6.7 Hz), 4.60 (1H, q, J=6.8 Hz), 6.33 (1H, d, J=2.3 Hz), 6.58 (1H, dd, J=8.5, 2.1 Hz), 6.84-6.94 (2H, m), 7.35-7.49 (4H, m), 7.77-7.86 (2H, m), 7.91-8.03 (2H, m).

### Example 51

A solution (5 ml) of tert-butyl 2-{5-methoxy-2-[(6-phenoxypyridin-3-yl)carbonyl]phenoxy}-2-methylpropanoate (436 mg) in trifluoroacetic acid was stirred at room temperature for 30 min. The reaction mixture was concentrated, and the residue was recrystallized from ethyl acetate-hexane to give 2-{5-methoxy-2-[(6-phenoxypyridin-3-yl)carbonyl]phenoxy}-2-methylpropanoic acid as colorless prism crystals (344 mg, 90%). melting point 167.5 - 168.5°C.
¹H-NMR (300 MHz, DMSO-d₆) δ:1.26 (6H, s), 3.79 (3H, s), 6.28 (1H, d, J=2.1 Hz), 6.74 (1H, dd, J=8.7, 2.3 Hz), 7.05-7.21 (3H, m), 7.21-7.31 (1H, m), 7.40-7.53 (3H, m), 8.09 (1H, dd, J=8.5, 2.5 Hz), 8.38-8.45 (1H, m), 13.3 (1H, br s,).

### Example 52

A solution (4 ml) of tert-butyl 2-(5-methoxy-2-{[6-(2-phenylethoxy)pyridin-3-yl]carbonyl}phenoxy)-2-methylpropanoate (200 mg) in trifluoroacetic acid was stirred at room temperature for 30 min. The reaction mixture was concentrated, and the residue was subjected to silica gel column chromatography, eluted with ethyl acetate-hexane (1:1 - 2:1, v/v) to give 2-(5-methoxy-2-{[6-(2-phenylethoxy)pyridin-3-yl]carbonyl}phenoxy)-2-methylpropanoic acid as pale-yellow oil (168 mg, yield 95%).
¹H-NMR (300 MHz, CDCl₃)δ:1.67 (6H, s), 3.12 (2H, t, J=7.0 Hz), 3.87 (3H, s), 4.61 (2H, t, J=7.0 Hz), 6.59-6.73 (2H, m), 6.81 (1H, d, J=8.7 Hz), 7.17-7.39 (5H, m), 7.47 (1H, d, J=8.7 Hz), 8.06 (1H, dd, J=8.7, 2.3 Hz), 8.52 (1H, d, J=2.3 Hz).

### Example 53

To allyl (2R)-2-(5-methoxy-2-{4-[(5-methyl-2-phenyl-1,3-oxazol-4-yl)methoxy]benzoyl}phenoxy)propanoate (0.83 g) in tetrahydrofuran-water mixed solution (3:1, v/v, 20 ml) was added lithium hydroxide monohydrate (101 mg) at 0°C. After stirring for 1 hr, 1 N hydrochloric acid (2.4 ml) was added and the mixture was extracted with ethyl acetate. The ethyl acetate layer was washed with saturated brine, dried over anhydrous magnesium sulfate and concentrated. The residue was recrystallized from ethyl acetate-hexane to give (2R)-2-(5-methoxy-2-{4-[(5-methyl-2-phenyl-1,3-oxazol-4-yl)methoxy]benzoyl}phenoxy)propanoic acid as colorless prism crystals (622 mg, yield 81%). melting point 169 - 170°C.
¹H-NMR (300 MHz, CDCl₃) δ:1.71 (3H, d, J=6.9 Hz), 2.47 (3H, s), 3.87 (3H, s), 5.00 (1H, q, J=6.9 Hz), 5.09 (2H, s), 6.55-6.67 (2H, m), 7.04-7.16 (2H, m), 7.35-7.51 (4H, m), 7.80-7.91 (2H, m), 7.96-8.07 (2H, m).

### Example 54

To allyl (2R)-2-[5-methoxy-2-({6-[(5-methyl-2-phenyl-1,3-oxazol-4-yl)methoxy]pyridin-3-yl}carbonyl)phenoxy]propanoate (1.0 g) in tetrahydrofuran-water mixed solution (3:1, v/v, 20 ml) was added lithium hydroxide monohydrate (126 mg) at 0°C. After stirring for 1 hr, 1 N hydrochloric acid (3.0 ml) was added and the mixture was extracted with ethyl acetate. The ethyl acetate layer was washed with saturated brine, dried over anhydrous magnesium sulfate and concentrated. The residue was recrystallized from ethyl acetate-hexane to give (2R)-2-[5-methoxy-2-({6-[(5-methyl-2-phenyl-1,3-oxazol-4-yl)methoxy]pyridin-3-yl}carbonyl)phenoxy]propanoic acid as colorless prism crystals (625 mg, yield 68%). melting point 164.5 - 165.5°C.
¹H-NMR (300 MHz, CDCl₃)δ:1.70 (3H, d, J=6.9 Hz), 2.50 (3H, s), 3.89 (3H, s), 4.98 (1H, q, J=6.9 Hz), 5.41 (2H, s), 6.56-6.66 (2H, m), 6.90 (1H, d, J=8.7 Hz), 7.38-7.49 (4H, m), 7.96-8.07 (2H, m), 8.10 (1H, dd, J=8.7, 2.5 Hz), 8.63 (1H, d, J=2.5 Hz).

### Example 55

To allyl (2R)-2-[5-methoxy-2-({6-[2-(5-methyl-2-phenyl-1,3-oxazol-4-yl)ethoxy]pyridin-3-yl}carbonyl)phenoxy]propanoate (1.59 g) in tetrahydrofuran-water mixed solution (3:1, v/v, 20 ml) was added lithium hydroxide monohydrate (189 mg) at 0°C. After stirring for 1.5 hr, 1 N hydrochloric acid (4.5 ml) was added and the mixture was extracted with ethyl acetate. The ethyl acetate layer was washed with saturated brine, dried over anhydrous magnesium sulfate and concentrated. The residue was subjected to silica gel column chromatography, eluted with ethyl acetate-hexane (1:1, v/v) to give (2R)-2-[5-methoxy-2-({6-[2-(5-methyl-2-phenyl-1,3-oxazol-4-yl)ethoxy]pyridin-3-yl}carbonyl)phenoxy]propanoic acid as a colorless oil (1.16 g, yield 79%).
¹H-NMR (300 MHz, CDCl₃) δ:1.65 (3H, d, J=6.8 Hz), 2.36 (3H, s), 2.92-3.14 (2H, m), 3.87 (3H, s), 4.56-4.78 (2H, m), 4.93 (1H, q, J=6.8 Hz), 6.53-6.65 (2H, m), 6.80 (1H, d, J=8.7 Hz), 7.36-7.50 (4H, m), 7.90-8.01 (2H, m), 8.11 (1H, dd, J=8.7, 2.5 Hz), 8.56 (1H, d, J=2.3 Hz).

### Example 56

To propyl (2R)-2-(5-methoxy-2-{4-[2-(5-methyl-2-phenyl-1,3-oxazol-4-yl)ethoxy]benzoyl}phenoxy)propanoate (736 mg) in tetrahydrofuran-water mixed solution (3:1, v/v, 12 ml) was added lithium hydroxide monohydrate (170 mg) at 0°C. After stirring for 2 hr, 1 N hydrochloric acid (4.1 ml) was added thereto and the mixture was extracted with ethyl acetate. The ethyl acetate layer was washed with saturated brine, dried over anhydrous magnesium sulfate and concentrated. The residue was subjected to silica gel column chromatography, eluted with ethyl acetate-hexane (1:1, v/v) - ethyl acetate to give (2R)-2-(5-methoxy-2-{4-[2-(5-methyl-2-phenyl-1,3-oxazol-4-yl)ethoxy]benzoyl}phenoxy)propanoic acid as a colorless oil (396 mg, yield 58%).
¹H-NMR (300 MHz, CDCl₃) δ:1.70 (3H, d, J=6.8 Hz), 2.39 (3H, s), 3.02 (2H, t, J=6.6 Hz), 3.87 (3H, s), 4.35 (2H, t, J=6.6 Hz), 4.99 (1H, q, J=6.8 Hz), 6.53-6.67 (2H, m), 6.91-7.02 (2H, m), 7.32-7.49 (4H, m), 7.76-7.87 (2H, m), 7.92-8.02 (2H, m).

### Example 57

To a solution (3 ml) of (2-hydroxy-4-methoxyphenyl){4-[(5-methyl-2-phenyl-1,3-oxazol-4-yl)methoxy]phenyl}methanone (500 mg), methyl (2R)-2-hydroxypropanoate (208 mg) and triphenylphosphine (789 mg) in dichloromethane was added dropwise diethyl azodicarboxylate (40% toluene solution, 1.37 ml) at 0°C, and the mixture was stirred at room temperature for 2 hr. The reaction mixture was concentrated, and the residue was subjected to silica gel column chromatography, eluted with ethyl acetate-hexane (1:6→1:2, v/v), and concentrated. The residue was dissolved in tetrahydrofuran (7 ml), water (3 ml) was added and the mixture was cooled to 0°C. Lithium hydroxide monohydrate (101 mg) was added thereto, and the mixture was stirred at 0°C for 2 hr. The reaction mixture was neutralized with dil. hydrochloric acid, and extracted with ethyl acetate. The ethyl acetate layer was washed with water and saturated brine, dried (MgSO₄) and concentrated. The residue was subjected to silica gel column chromatography, eluted with ethyl acetate-hexane (1:3→10:1, v/v), concentrated, and crystallized from ethyl acetate-hexane to give (2S)-2-(5-methoxy-2-{4-[(5-methyl-2-phenyl-1,3-oxazol-4-yl)methoxy]benzoyl}phenoxy)propanoic acid (151 mg, yield 26%) as colorless crystals. melting point 169 - 170°C.

### Example 58

To a solution of tert-butyl 2-[2-(4-hydroxybenzoyl)-5-methoxyphenoxy]-2-methylpropanoate (0.023 g), 2-phenylethyl alcohol (0.009 g) and tributylphosphine (0.030 g) in dry tetrahydrofuran (2.5 ml) was added 1,1'-(azodiarbonyl)dipiperidine (0.030 g) and the mixture was stirred for 24 hr. Hexane (2.0 ml) was added to the reaction mixture, and the precipitate was filtered off. The filtrate was concentrated, and the obtained residue was subjected to preparative HPLC (formic acid series), and trifluoroacetic acid (0.5 ml) was added to the obtained residue. Ten minutes later, the reaction mixture was concentrated under reduced pressure, and the obtained residue was subjected to preparative HPLC (trifluoroacetic acid series) again to give 2-{5-methoxy-2-[4-(2-phenylethoxy)benzoyl]phenoxy}-2-methylpropanoic acid (0.012 g, yield 48%).
LC-MS purity:88%, m/z=435[M+H⁺], retention time=2.26 min.

The compounds of Example 59 - Example 92 were obtained by reacting tert-butyl 2-[2-(4-hydroxybenzoyl)-5-methoxyphenoxy]-2-methylpropanoate with various alcohols by a method similar to that of Example 58.

### Example 59

### 2-(5-methoxy-2-{4-[(1S)-1-phenylethoxy]benzoyl}phenoxy)-2-methylpropanoic acid

Yield 9%, LC-MS purity:98%, m/z=435[M+H⁺], retention time=2.09 min.

### Example 60

### 2-(5-methoxy-2-{4-[2-(3-thienyl)ethoxy]benzoyl}phenoxy)-2-methylpropanoic acid

Yield 73%, LC-MS purity:91%, m/z=441[M+H⁺], retention time=2.22 min.

### Example 61

### 2-{5-methoxy-2-[4-(3-pyridin-3-ylpropoxy)benzoyl]phenoxy}-2-methylpropanoic acid

Yield 63%, LC-MS purity:98%, m/z=450[M+H⁺], retention time=1.60 min.

### Example 62

### 2-(5-methoxy-2-{4-[2-(4-methyl-1,3-thiazol-5-yl)ethoxy]benzoyl}phenoxy)-2-methylpropanoic acid

yield 77%, LC-MS purity:95%, m/z=456[M+H⁺], retention time=1.83 min.
¹H-NMR (400 MHz, DMSO-d₆) δ:1.24 (6 H, s), 2.23(3 H,s), 3.78 (3 H, s), 4.28 (2 H, s), 4.38 (2 H, s), 6.31 (1 H, s), 6.71 (1 H, d, J=6.30 Hz), 6.87 (2 H, d, J= 8.30 Hz), 7.00-7.15 (4 H, m), 7.35 (1 H, d, J=8.60 Hz), 7.67 (2 H, d, J=8.80 Hz), 13.23 (1H, br s).

### Example 63

### 2-[5-methoxy-2-(4-{2-[methyl(phenyl)amino]ethoxy}benzoyl)phenoxy]-2-methylpropanoic acid

yield 79%, LC-MS purity:96%, m/z=464[M+H⁺], retention time=1.94 min.

### Example 64

### 2-(5-methoxy-2-{4-[2-(4-methylphenoxy)ethoxy]benzoyl}phenoxy)-2-methylpropanoic acid

yield 72%, LC-MS purity:91%, m/z=465[M+H⁺], retention time=2.25 min.

### Example 65

### 2-(5-methoxy-2-{4-[2-(phenylthio)ethoxy]benzoyl}phenoxy)-2-methylpropanoic acid

yield 46%, LC-MS purity:97%, m/z=467[M+H⁺], retention time=2.29 min.

### Example 66

### 2-(2-{4-[2-(1H-indol-3-yl)ethoxy]benzoyl}-5-methoxyphenoxy)-2-methylpropanoic acid

yield 14%, LC-MS purity:90%, m/z=474[M+H⁺], retention time=2.37 min.

### Example 67

### 2-{2-[4-(diphenylmethoxy)benzoyl]-5-methoxyphenoxy}-2-methylpropanoic acid

yield 33%, LC-MS purity:98%, m/z=497[M+H⁺], retention time=2.19 min.

### Example 68

### 2-(2-{4-[2-(1,3-dioxo-1,3-dihydro-2H-isoindol-2-yl)ethoxy]benzoyl}-5-methoxyphenoxy)-2-methylpropanoic acid

yield 23%, LC-MS purity:90%, m/z=504[M+H⁺], retention time=2.06 min.

### Example 69

### 2-[2-(4-{2-[4-(benzyloxy)phenyl]ethoxy}benzoyl)-5-methoxyphenoxy]-2-methylpropanoic acid

yield 30%, LC-MS purity:97%, m/z=541[M+H⁺], retention time=2.44 min.

### Example 70

### 2-{5-methoxy-2-[4-(2-naphthylmethoxy)benzoyl]phenoxy}-2-methylpropanoic acid

yield 4%, LC-MS purity:99%, m/z=471[M+H⁺], retention time=2.14 min.

### Example 71

### 2-{5-methoxy-2-[4-(pyridin-4-ylmethoxy)benzoyl]phenoxy}-2-methylpropanoic acid

yield 77%, LC-MS purity:99%, m/z=422[M+H⁺], retention time=1.54 min.

### Example 72

### 2-(2-{4-[(2-chloro-4-fluorobenzyl)oxy]benzoyl}-5-methoxyphenoxy)-2-methylpropanoic acid

yield 91%, LC-MS purity:96%, m/z=473[M+H⁺], retention time=2.32 min.

### Example 73

### 2-(2-{4-[2-(3,4-dimethoxyphenyl)ethoxy]benzoyl}-5-methoxyphenoxy)-2-methylpropanoic acid

yield 92%, LC-MS purity:100%, m/z=495[M+H⁺], retention time=2.14 min.

### Example 74

### 2-(2-{4-[(3,5-dichlorobenzyl)oxy]benzoyl}-5-methoxyphenoxy)-2-methylpropanoic acid

yield 70%, LC-MS purity:99%, m/z=489[M+H⁺], retention time=2.43 min.

### Example 75

### 2-(2-{4-[(1-benzyl-1H-imidazol-5-yl)methoxy]benzoyl}-5-methoxyphenoxy)-2-methylpropanoic acid

yield 19%, LC-MS purity:99%, m/z=501[M+H⁺], retention time=1.70 min.

### Example 76

### 2-(2-{4-[(4-bromo-2-fluorobenzyl)oxy]benzoyl}-5-methoxyphenoxy)-2-methylpropanoic acid

yield 79%, LC-MS purity:97%, m/z=517[M+H⁺], retention time=2.3 5 min.

### Example 77

### 2-[2-(4-{[3,5-bis(trifluoromethyl)benzyl]oxy}benzoyl)-5-methoxyphenoxy]-2-methylpropanoic acid

yield 81%, LC-MS purity:97%, m/z=557[M+H⁺], retention time=2.43 min.

### Example 78

### 2-(5-methoxy-2-{4-[2-(2-naphthyl)ethoxy]benzoyl}phenoxy)-2-methylpropanoic acid

yield 45%, LC-MS purity:100%, m/z=485[M+H⁺], retention time=2.40 min.

### Example 79

### 2-(2-{4-[(4-cyanobenzyl)oxy]benzoyl}-5-methoxyphenoxy)-2-methylpropanoic acid

yield 99%, LC-MS purity:99%, m/z=446[M+H⁺], retention time=2.11 min.

### Example 80

### 2-(2-{4-[2-(4-cyanophenyl)ethoxy]benzoyl}-5-methoxyphenoxy)-2-methylpropanoic acid

yield 28%, LC-MS purity:98%, m/z=460[M+H⁺], retention time=2.15 min.

### Example 81

### 2-[5-methoxy-2-(4-{[6-(morpholin-4-yl)pyridin-3-yl]methoxy}benzoyl)phenoxy]-2-methylpropanoic acid

yield 42%, LC-MS purity:98%, m/z=507[M+H⁺], retention time=1.63 min.

### Example 82

### 2-[5-methoxy-2-(4-{[2-(morpholin-4-yl)pyridin-3-yl]methoxy}benzoyl)phenoxy]-2-methylpropanoic acid

yield 87%, LC-MS purity:100%, m/z=507[M+H⁺], retention time=1.76 min.

### Example 83

### 2-[2-(4-{[3-chloro-4-(trifluoromethoxy)benzyl]oxy}benzoyl)-5-methoxyphenoxy]-2-methylpropanoic acid

yield 85%, LC-MS purity:97%, m/z=539[M+H⁺], retention time=2.43 min.

### Example 84

### 2-[5-methoxy-2-(4-{[5-phenyl-2-(trifluoromethyl)-3-furyl]methoxy}benzoyl)phenoxy]-2-methylpropanoic acid

yield 71%, LC-MS purity:99%, m/z=555[M+H⁺], retention time=2.49 min.

### Example 85

### 2-(2-{4-[(2,4-dichlorobenzyl)oxy]benzoyl}-5-methoxyphenoxy)-2-methylpropanoic acid

yield 69%, LC-MS purity:99%, m/z=489[M+H⁺], retention time=2.42 min.
¹H-NMR (400 MHz, CDCl₃) δ:2.18 (6 H, s), 3.87 (3 H, s), 5.21 (2 H, s), 6.64 (1 H, dd, J=9.05, 1.47 Hz), 6.72 (1 H, s), 7.02 (2 H, d, J=8.56 Hz), 7.30 (1 H, dd, J=8.56, 1.47 Hz), 7.40 - 7.46 (2 H, m), 7.48 (1 H, d, J=8.07 Hz), 7.79 (2 H, d, J=8.56 Hz).

### Example 86

### 2-(5-methoxy-2-{4-[3-(4-methoxyphenoxy)propoxy]benzoyl}phenoxy)-2-methylpropanoic acid yield 19%, LC-MS purity:96%, m/z=479[M+H⁺], retention time=2.31 min.

### Example 87

### 2-(5-methoxy-2-{4-[3-(2-oxopyrrolidin-1-yl)propoxy]benzoyl}phenoxy)-2-methylpropanoic acid

yield 6%, LC-MS purity:84%, m/z=456[M+H⁺], retention time=1.83 min.

### Example 88

### 2-{2-[4-(2-cyclohexylethoxy)benzoyl]-5-methoxyphenoxy}-2-methylpropanoic acid

yield 51%, LC-MS purity:80%, m/z=441[M+H⁺], retention time=2.54 min.

### Example 89

### 2-{2-[4-(3-{[(benzyloxy)carbonyl]amino}propoxy)benzoyl]-5-methoxyphenoxy}-2-methylpropanoic acid

yield 23%, LC-MS purity:97%, m/z=522[M+H⁺], retention time=2.12 min.

### Example 90

### 2-(2-{4-[((2S)-2-{[(benzyloxy)carbonyl]amino}propyl)oxy]benzoyl}-5-methoxyphenoxy)-2-methylpropanoic acid

yield 17%, LC-MS purity:89%, m/z=522[M+H⁺], retention time=2.14 min.

### Example 91

### 2-(2-{4-[((2R)-2-{[(benzyloxy)carbonyl]amino}propyl)oxy]benzoyl}-5-methoxyphenoxy)-2-methylpropanoic acid

yield 58%, LC-MS purity:93%, m/z=522[M+H⁺], retention time=2.14 min.

### Example 92

### 2-[5-methoxy-2-(4-{[4-(1H-1,2,3-triazol-1-yl)benzyl]oxy}benzoyl)phenoxy]-2-methylpropanoic acid

yield 24%, LC-MS purity:90%, m/z=488[M+H⁺], retention time=1.99 min.

### Example 93

A mixture of tert-butyl 2-{2-[(6-chloropyridin-3-yl)carbonyl]-5-methoxyphenoxy}-2-methylpropanoate (0.2 M N,N-dimethylformamide solution, 0.5 ml) and 4-bromo-2-fluorobenzylalcohol (0.2 M N,N-dimethylformamide solution, 0.75 ml) was cooled to 0°C, and sodium hydride (60%, oily, 10 mg) was added thereto under argon atmosphere. The reaction mixture was stirred at 0°C for 3 hr, water (3 ml) and dichloromethane (3 ml) were added to the mixture and the mixture was vigorously stirred. The organic layer was separated, and filtered through a PTFE filter (polytetrafluoroethylene film processing). The filtrate was concentrated, dissolved in dimethyl sulfoxide (1 ml), subjected to preparative HPLC (trifluoroacetic acid series) and concentrated. The residue was dissolved in dichloromethane (1 ml), trifluoroacetic acid (0.5 ml) was added thereto and the mixture was stirred at room temperature for 30 min. The reaction mixture was concentrated, and the residue was dissolved in dimethyl sulfoxide (1 ml) and subjected to preparative HPLC (trifluoroacetic acid series) to give 2-[2-({6-[(4-bromo-2-fluorobenzyl)oxy]pyridin-3-yl}carbonyl)-5-methoxyphenoxy]-2-methylpropanoic acid trifluoroacetate as a colorless oil. yield 34%, LC-MS purity:97%, m/z=518[M+H⁺], retention time=2.35 min.
¹H-NMR (400 MHz, CDCl₃) δ: 1.47 (3 H, s), 1.48 (3 H, s),3.83 (3 H, s), 5.47 (2 H, s), 6.49 (1 H, s), 6.62 (1 H, d, J=8.8Hz), 6.84 (1 H, dd, J=8.8, 2.0 Hz), 7.26-7.30 (2 H, m), 7.38-7.46 (2 H, m), 8.08 (1 H, d, J=8.8Hz), 8.55 (1 H, d, 2.0Hz).

The compounds of Example 94 - Example 120 were obtained by reacting tert-butyl 2-{2-[(6-chloropyridin-3-yl)carbonyl]-5-methoxyphenoxy}-2-methylpropanoate with various alcohols by a method similar to that of Example 93.

### Example 94

### 2-(2-{[6-(cyclohexyloxy)pyridin-3-yl]carbonyl}-5-methoxyphenoxy)-2-methylpropanoic acid trifluoroacetate

yield 10%, LC-MS purity:96%, m/z=414[M+H⁺], retention time=2.30 min.

### Example 95

### 2-(5-methoxy-2-{[6-(tetrahydrofuran-2-ylmethoxy)pyridin-3-yl]carbonyl}phenoxy)-2-methylpropanoic acid trifluoroacetate

yield 10%, LC-MS purity:97%, m/z=416[M+H⁺], retention time=1.92 min.

### Example 96

### 2-{5-methoxy-2-[(6-{[(2S)-5-oxopyrrolidin-2-yl]methoxy}pyridin-3-yl)carbonyl]phenoxy}-2-methylpropanoic acid trifluoroacetate

yield 3%, LC-MS purity:80%, m/z=429[M+H⁺], retention time=2.05 min.

### Example 97

### 2-(5-methoxy-2-{[6-(tetrahydro-2H-pyran-2-ylmethoxy)pyridin-3-yl]carbonyl}phenoxy)-2-methylpropanoic acid trifluoroacetate yield 17%, LC-MS purity:99%, m/z=430[M+H⁺], retention time=2.05 min.

### Example 98

### 2-[5-methoxy-2-({6-[2-(3-thienyl)ethoxy]pyridin-3-yl}carbonyl)phenoxy]-2-methylpropanoic acid trifluoroacetate yield 14%, LC-MS purity:97%, m/z=442[M+H⁺], retention time=2.19 min.

### Example 99

### 2-[5-methoxy-2-({6-[(1-methylpiperidin-3-yl)methoxy]pyridin-3-yl}carbonyl)phenoxy]-2-methylpropanoic acid ditrifluoroacetate yield 7%, LC-MS purity:99%, m/z=443[M+H⁺], retention time=1.53 min.

### Example 100

### 2-[5-methoxy-2-({6-[3-(pyridin-3-yl)propoxy]pyridin-3-yl}carbonyl)phenoxy]-2-methylpropanoic acid ditrifluoroacetate yield 59%, LC-MS purity:98%, m/z=451[M+H⁺], retention time=1.56 min.

### Example 101

### 2-{5-methoxy-2-[(6-{2-[methyl(phenyl)amino]ethoxy}pyridin-3-yl)carbonyl]phenoxy}-2-methylpropanoic acid ditrifluoroacetate yield 10%, LC-MS purity:84%, m/z=465[M+H⁺], retention time=1.91 min.

### Example 102

### 2-(5-methoxy-2-{[6-(1-methyl-2-phenoxyethoxy)pyridin-3-yl]carbonyl}phenoxy)-2-methylpropanoic acid trifluoroacetate yield 21%, LC-MS purity:98%, m/z=466[M+H⁺], retention time=2.26 min.

### Example 103

### 2-[5-methoxy-2-({6-[2-(4-methylphenoxy)ethoxy]pyridin-3-yl}carbonyl)phenoxy]-2-methylpropanoic acid trifluoroacetate

yield 11%, LC-MS purity:98%, m/z=466[M+H⁺], retention time=2.25 min.
¹H-NMR (400 MHz, CDCl₃) δ:1.49 (6 H, s), 3.84 (3 H, s), 4.32 (2 H, t, J=4.80 Hz), 4.72 (2 H, t, J=4.80 Hz), 6.50 (1 H, d, J=2.4Hz), 6.62 (1 H, d, J=8.60 Hz), 6.85 (3 H, m), 7.09 (2 H, d, J=8.40 Hz),7.43 (5 H, m), 7.44 (1 H, d, J=8.80 Hz), 8.01 (1 H, d), 8.55 (1 H, d, J=2.4Hz).

### Example 104

### 2-[5-methoxy-2-({6-[2-(phenylthio)ethoxy]pyridin-3-yl}carbonyl)phenoxy]-2-methylpropanoic acid trifluoroacetate yield 12%, LC-MS purity:96%, m/z=468[M+H⁺], retention time=2.27 min.

### Example 105

### 2-{2-[(6-{[4-(acetylamino)cyclohexyl]oxy}pyridin-3-yl)carbonyl]-5-methoxyphenoxy}-2-methylpropanoic acid trifluoroacetate

yield 3%, LC-MS purity:96%, m/z=471[M+H⁺], retention time=1.80 min.

### Example 106

### 2-[2-({6-[2-(1H-indol-3-yl)ethoxy]pyridin-3-yl}carbonyl)-5-methoxyphenoxy]-2-methylpropanoic acid trifluoroacetate

yield 11%, LC-MS purity:91%, m/z=475[M+H⁺], retention time=2.16 min.

### Example 107

### 2-[2-({6-[1-(1H-benzoimidazol-2-yl)ethoxy]pyridin-3-yl}carbonyl)-5-methoxyphenoxy]-2-methylpropanoic acid ditrifluoroacetate

yield 6%, LC-MS purity:95%, m/z=476[M+H⁺], retention time=1.59 min.

### Example 108

### 2-[2-({6-[(1-benzylpyrrolidin-3-yl)oxy]pyridin-3-yl}carbonyl)-5-methoxyphenoxy]-2-methylpropanoic acid trifluoroacetate

yield 11%, LC-MS purity:97%, m/z=491[M+H⁺], retention time=1.65 min.
¹H-NMR (400 MHz, CDCl₃) δ:1.41 (3 H, s),1.44 (3 H, s), 3.84 (3 H, s), 5.71 (1 H, m), 6.47 (1 H, s), 6.62 (1 H, J=8.60 Hz), 6.79 (1 H, d, J=8.80 Hz), 7.43 (5 H, m), 7.48 (1 H, d, J=8.80 Hz), 8.01 (1 H, m), 8.50 (1 H, s).

### Example 109

### 2-[2-({6-[2-(1,3-dioxo-1,3-dihydro-2H-isoindol-2-yl)ethoxy]pyridin-3-yl}carbonyl)-5-methoxyphenoxy]-2-methylpropanoic acid trifluoroacetate

yield 3%, LC-MS purity:90%, m/z=505[M+H⁺], retention time=2.03 min.

### Example 110

### 2-{2-[(6-{2-[4-(benzyloxy)phenyl]ethoxy}pyridin-3-yl)carbonyl]-5-methoxyphenoxy}-2-methylpropanoic acid trifluoroacetate

yield 42%, LC-MS purity:96%, m/z=542[M+H⁺], retention time=2.42 min.

### Example 111

### 2-(2-{[6-(2,2-diphenylethoxy)pyridin-3-yl]carbonyl}-5-methoxyphenoxy)-2-methylpropanoic acid trifluoroacetate

yield 3%, LC-MS purity:92%, m/z=512[M+H⁺], retention time=2.39 min.

### Example 112

### 2-[2-({6-[(2-chloro-4-fluorobenzyl)oxy]pyridin-3-yl}carbonyl)-5-methoxyphenoxy]-2-methylpropanoic acid trifluoroacetate

yield 28%, LC-MS purity:94%, m/z=474[M+H⁺], retention time=2.31 min.

### Example 113

### 2-[2-({6-[2-(3,4-dimethoxyphenyl)ethoxy]pyridin-3-yl}carbonyl)-5-methoxyphenoxy]-2-methylpropanoic acid trifluoroacetate

yield 20%, LC-MS purity:98%, m/z=496[M+H⁺], retention time=2.10 min.

### Example 114

### 2-[2-({6-[(3,5-dichlorobenzyl)oxy]pyridin-3-yl}carbonyl)-5-methoxyphenoxy]-2-methylpropanoic acid trifluoroacetate

yield 5%, LC-MS purity:93%, m/z=490[M+H⁺], retention time=2.43 min.

### Example 115

### 2-{2-[(6-{[3,5-bis(trifluoromethyl)benzyl]oxy}pyridin-3-yl)carbonyl]-5-methoxyphenoxy}-2-methylpropanoic acid trifluoroacetate

yield 5%, LC-MS purity:95%, m/z=558[M+H⁺], retention time=2.42 min.

### Example 116

### 2-[5-methoxy-2-({6-[2-(2-naphthyl)ethoxy]pyridin-3-yl}carbonyl)phenoxy]-2-methylpropanoic acid trifluoroacetate yield 10%, LC-MS purity:99%, m/z=486[M+H⁺], retention time=2.37 min.

### Example 117

### 2-{2-[(6-{[3-chloro-4-(trifluoromethoxy)benzyl]oxy}pyridin-3-yl)carbonyl]-5-methoxyphenoxy}-2-methylpropanoic acid trifluoroacetate

yield 45%, LC-MS purity:96%, m/z=540[M+H⁺], retention time=2.43 min.

### Example 118

### 2-[5-methoxy-2-({6-[(4-methyl-3-oxo-1-phenylpyrazolidin-4-yl)methoxy]pyridin-3-yl}carbonyl)phenoxy]-2-methylpropanoic acid trifluoroacetate

yield 9%, LC-MS purity:91%, m/z=520[M+H⁺], retention time=1.95 min.

### Example 119

### 2-(2-{[6-(benzyloxy)pyridin-3-yl]carbonyl}-5-methoxyphenoxy)-2-methylpropanoic acid trifluoroacetate

yield 23%, LC-MS purity:85%, m/z=422[M+H⁺], retention time=2.18 min.
¹H-NMR (400 MHz, CDCl₃) δ:1.47 (6 H, s),3.84 (3 H, s), 5.46 (2 H, s), 6.49 (1 H, s), 6.62 (1 H, d, J=8.4Hz), 6.86 (1 H, d, J=8.40 Hz), 7.28-7.47 (6 H, m), 8.08 (1 H, d, J=8.8Hz), 8.57 (1 H, s).

### Example 120

### 2-[2-({6-[(2,4-dichlorobenzyl)oxy]pyridin-3-yl}carbonyl)-5-methoxyphenoxy]-2-methylpropanoic acid trifluoroacetate

yield 17%, LC-MS purity:100%, m/z=490[M+H⁺], retention time=2.42 min.
¹H-NMR (400 MHz, CDCl₃) δ:1.46 (6 H, s), 3.83 (3 H, s), 5.52 (2 H, s), 6.47 (1 H, s), 6.62 (1 H, m), 6.88 (1 H, d, J=8.30 Hz), 7.25-7.29 (1 H, m), 7.41-7.48 (3 H, m), 8.01 (1 H, m), 8.55 (1 H, s).

### Formulation Example 1 (production of capsule)

| | |
|---|---|
| (1) Compound of Example 1 | 30 mg |
| (2) Crystalline cellulose | 10 mg |
| (3) Lactose | 19 mg |
| (4) Magnesium stearate | 1 mg |
| total | 60 mg |

| | |
|---|---|
| (1), (2) and (3) and (4) are admixed and filled in a gelatin capsule. | |

### Formulation Example 2 (production of tablet)

| | |
|---|---|
| (1) Compound of Example 1 | 30 g |
| (2) Lactose | 50 g |
| (3) Corn starch | 15 g |
| (4) Carboxymethylcellulose calcium | 44 g |
| (5) Magnesium stearate | 1 g |
| 1000 tablets total | 140 g |

The entire amount of (1), (2) and (3), and 30 g of (4) are admixed with water. After drying in vacuo, the mixture is granulated. Thereto are added 14 g of (4) and 1 g of (5) and the mixture is tableted with a tableting machine. In this way, 1,000 tablets containing 30 mg of the compound of Example 1 per tablet are obtained.

### Industrial Applicability

The compound of the present invention has superior hypoglycemic action and superior hypolipidemic action, and is useful as an agent for the prophylaxis or treatment of diabetes, hyperlipidemia, impaired glucose tolerance and the like.

This application is based on a patent application No. 2005-221627 filed in Japan, the contents of which are incorporated in full herein by this reference.

## Claims

1. A compound represented by the formula (I): 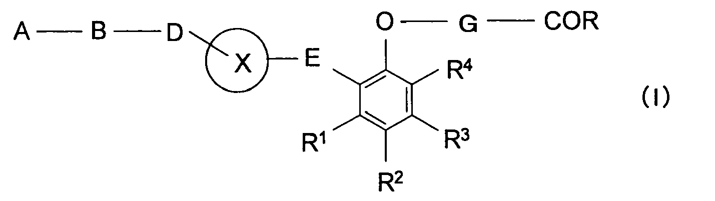 wherein
A is an optionally substituted cyclic group;
B is a bond or a spacer having 1 to 10 carbon atoms in the main chain;
D is O or NR⁵
wherein
R⁵ is a hydrogen atom or a substituent;
X is an optionally further substituted aromatic ring;
E is CO, C(OR⁶)R⁷ or C=NR⁸
wherein
R⁶, R⁷ and R⁸ are the same or different and each is a hydrogen atom or a substituent;
G is an optionally substituted divalent C₁₋₆ hydrocarbon group; R is -OR⁹
wherein R⁹ is a hydrogen atom or an optionally substituted hydrocarbon group, or
-NR¹⁰R¹¹
wherein R¹⁰ and R¹¹ are the same or different and each is a hydrogen atom, an optionally substituted hydrocarbon group or an optionally substituted heterocyclic group, or R¹⁰ and R¹¹ form, together with the adjacent nitrogen atom, an optionally substituted heterocycle; and
R¹, R², R³ and R⁴ are the same or different and each is a hydrogen atom or a substituent,
or a salt thereof.

2. The compound of claim 1, wherein A is a C₆₋₁₄ aryl group, a C₃₋₁₀ cycloalkyl group, a 5- or 6-membered aromatic heterocyclic group optionally condensed with a benzene ring, or a 5- or 6-membered non-aromatic heterocyclic group optionally condensed with a benzene ring, each of which is optionally substituted.

3. The compound of claim 1, wherein the spacer having 1 to 10 carbon atoms in the main chain for B is -(CH₂)ₖ-
wherein k is an integer of 1 to 10.

4. The compound of claim 1, wherein D is O.

5. The compound of claim 1, wherein X is a C₆₋₁₄ arene or a 6-membered aromatic heterocycle, each of which is optionally substituted.

6. The compound of claim 1, wherein E is CO.

7. The compound of claim 1, wherein G is a C₁₋₆ alkylene group optionally substituted by 1 to 3 substituents selected from a halogen atom and a C₆₋₁₄ aryl group.

8. The compound of claim 1, wherein R is a hydroxy group.

9. The compound of claim 1, wherein R¹, R², R³ and R⁴ are the same or different and each is a hydrogen atom, a C₁₋₆ alkoxy group optionally substituted by C₁₋₆ alkoxy group(s), a C₁₋₆ alkyl group, a carboxyl group, a carbamoyl group, a thiocarbamoyl group, a C₁₋₆ alkoxy-carbonyl group, a halogen atom, a cyano group or a nitro group.

10. The compound of claim 1, which is selected from
(5-methoxy-2-(4-[(5-methyl-2-phenyl-1,3-oxazol-4-yl)methoxy]benzoyl}phenoxy)acetic acid,
2-(5-methoxy-2-{4-[(5-methyl-2-phenyl-1,3-oxazol-4-yl)methoxy]benzoyl}phenoxy)-2-methylpropanoic acid,
2-[5-methoxy-2-({6-[(5-methyl-2-phenyl-1,3-oxazol-4-yl)methoxy]pyridin-3-yl}carbonyl)phenoxy]propanoic acid,
2-(2-{4-[(5-methyl-2-phenyl-1,3-oxazol-4-yl)methoxy]benzoyl}phenoxy)propanoic acid, and
(2R)-2-(5-methoxy-2-{4-[(5-methyl-2-phenyl-1,3-oxazol-4-yl)methoxy]benzoyl}phenoxy)propanoic acid.

11. A prodrug of the compound of claim 1.

12. A pharmaceutical agent comprising the compound of claim 1 or a prodrug thereof.

13. The pharmaceutical agent of claim 12, which is an agent for the prophylaxis or treatment of diabetes.

14. An insulin sensitizer comprising the compound of claim 1 or a prodrug thereof.

15. A method for the prophylaxis or treatment of diabetes in a mammal, which comprises administering the compound of claim 1 or a prodrug thereof to the mammal.

16. A method of improving insulin resistance in a mammal, which comprises administering the compound of claim 1 or a prodrug thereof to the mammal.

17. Use of the compound of claim 1 or a prodrug thereof for the production of an agent for the prophylaxis or treatment of diabetes.

18. Use of the compound of claim 1 or a prodrug thereof for the production of an insulin sensitizer.
